# EUROPEAN PATENT SPECIFICATION

(11) **EP 2 780 452 B1**
(45) Date of publication and mention of the grant of the patent: **28.12.2016**
(21) Application number: 12730698.3
(22) Date of filing: 21.06.2012
(51) Int. Cl.: C07K 16/40, C12P 7/26

(54) **VALENCENE SYNTHASE**
VALENCENSYNTHASE
VALENCÈNE SYNTHASE

(30) Priority: 21.06.2011 EP 11170805
(43) Date of publication of application: 24.09.2014
(73) Proprietor: Isobionics B.V., 6167 RD Geleen (NL)
(72) Inventor: SONKE, Theodorus, NL-6143 BK Guttecoven (NL); DE JONG, Rene M., NL-1091 VE Amsterdam (NL)
(74) Representative: Wulferink, Marty Bernardus Franciscus
(86) International application number: PCT/NL2012/050434
(87) International publication number: WO 2012/177129

(56) References cited:
- MARTIN D M ET AL: "Functional characterization of nine Norway Spruce TPS genes and evolution of gymnosperm terpene synthases of the TPS-d subfamily", PLANT PHYSIOLOGY, AMERICAN SOCIETY OF PLANT PHYSIOLOGISTS, ROCKVILLE, MD, US, vol. 135, no. 4, 1 August 2004 (2004-08-01), pages 1908-1927, XP002579008, ISSN: 0032-0889, DOI: 10.1104/PP.104.042028 [retrieved on 2004-08-13]
- SHARON-ASA L ET AL: "Citrus fruit flavor and aroma biosynthesis: Isolation, functional characterization, and developmental regulation of Cstps1, a key gene in the production of the sesquiterpene aroma compound valencene", THE PLANT JOURNAL, BLACKWELL SCIENTIFIC PUBLICATIONS, OXFORD, GB, vol. 36, no. 5, 1 December 2003 (2003-12-01), pages 664-674, XP002282506, ISSN: 0960-7412, DOI: 10.1046/J.1365-313X.2003.01910.X

## Description

The invention is directed to a valencene synthase, to a nucleic acid encoding said valencene synthase, to an expression vector comprising said nucleic acid, to a host cell comprising said expression vector, to a method of preparing valencene, and to a method of preparing nootkatone.

Many organisms have the capacity to produce a wide array of terpenes and terpenoids. Terpenes are actually or conceptually built up from 2-methylbutane residues, usually referred to as units of isoprene, which has the molecular formula C₅H₈. One can consider the isoprene unit as one of nature's common building blocks. The basic molecular formulae of terpenes are multiples of that formula: (C₅H₈)ₙ, wherein n is the number of linked isoprene units. This is called the isoprene rule, as a result of which terpenes are also denoted as isoprenoids. The isoprene units may be linked together "head to tail" to form linear chains or they may be arranged to form rings. In their biosynthesis, terpenes are formed from the universal 5 carbon precursors isopentenyl diphosphate (IPP) and its isomer, dimethylallyl diphosphate (DMAPP). Accordingly, a terpene carbon skeleton generally comprises a multiple of 5 carbon atoms. Most common are the 5-, 10-, 15-, 20-, 30- and 40-carbon terpenes, which are referred to as hemi-, mono-, sesqui-, di-, tri- and tetraterpenes, respectively. Besides "head-to-tail" connections, tri- and tetraterpenes also contain one "tail-to-tail" connection in their centre. The terpenes may comprise further functional groups, like alcohols and their glycosides, ethers, aldehydes, ketones, carboxylic acids and esters. These functionalised terpenes are herein referred to as terpenoids. Like terpenes, terpenoids generally have a carbon skeleton having a multiple of 5 carbon atoms. It should be noted that the total number of carbons in a terpenoid does not need to be a multiple of 5, *e.g.* the functional group may be an ester group comprising an alkyl group having any number of carbon atoms.

Apart from the definitions given above, it is important to note that the terms "terpene", "terpenoid" and "isoprenoid" are frequently used interchangeably in open as well as patent literature.

Valencene is a naturally occurring terpene, produced in specific plants, such as various citrus fruits. In these plants farnesyl diphosphate (FPP) is enzymatically converted into valencene in the presence of a valencene synthase.

Valencene is, e.g., industrially applicable as an aroma or flavour. Valencene can be obtained by distillation from citrus essential oils obtained from citrus fruits, but isolation from these oils is cumbersome because of the low valencene concentration in these fruits (0.2 to 0.6% by weight).

It has been proposed to prepare valencene microbiologically, making use of micro-organisms genetically modified by incorporation of a gene that is coding for a protein having valencene synthase activity. Thus produced valencene synthase can be used for the preparation of valencene from FPP, a conversion which might be executed as an isolated reaction (*in vitro*) or as part of a longer metabolic pathway eventually leading to the production of valencene from sugar (*in vivo*).

Several valencene synthases from citrus are known. For instance, in US 7,273,735 and US 7,442,785 the expression of valencene synthase from *Citrus x paradisi* in *E. coli* is described. Further, valencene synthase from *Vitis vinifera* has been described by Lücker et al. (Phytochemistry (2004) 65: 2649-2659). Although the expression of these valencene synthases in a host organism has been described, the actual enzymatic activity is only shown under *in vitro* conditions.

A number of papers also describe the activity of valencene synthases *in vivo*. Takahashi et al. (Biotechnol. Bioeng. (2007) 97: 17-181), for instance, report the expression of a *Citrus x paradisi* valencene synthase gene (accession number AF411120) in *Saccharomyces cerevisiae* strains that have been optimized for enhanced levels of the key intermediate FPP by amongst other things inactivating the *ERG9* gene through a knockout mutation. Cultivation of the best strain in a defined minimal medium containing ergosterol to complement the *ERG9* mutation for 216 h led to production of 20 mg/L valencene. Asadollahi et al. (Biotechnol. Bioeng. (2008) 99: 666-677) describe a rather similar valencene production system, which is based on the expression of a *Citrus x paradisi* valencene synthase gene (accession number CQ813508; 3 out of 548 amino acids difference compared to AF411120) in a *S. cerevisiae* strain in which the expression of the *ERG9* gene was downregulated via replacement of the native *ERG9* promoter with the regulatable *MET3* promoter. Cultivation of this strain in a minimal medium applying a two-liquid phase fermentation with dodecane as the organic solvent resulted in the formation of 3 mg/L valencene in 60 h.

The currently known valencene synthases have a number of distinct drawbacks which are in particular undesirable when they are applied in an industrial valencene production process wherein valencene is prepared from FPP, either in an isolated reaction (*in vitro*), *e.g.* using an isolated valencene synthase or (permeabilized) whole cells, or otherwise, *e.g*. in a fermentative process being part of a longer metabolic pathway eventually leading to the production of valencene from sugar (*in vivo*). Internal research by the present inventors revealed, for instance, that overexpression of the valencene synthase from *Citrus x paradisi* (CQ813508) or from *Citrus sinensis* (AF441124) in different microorganisms (*E. coli, Rhodobacter sphaeroides, Saccharomyces cerevisiae*) in active form is troublesome, resulting in a severely impaired production rate of valencene. Similarly, Asadollahi et al. (Biotechnol. Bioeng. (2008) 99: 666-677) found that the low valencene synthesis in a recombinant *S. cerevisiae* strain was caused by poor heterologous expression of the *Citrus x paradisi* valencene synthase gene.

Moreover, the *C. x paradisi* valencene synthase, which is nearly identical to the enzyme from C. *sinensis,* has been found to catalyse the conversion of FPP not only into valencene but also into significant amounts of germacrene A (US 7,442,785 B2), at neutral or mildly alkaline pH.

An incubation of this enzyme with FPP at pH 7.5, for instance, resulted in the formation of two compounds accounting for over 95% of the total reaction products formed, 30% of which was beta-elemene (a thermal rearrangement product of germacrene A) and 65% of which was valencene. The inventors further found that also under *in vivo* conditions, significant amounts of the germacrene A side product are formed by this enzyme; cultivation of a *Rhodobacter sphaeroides* strain optimised for isoprenoids production and carrying the *C. x paradisi* valencene synthase gene (accession number CQ813508) led to the formation of valencene and germacrene A (analysed by GC as beta-elemene) in 48% and 25% of the total amount of sesquiterpenes formed, respectively.

The valencene synthase from grapevine *Vitis vinifera* (accession number AAS66358) displays a similar lack of specificity. Expression in *E. coli* followed by an *in vitro* enzyme assay showed that this synthase converts FPP into (+)-valencene (49.5% of total product) and (-)-7-epi-α-selinene (35.5% of total product) along with five minor products (Lücker et al. Phytochemistry (2004) 65: 2649-2659).

Besides the above enzymes with biochemically proven valencene synthase activity, the GenBank nucleic acid sequence database contains yet another entry annotated as a valencene synthase, i.e. the *Perilla frutescens* var. *frutescens* valencene synthase gene (accession number AY917195). In literature, however, nothing has been reported on this specific putative valencene synthase, so a biochemical proof for its activity and specificity is lacking.

Thus, there is a need for an alternative valencene synthase which may be used in the preparation of valencene. In particular there is a need for an alternative valencene synthase that displays an improved expression, at least in selected host cells; an alternative valencene synthase that has a high enzymatic activity at least under specific conditions, such as at a neutral or alkaline pH and/or intracellularly in the cell wherein it has been produced; and/or an alternative valencene synthase that is highly specific, in particular that has improved specificity compared to valencene synthase from *Citrus x paradisi*, with respect to catalysing the conversion of FPP into valencene, at least under specific conditions, such as at about neutral or at alkaline pH and/or intracellularly in the cell wherein it has been produced.

It has been found that a specific polypeptide that was hitherto unknown has valencene synthase activity and that this polypeptide can be used as a catalyst that may serve as an alternative to known valencene synthases. In particular such enzyme has improved specificity and/or productivity compared to valencene synthase from *Citrus x paradisi*, with respect to catalysing the conversion of FPP into valencene.

Accordingly, a valencene synthase comprising an amino acid sequence as shown in SEQ ID NO: 2, or a functional homologue thereof, said functional homologue being a valencene synthase comprising an amino acid sequence which has a sequence identity of at least 40 %, preferably of at least 50 % with SEQ ID NO: 2. Said homologue may in particular be a valencene synthase comprising an amino acid sequence which has a sequence identity of at least 55 %, at least 60 %, at least 70 %, at least 75 %, at least 80 %, at least 85 %, at least 90 %, at least 95 %, at least 98 % or at least 99 % with SEQ ID NO: 2 is described.

The inventors further found that it is possible to provide a valencene synthase with improved productivity towards the conversion of farnesyl diphosphate into valencene, compared to a valencene synthase having the amino acid sequence of SEQ ID NO: 2.

Accordingly, a valencene synthase having an increased productivity towards the conversion of farnesyl diphosphate into valencene (expressed as molar amount of valencene formed per hour under test conditions) compared to a valencene synthase represented by SEQ ID NO:2 is described.

Further, a valencene synthase comprising an amino acid sequence represented by SEQ ID NO:3, provided that at least one position marked 'X' in SEQ ID NO:3 is different from the corresponding position in SEQ ID NO: 2.. In a preferred embodiment, said valencene synthase comprises an amino acid sequence as shown in SEQ ID NO:4 is described, provided that this sequence comprises at least one difference in the amino acid sequence, compared to SEQ ID NO: 2.

In one aspect, the invention provides a valencene synthase comprising an amino acid sequence having at least 85 % sequence identity with SEQ ID NO: 2, wherein
the valencene synthase has one or more modifications, in particular one or more substitutions, compared to the valence synthase represented by SEQ ID NO: 2, at an amino acid position corresponding to a position selected from the group of 16A, 16T, 16S, 128L, 171R, 187K, 225S, 244S. 244T, 300Y, 302D, 307T, 307A, 319Q, 323A, 327L, 331G, 334L, 398I, 398M, 398T, 405T, 405V, 409F, 410F, 410V, 410L, 412G, 436L, 436K, 436T, 436W, 438T, 439G, 439A, 444I, 444V, 448S, 449F, 449I, 449Y, 450L, 450M, 450V, 463E, 463S, 463G, 463W, 488Y, 488H, 488S, 490N, 490A, 490T, 490F, 492A, 492K, 502Q, 503S, 507E, 507Q, 527T, 527S, 527A, 556T, 559H, 559L, 559V, 560L, 566S, 566A, 566G, 568S, 569I, 569V,570T, 570G, 570A and 570P,and wherein
the valence synthase has an increased productivity towards the conversion of farnesyl diphosphate into valencene (expressed as molar amount of valencene formed per hour) compared to a valencene synthase represented by SEQ ID NO: 2.

Further, the invention relates to an antibody having specific binding affinity to a valencene synthase according to the invention. An antibody according to the invention thus specifically binds to a valencene synthase according to the invention.

Further, the invention relates to a protein displaying immunological cross-reactivity with an antibody raised against a fragment of the valencene synthase of the invention, in particular such a protein having valencene synthase activity.

The immunological cross reactivity may be assayed using an antibody raised against, or reactive with, at least one epitope of an isolated polypeptide according to the present invention having valencene synthase activity. The antibody, which may either be monoclonal or polyclonal, may be produced by methods known in the art, *e.g.* as described by Hudson et al., Practical Immunology, Third Edition (1989), Blackwell Scientific Publications. The immunochemical cross-reactivity may be determined using assays known in the art, an example of which is Western blotting, *e.g.* as described in Hudson et al., Practical Immunology, Third Edition (1989), Blackwell Scientific Publications.

The invention further relates to a nucleic acid, comprising a nucleic acid sequence encoding a valencene synthase according to the invention, in particular a nucleic acid sequence encoding a valencene synthase comprising an amino acid sequence as shown in SEQ ID NO: 2, 3 or 4 or a functional homologue thereof, or comprising a nucleic acid sequence complementary to said encoding sequence. In particular, the nucleic acid may be selected from nucleic acids comprising a nucleic acid sequence as shown in SEQ ID NO: 1, and other nucleic acid sequences encoding a valencene synthase according to the invention, said other sequences comprising a nucleic acid sequence having a sequence identity of at least 50 %, in particular of at least 60 %, at least 70 %, at least 80 %, at least 85 %, at least 90 %, at least 95 %, at least 98 % or at least 99 % with the nucleic acid sequence shown in SEQ ID NO: 1, or nucleic acid sequences encoding a valencene synthase comprising a sequence as shown in SEQ ID NO: 3 or 4, respectively nucleic acids complementary thereto. Said other nucleic acid sequence encoding a valencene synthase according to the invention may herein after be referred to as a functional analogue.

The present invention also relates to a nucleic acid, comprising a nucleic acid sequence encoding a valencene synthase according to the invention, which hybridizes under low stringency conditions, preferably under medium stringency conditions, more preferably under high stringency conditions and most preferably under very high stringency conditions with the nucleic acid sequence shown in SEQ ID NO: 1, or a nucleic acid sequence encoding an amino acid sequence as shown in SEQ ID NO: 3 or 4, respectively nucleic acids complementary thereto.

Hybridization experiments can be performed by a variety of methods, which are well available to the skilled man. General guidelines for choosing among these various methods can be found in e.g. Sambrook, J., and Russell, D.W. Molecular Cloning: A Laboratory Manual.3d ed., Cold Spring Harbour Laboratory Press, Cold Spring Harbour, NY, (2001).

With stringency of the hybridization conditions is meant, the conditions under which the hybridization, consisting of the actual hybridization and wash steps, are performed. Wash steps are used to wash off the nucleic acids, which do not hybridize with the target nucleic acid immobilized on for example a nitrocellulose filter. The stringency of the hybridization conditions can for example be changed by changing the salt concentration of the wash solution and/or by changing the temperature under which the wash step is performed (wash temperature). Stringency of the hybridization increases by lowering the salt concentration in the wash solution or by raising the wash temperature. For purpose of this application, low, medium, high and very high stringency conditions are in particular the following conditions and equivalents thereof: the hybridization is performed in an aqueous solution comprising 6 X SSC (20 X SSC stock solution is 3.0 M NaCl and 0.3 M trisodium citrate in water), 5 X Denhardt's reagent (100 X Denhardt's reagent is 2% (w/v) BSA Fraction V, 20% (w/v) Ficoll 400 and 2% (w/v) polyvinylpyrrollidone in water), 0.5% (w/v) SDS and 100 µg/mL denaturated, fragmented salmon sperm DNA, at about 45°C for about 12 hours. After removal of non-bonded nucleic acid probe by two consecutive 5 minutes wash steps in 2 X SSC, 0.1% (w/v) SDS at room temperature, execution of two consecutive 5 minutes wash steps in 0.2 X SSC, 0.1% (w/v) SDS at room temperature is an example of low stringency, of two consecutive 15 minutes wash steps in 0.2 X SSC, 0.1% (w/v) SDS at 42°C an example of medium stringency, of two consecutive 15 minutes wash steps in 0.1 X SSC, 0.1% (w/v) SDS at 55°C an example of high stringency, and two consecutive 30 minutes wash steps in 0.1 X SSC, 0.1% (w/v) SDS at 68°C an example of very high stringency.

A valencene synthase or nucleic acid according to the invention may be a natural compound or fragment of a compound isolated from its natural source, be a chemically or enzymatically synthesised compound or fragment of a compound or a compound or fragment of a compound produced in a recombinant cell, in which recombinant cell it may be present or from which cell it may have been isolated.

The invention further relates to an expression vector comprising a nucleic acid according to the invention.

The invention further relates to a host cell, comprising an expression vector according to the invention.

The invention further relates to a method for preparing valencene, comprising converting FPP to valencene in the presence of a valencene synthase according to the invention. Four different geometric isomers of FPP can exist, *i.e.* 2*E*,6*E*-FPP, 2*Z*,6*E*-FPP, 2*E*,6*Z*-FPP, and 2Z,6Z-FPP. Good results have been obtained with 2E,6E-FPP, although in principle any other isomer of FPP may be a suitable substrate for an enzyme according to the invention.

The invention further relates to a method for preparing nootkatone, wherein valencene prepared in a method according to the invention is converted into nootkatone.

The invention is further directed to a method for producing a valencene synthase according to the invention, comprising culturing a host cell according to the invention under conditions conducive to the production of the valencene synthase and recovering the valencene synthase from the host cell.

In accordance with the invention it has been found possible to bring a valencene synthase to expression with good yield in distinct organisms. A valencene synthase (represented by Sequence ID NO: 2) has been found to be expressed well in *E. coli* and in *Saccharomyces cerevisiae* (baker's yeast). Also it has been found that in a method according to the invention wherein a valencene synthase according to the invention is expressed in an isoprenoid producing host organism *(Rhodobacter sphaeroides*) the valencene production is higher than in a comparative method wherein a citrus valencene synthase is expressed.

Of a valencene synthase according to SEQ ID NO: 2 it has been found that it is more specific towards valencene synthesis than a valencene synthase from citrus, in particular at or around neutral pH in an *in vitro* assay or in a method wherein valencene is synthesised intracellularly in a host cell genetically modified to produce a valencene synthase according to the invention and a citrus valencene synthase, respectively. Initial results show that under identical conditions, the amount of major side product (germacrene A) formed with the novel enzyme of the invention is significantly lower, namely a molar ratio valencene:germacrene A of 4:1 compared to 2:1 with the citrus valencene synthase. Without being bound by theory, it is thought that a high specificity towards the catalysis of valencene synthesis at neutral or mildly alkaline pH is in particular considered desirable for methods wherein the valencene is prepared intracellularly, because various host cells are thought to have a neutral or slightly alkaline intracellular pH, such as a pH of 7.0-8.5 (for intracellular pH values of bacteria, see for instance: Booth, Microbiological Reviews (1985) 49: 359-378). When, for instance, *E. coli* cells were exposed to pH values ranging from 5.5 to 8.0, the intracellular pH was between 7.1 and 7.9 (Olsen et al., Appl. Environ. Microbiol. (2002) 68: 4145-4147). This may explain an improved specificity towards the synthesis of valencene of a valencene synthase according to the invention, also intracellularly.

The productivity of the valencene synthase according to the current invention may be at least 1.1 times, at least 1.5 times, at least 2.0 times or at least 2.5 times the productivity of the valencene synthase represented by SEQ ID NO: 2. The productivity may be up to 100 times higher, or even more. In a specific embodiment, the productivity is up to 10 times higher, in particular up to 5 times higher, more in particular up to 4 times higher.

A valencene synthase according to the invention having increased productivity compared to a valencene synthase according to SEQ ID NO: 2 may in particular have an increased specific productivity, increased stability (reduced deactivation rate of the enzyme), increased product specificity (relative to the conversion of farnesyl diphosphate into Germacrene A) or an increased expression in a host cell, compared to a valencene synthase represented by SEQ ID NO: 2.

The specific productivity of the valencene synthase having increased productivity compared to the valencene synthase according to SEQ ID NO: 2 usually is about the same or higher than the specific productivity of the valencene synthase according to SEQ ID NO: 2, expressed as the molar amount of valencene formed per hour per amount of enzyme. Preferably the specific productivity is at least 1.1 times the specific productivity of the valencene synthase according to SEQ ID NO: 2, more preferably at least 1.5 times, in particular at least 2.0 times, more in particular at least 2.5 times. The upper limit is not critical. The specific productivity of the valencene synthase having increased productivity may be up to 100 times the specific productivity of the valencene synthase of SEQ ID NO: 2, but it may be higher. In a specific embodiment, the specific productivity is up to 10 times, in particular 5 times or less, more in particular 4 times or less, the specific productivity of the valencene synthase represented by SEQ ID NO: 2.

The product specificity, expressed as the molar ratio valencene formed from farnesyl diphospate to Germacrene A formed from farnesyl diphosphate (under test conditions specified herein), usually is 6 or more, preferably 10 or more, more preferably at least 13, in particular at least 15. The product specificity may be up to 1000, although it may be higher than 1000. In a specific embodiment, the product specificity is 100 or less, in particular 30 or less, more in particular 25 or less, or 22 or less.

As used herein 'productivity', is defined as the molar amount of reaction product, more specifically valencene, formed from the substrate, more specifically farnesyl diphosphate per unit of time, more specifically hour. The 'productivity' of the valencene synthase according to the invention is determined under standard, well controllable, conditions, preferably in an *in vitro* assay, as described in Example 2. To this end valencene synthases according to the invention were incubated with a surplus of farnesyl diphosphate at 30°C and buffered at pH 7.0-7.5 for 2 hours, after which the reaction was stopped by extraction with ethylacetate. Because the valencene synthases were assayed in the form of a cleared lysate, Na-orthovanadate was added to inhibit phosphatises and thus to prevent undesired hydrolysis of farnesyl diphosphate. The reaction conditions indicated in example 2 (including the amount of cleared lysate, the amount of farnesyl diphosphate and the reaction time) were chosen such that the amount of product formed was still linearly dependent on the amount of lysate.

As used herein 'specific productivity' for valencene production, is defined as the molar amount of valencene formed from the substrate farnesyl diphosphate per unit of time (hour) per amount of valencene synthase. It is thus the 'productivity' as defined above per amount of valencene synthase, and is thus meant to compensate for the presence of different amounts of valencene synthase in the enzyme preparations used to determine the 'productivity'. In case a pure valencene synthase preparation is assayed, a standard total protein quantification method (e.g. the Bradford method [Bradford M.M, Anal. Biochem. 1976, 72: 248-254]) can be used to quantify the amount of valencene synthase. In case a non-pure valencene synthase preparation is used in the valencene productivity determination, a method that discriminates the valencene synthase from the other proteins present in the preparation must be applied. Example 2 describes such method that is based on the separation of the proteins on an SDS-PAGE gel combined with their quantification via staining of the protein bands with a fluorescent dye followed by their imaging.

As used herein 'stability', is defined as 'operational stability', meaning the stability of the valencene synthase under the conditions applied to convert the substrate farnesyl diphosphate into the reaction product valencene. Because a reaction time of 2 hours is used in the 'productivity' determination method described in example 2, this method also intrinsically determines the operational stability of the valencene synthase according to the invention under the *in vitro* conditions applied, because instable enzymes will cease to act more rapidly than more stable ones, and will thus lead to less product formation and thus decreased productivity.

As used herein 'product specificity, is defined as the molar ratio of valencene to germacrene A formed from farnesyl diphosphate in the standard productivity assay described in example 2. In case a valencene synthase according to the invention would produce significant amounts of other sesquiterpene-like side products, the product specificity is defined as the molar ratio of valencene to the sum of the other sesquiterpenes formed from farnesyl diphosphate in the standard productivity assay described in example 2.

As used herein 'expression in a host cell', is defined as the amount of the valencene synthase according to the invention formed in a specific host cell relative to the total amount of proteins formed in this host cell. A method that is typically used for the determination of the expression level of a specific protein is based on separation of all proteins present *(e.g.* in the cleared lysate) on an SDS-PAGE gel followed by their staining and subsequent quantification. The expression level (in %) is then defined as the relative intensity of the band belonging to the protein of interest compared to the sum of intensities of all bands on the gel multiplied by 100%.

Because of the fact that the valencene synthase according to the invention was analysed as a cleared lysate, both the 'specific productivity' and the 'expression in a host cell' were determined relative to a standard protein, i.e. the valencene synthase of SEQ ID NO: 2.

The term "or" as used herein is defined as "and/or" unless specified otherwise.

The term "a" or "an" as used herein is defined as "at least one" unless specified otherwise.

When referring to a noun (e.g. a compound, an additive, etc.) in the singular, the plural is meant to be included.

The terms farnesyl diphosphate and farnesyl pyrophosphate (both abbreviated as FPP) as interchangeably used herein refer to the compound 3,7,11-trimethyl-2,6,10-dodecatrien-1-yl pyrophosphate and include all known isomers of this compound.

The term "recombinant" in relation to a recombinant cell, vector, nucleic acid or the like as used herein, refers to a cell, vector, nucleic acid or the like, containing nucleic acid not naturally occurring in that cell, vector, nucleic acid or the like and/or not naturally occurring at that same location. Generally, said nucleic acid has been introduced into that strain (cell) using recombinant DNA techniques.

The term "heterologous" when used with respect to a nucleic acid (DNA or RNA) or protein refers to a nucleic acid or protein that does not occur naturally as part of the organism, cell, genome or DNA or RNA sequence in which it is present, or that is found in a cell or location or locations in the genome or DNA or RNA sequence that differ from that in which it is found in nature. Heterologous nucleic acids or proteins are not endogenous to the cell into which they are introduced, but have been obtained from another cell or synthetically or recombinantly produced. Generally, though not necessarily, such nucleic acids encode proteins that are not normally produced by the cell in which the DNA is expressed.

A gene that is endogenous to a particular host cell but has been modified from its natural form, through, for example, the use of DNA shuffling, is also called heterologous. The term "heterologous" also includes non-naturally occurring multiple copies of a naturally occurring DNA sequence. Thus, the term "heterologous" may refer to a DNA segment that is foreign or heterologous to the cell, or homologous to the cell but in a position and/or a number within the host cell nucleic acid in which the segment is not ordinarily found. Exogenous DNA segments are expressed to yield exogenous polypeptides. A "homologous" DNA sequence is a DNA sequence that is naturally associated with a host cell into which it is introduced.

Any nucleic acid or protein that one of skill in the art would recognize as heterologous or foreign to the cell in which it is expressed is herein encompassed by the term heterologous nucleic acid or protein.

The terms "modified", "modification", "mutated", "mutation", or "variant" as used herein regarding proteins or polypeptides compared to another protein or peptide (in particular compared to the polypeptide consisting of amino acids in the sequence shown in SEQ ID NO: 2), is use to indicate that the modified protein or polypeptide has at least one difference in the amino acid sequence compared tot the protein or polypeptide with which it is compared, *e.g*. a wild-type protein/polypeptide. The terms are used irrespective of whether the modified/mutated protein actually has been obtained by mutagenesis of nucleic acids encoding these amino acids or modification of the polypeptide/protein or in another manner, e.g. using artificial gene-synthesis methodology. Mutagenesis is a well-known method in the art, and includes, for example, site-directed mutagenesis by means of PCR or via oligonucleotide-mediated mutagenesis as described in Sambrook, J., and Russell, D.W. Molecular Cloning: A Laboratory Manual.3d ed., Cold Spring Harbor Laboratory Press, Cold Spring Harbor, NY, (2001). The term "modified", "modification", "mutated", "mutation" or "variant" as used herein regarding genes is used to indicate that at least one nucleotide in the nucleotide sequence of that gene or a regulatory sequence thereof, is different from the nucleotide sequence that it is compared with, *e.g.* a wild-type nucleotide sequence, such as SEQ ID NO: 1. The terms are used irrespective of whether the modified/mutated nucleotide sequence actually has been obtained by mutagenesis.

A modification/mutation may in a particular be a replacement of an amino acid respectively nucleotide by a different one, a deletion of an amino acid respectively nucleotide or an insertion of an amino acid respectively nucleotide.

The terms "open reading frame" and "ORF" refer to the amino acid sequence encoded between translation initiation and termination codons of a coding sequence. The terms "initiation codon" and "termination codon" refer to a unit of three adjacent nucleotides ('codon') in a coding sequence that specifies initiation and chain termination, respectively, of protein synthesis (mRNA translation).

The term "gene" is used broadly to refer to any segment of nucleic acid associated with a biological function. Thus, genes include coding sequences and/or the regulatory sequences required for their expression. For example, gene refers to a nucleic acid fragment that expresses mRNA or functional RNA, or encodes a specific protein, and which includes regulatory sequences. Genes also include non-expressed DNA segments that, for example, form recognition sequences for other proteins. Genes can be obtained from a variety of sources, including cloning from a source of interest or synthesizing from known or predicted sequence information, and may include sequences designed to have desired parameters.

The term "chimeric gene" refers to any gene that contains 1) DNA sequences, including regulatory and coding sequences that are not found together in nature, or 2) sequences encoding parts of proteins not naturally adjoined, or 3) parts of promoters that are not naturally adjoined. Accordingly, a chimeric gene may comprise regulatory sequences and coding sequences that are derived from different sources, or comprise regulatory sequences and coding sequences derived from the same source, but arranged in a manner different from that found in nature.

The term "transgenic" for a transgenic cell or organism as used herein, refers to an organism or cell (which cell may be an organism *per se* or a cell of a multi-cellular organism from which it has been isolated) containing a nucleic acid not naturally occurring in that organism or cell and which nucleic acid has been introduced into that organism or cell *(i.e.* has been introduced in the organism or cell itself or in an ancestor of the organism or an ancestral organism of an organism of which the cell has been isolated) using recombinant DNA techniques.

A "transgene" refers to a gene that has been introduced into the genome by transformation and preferably is stably maintained. Transgenes may include, for example, genes that are either heterologous or homologous to the genes of a particular plant to be transformed. Additionally, transgenes may comprise native genes inserted into a non-native organism, or chimeric genes. The term "endogenous gene" refers to a native gene in its natural location in the genome of an organism. A "foreign" gene refers to a gene not normally found in the host organism but that is introduced by gene transfer.

"Transformation" and "transforming", as used herein, refers to the introduction of a heterologous nucleotide sequence into a host cell, irrespective of the method used for the insertion, for example, direct uptake, transduction, conjugation, f-mating or electroporation. The exogenous polynucleotide may be maintained as a non-integrated vector, for example, a plasmid, or alternatively, may be integrated into the host cell genome.

"Coding sequence" refers to a DNA or RNA sequence that codes for a specific amino acid sequence and excludes the non-coding sequences. It may constitute an "uninterrupted coding sequence", i.e. lacking an intron, such as in a cDNA or it may include one or more introns bound by appropriate splice junctions. An "intron" is a sequence of RNA which is contained in the primary transcript but which is removed through cleavage and re-ligation of the RNA within the cell to create the mature mRNA that can be translated into a protein.

"Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences include enhancers, promoters, translation leader sequences, introns, and polyadenylation signal sequences. They include natural and synthetic sequences as well as sequences which may be a combination of synthetic and natural sequences. As is noted above, the term "suitable regulatory sequences" is not limited to promoters.

Examples of regulatory sequences include promoters (such as transcriptional promoters, constitutive promoters, inducible promoters), operators, or enhancers, mRNA ribosomal binding sites, and appropriate sequences which control transcription and translation initiation and termination. Nucleic acid sequences are "operably linked" when the regulatory sequence functionally relates to the DNA or cDNA sequence of the invention.

Each of the regulatory sequences may independently be selected from heterologous and homologous regulatory sequences.

"Promoter" refers to a nucleotide sequence, usually upstream (5') to its coding sequence, which controls the expression of said coding sequence by providing the recognition for RNA polymerase and other factors required for proper transcription. "Promoter" includes a minimal promoter that is a short DNA sequence comprised of a TATA box and other sequences that serve to specify the site of transcription initiation, to which regulatory elements are added for control of expression. "Promoter" also refers to a nucleotide sequence that includes a minimal promoter plus regulatory elements that is capable of controlling the expression of a coding sequence or functional RNA. This type of promoter sequence consists of proximal and more distal upstream elements, the latter elements often referred to as enhancers. Accordingly, an "enhancer" is a DNA sequence which can stimulate promoter activity and may be an innate element of the promoter or a heterologous element inserted to enhance the level or tissue specificity of a promoter. It is capable of operating in both orientations (normal or flipped), and is capable of functioning even when moved either upstream or downstream from the promoter. Both enhancers and other upstream promoter elements bind sequence-specific DNA-binding proteins that mediate their effects. Promoters may be derived in their entirety from a native gene, or be composed of different elements derived from different promoters found in nature, or even be comprised of synthetic DNA segments. A promoter may also contain DNA sequences that are involved in the binding of protein factors which control the effectiveness of transcription initiation in response to physiological or developmental conditions.

The term "nucleic acid" as used herein, includes reference to a deoxyribonucleotide or ribonucleotide polymer, i.e. a polynucleotide, in either single-or double-stranded form, and unless otherwise limited, encompasses known analogues having the essential nature of natural nucleotides in that they hybridize to single-stranded nucleic acids in a manner similar to naturally occurring nucleotides (e.g., peptide nucleic acids). A polynucleotide can be full-length or a subsequence of a native or heterologous structural or regulatory gene. Unless otherwise indicated, the term includes reference to the specified sequence as well as the complementary sequence thereof. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are "polynucleotides" as the term is used herein. It will be appreciated that a great variety of modifications have been made to DNA and RNA that serve many useful purposes known to those of skill in the art. The term "polynucleotide" as it is employed herein embraces such chemically, enzymatically or metabolically modified forms of polynucleotides, as well as the chemical forms of DNA and RNA characteristic of viruses and cells, including among other things, simple and complex cells.

Every nucleic acid sequence herein that encodes a polypeptide also, by reference to the genetic code, describes every possible silent variation of the nucleic acid. The term "conservatively modified variants" applies to both amino acid and nucleic acid sequences. With respect to particular nucleic acid sequences, the term "conservatively modified variants" refers to those nucleic acids which encode identical or conservatively modified variants of the amino acid sequences due to the degeneracy of the genetic code. The term "degeneracy of the genetic code" refers to the fact that a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" and represent one species of conservatively modified variation. The terms "polypeptide", "peptide" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The essential nature of such analogues of naturally occurring amino acids is that, when incorporated into a protein, that protein is specifically reactive to antibodies elicited to the same protein but consisting entirely of naturally occurring amino acids. The terms "polypeptide", "peptide" and "protein" are also inclusive of modifications including, but not limited to, glycosylation, lipid attachment, sulphation, gamma-carboxylation of glutamic acid residues, hydroxylation and ADP-ribosylation.

Within the context of the present application, oligomers (such as oligonucleotides, oligopeptides) are considered a species of the group of polymers. Oligomers have a relatively low number of monomeric units, in general 2-100, in particular 6-100.

"Expression cassette" as used herein means a DNA sequence capable of directing expression of a particular nucleotide sequence in an appropriate host cell, comprising a promoter operably linked to the nucleotide sequence of interest which is operably linked to termination signals. It also typically comprises sequences required for proper translation of the nucleotide sequence. The coding region usually codes for a protein of interest but may also code for a functional RNA of interest, for example antisense RNA or a non-translated RNA, in the sense or antisense direction. The expression cassette comprising the nucleotide sequence of interest may be chimeric, meaning that at least one of its components is heterologous with respect to at least one of its other components. The expression cassette may also be one which is naturally occurring but has been obtained in a recombinant form useful for heterologous expression. The expression of the nucleotide sequence in the expression cassette may be under the control of a constitutive promoter or of an inducible promoter which initiates transcription only when the host cell is exposed to some particular external stimulus. In the case of a multicellular organism, the promoter can also be specific to a particular tissue or organ or stage of development.

The term "vector" as used herein refers to a construction comprised of genetic material designed to direct transformation of a targeted cell. A vector contains multiple genetic elements positionally and sequentially oriented, i.e., operatively linked with other necessary elements such that the nucleic acid in a nucleic acid cassette can be transcribed and when necessary, translated in the transformed cells.

In particular, the vector may be selected from the group of viral vectors, (bacterio)phages, cosmids or plasmids. The vector may also be a yeast artificial chromosome (YAC), a bacterial artificial chromosome (BAC) or *Agrobacterium* binary vector. The vector may be in double or single stranded linear or circular form which may or may not be self transmissible or mobilizable, and which can transform prokaryotic or eukaryotic host organisms either by integration into the cellular genome or exist extrachromosomally (*e.g*. autonomous replicating plasmid with an origin of replication). Specifically included are shuttle vectors by which is meant a DNA vehicle capable, naturally or by design, of replication in two different host organisms, which may be selected from actinomycetes and related species, bacteria and eukaryotic (e.g. higher plant, mammalian, yeast or fungal) cells. Preferably the nucleic acid in the vector is under the control of, and operably linked to, an appropriate promoter or other regulatory elements for transcription in a host cell such as a microbial, e.g. bacterial, or plant cell. The vector may be a bi-functional expression vector which functions in multiple hosts. In the case of genomic DNA, this may contain its own promoter or other regulatory elements and in the case of cDNA this may be under the control of an appropriate promoter or other regulatory elements for expression in the host cell.

Vectors containing a nucleic acid according to the invention can be prepared based on methodology known in the art *per se.* For instance use can be made of a cDNA sequence encoding the polypeptide according to the invention operably linked to suitable regulatory elements, such as transcriptional or translational regulatory nucleic acid sequences.

The term "vector" as used herein, includes reference to a vector for standard cloning work ("cloning vector") as well as to more specialized type of vectors, like an (autosomal) expression vector and a cloning vector used for integration into the chromosome of the host cell ("integration vector").

"Cloning vectors" typically contain one or a small number of restriction endonuclease recognition sites at which foreign DNA sequences can be inserted in a determinable fashion without loss of essential biological function of the vector, as well as a marker gene that is suitable for use in the identification and selection of cells transformed with the cloning vector.

The term "expression vector" refers to a DNA molecule, linear or circular, that comprises a segment encoding a polypeptide of interest under the control of *(i.e.* operably linked to) additional nucleic acid segments that provide for its transcription. Such additional segments may include promoter and terminator sequences, and may optionally include one or more origins of replication, one or more selectable markers, an enhancer, a polyadenylation signal, and the like. Expression vectors are generally derived from plasmid or viral DNA, or may contain elements of both. In particular an expression vector comprises a nucleotide sequence that comprises in the 5' to 3' direction and operably linked: (a) a transcription and translation initiation region that are recognized by the host organism, (b) a coding sequence for a polypeptide of interest, and (c) a transcription and translation termination region that are recognized by the host organism. "Plasmid" refers to autonomously replicating extrachromosomal DNA which is not integrated into a microorganism's genome and is usually circular in nature.

An "integration vector" refers to a DNA molecule, linear or circular, that can be incorporated into a microorganism's genome and provides for stable inheritance of a gene encoding a polypeptide of interest. The integration vector generally comprises one or more segments comprising a gene sequence encoding a polypeptide of interest under the control of (*i.e.*, operably linked to) additional nucleic acid segments that provide for its transcription. Such additional segments may include promoter and terminator sequences, and one or more segments that drive the incorporation of the gene of interest into the genome of the target cell, usually by the process of homologous recombination. Typically, the integration vector will be one which can be transferred into the target cell, but which has a replicon which is non-functional in that organism. Integration of the segment comprising the gene of interest may be selected if an appropriate marker is included within that segment.

As used herein, the term "operably linked" or "operatively linked" refers to a juxtaposition wherein the components so described are in a relationship permitting them to function in their intended manner. A control sequence "operably linked" to another control sequence and/or to a coding sequence is ligated in such a way that transcription and/or expression of the coding sequence is achieved under conditions compatible with the control sequence. Generally, operably linked means that the nucleic acid sequences being linked are contiguous and, where necessary to join two protein coding regions, contiguous and in the same reading frame.

The term "valencene synthase" is used herein for polypeptides having catalytic activity in the formation of valencene from farnesyl diphosphate, and for other moieties comprising such a polypeptide. Examples of such other moieties include complexes of said polypeptide with one or more other polypeptides, other complexes of said polypeptides (e.g. metalloprotein complexes), macromolecular compounds comprising said polypeptide and another organic moiety, said polypeptide bound to a support material, *etc.* The valencene synthase can be provided in its natural environment, *i.e*. within a cell in which it has been produced, or in the medium into which it has been excreted by the cell producing it. It can also be provided separate from the source that has produced the polypeptide and can be manipulated by attachment to a carrier, labeled with a labeling moiety, and the like.

The term "functional homologue" of a sequence, or in short "homologue", as used herein, refers to a polypeptide comprising said specific sequence with the proviso that one or more amino acids are substituted, deleted, added, and/or inserted, and which polypeptide has (qualitatively) the same enzymatic functionality for substrate conversion, *i.e*. a homologue of the sequence with SEQ ID NO: 2 having catalytic activity in the formation of valencene from farnesyl diphosphate. In Example 2 a test is described that is suitable to verify whether a polypeptide or a moiety comprising a polypeptide has catalytic activity in the formation of valencene from farnesyl diphosphate ("Determination of (specific) productivity and product specificity of valencene synthase mutants"). Moreover, the skilled artisan recognises that equivalent nucleotide sequences encompassed by this invention can also be defined by their ability to hybridize, under low, moderate and/or stringent conditions, with the nucleotide sequences that are within the literal scope of the instant claims.

A preferred valencene synthase according to the invention has a specificity towards catalysis of valencene formation, expressed as the molar ratio valencene to germacrene A (a known side-product, formed in known valencene synthase catalysed reactions) of at least 3:1, in particular of at least 4:1, when determined at pH 7, using the "Determination of (specific) productivity and product specificity of valencene synthase mutants"-test described herein below in Example 2 (using a cleared lysate containing the valencene synthase according to the invention; however, the same test set-up can also be applied using a purified polypeptide). Said ratio may be infinite (1:0; *i.e.* no detectible amount of germacrene A formed), or up to 100:1, or up to 10:1 or up to 5:1.

Sequence identity, homology or similarity is defined herein as a relationship between two or more polypeptide sequences or two or more nucleic acid sequences, as determined by comparing those sequences. Usually, sequence identities or similarities are compared over the whole length of the sequences, but may however also be compared only for a part of the sequences aligning with each other. In the art, "identity" or "similarity" also means the degree of sequence relatedness between polypeptide sequences or nucleic acid sequences, as the case may be, as determined by the match between such sequences. Sequence identity as used herein is the value as determined by the EMBOSS Pairwise Alignment Algoritm "Needle". In particular, the NEEDLE program from the EMBOSS package can be used (version 2.8.0 or higher, EMBOSS: The European Molecular Biology Open Software Suite-Rice, P., et al. Trends in Genetics (2000) 16: 276-277; http://emboss.bioinformatics.nl/) using the NOBRIEF option ('Brief identity and similarity' to NO) which calculates the "longest-identity".

The identity, homology or similarity between the two aligned sequences is calculated as follows: Number of corresponding positions in the alignment showing an identical amino acid in both sequences divided by the total length of the alignment after subtraction of the total number of gaps in the alignment. For alignment of amino acid sequences the default parameters are: Matrix = Blosum62; Open Gap Penalty = 10.0; Gap Extension Penalty = 0.5. For alignment of nucleic acid sequences the default parameters are: Matrix = DNAfull; Open Gap Penalty = 10.0; Gap Extension Penalty = 0.5. Discrepancies between a valencene synthase according to SEQ ID NO: 2 or a nucleic acid according to SEQ ID NO: 1 on hand and a functional homologue of said valencene synthase may in particular be the result of modifications performed, e.g. to improve a property of the valencene synthase or nucleic acid (e.g. improved expression) by a biological technique known to the skilled person in the art, such as e.g. molecular evolution or rational design or by using a mutagenesis technique known in the art (random mutagenesis, site-directed mutagenesis, directed evolution, gene recombination, *etc.*)*.* The valencene synthase's or the nucleic acid's sequence may be altered compared to the sequences of SEQ ID NO: 2 and SEQ ID NO: 1, respectively, as a result of one or more natural occurring variations. Examples of such natural modifications/variations are differences in glycosylation (more broadly defined as "post-translational modifications"), differences due to alternative splicing, and single-nucleic acid polymorphisms (SNPs). The nucleic acid may be modified such that it encodes a polypeptide that differs by at least one amino acid from the polypeptide of SEQ ID NO: 2, so that it encodes a polypeptide comprising one or more amino acid substitutions, deletions and/or insertions compared to SEQ ID NO: 2, which polypeptide still has valencene synthase activity. Further, use may be made of artificial gene-synthesis (synthetic DNA). Further, use may be made of codon optimisation or codon pair optimisation, e.g. based on a method as described in WO 2008/000632 or as offered by commercial DNA synthesizing companies like DNA2.0, Geneart, and GenScript.

One or more nucleic acid sequences encoding appropriate signal peptides that are not naturally associated with the polypeptides of the invention can be incorporated into (expression) vectors. For example, a DNA sequence for a signal peptide leader can be fused in-frame to a nucleic acid sequence of the invention so that the polypeptide of the invention is initially translated as a fusion protein comprising the signal peptide. Depending on the nature of the signal peptide, the expressed polypeptide will be targeted differently. A secretory signal peptide that is functional in the intended host cells, for instance, enhances extracellular secretion of the expressed polypeptide. Other signal peptides direct the expressed polypeptides to certain organelles, like the chloroplasts, mitochondria and peroxisomes. The signal peptide can be cleaved from the polypeptide upon transportation to the intended organelle or from the cell. It is possible to provide a fusion of an additional peptide sequence at the amino or carboxyl terminal end of a polypeptide according to SEQ ID NO: 2 or homologue thereof.

The inventors have found various valencene synthases according to the invention that are different from the valencene synthase according to SEQ ID NO: 2 and have increased productivity. The valencene synthase according to the invention may be different in that it only contains a modification at one amino acid position or in that it contains two or more modifications. The one or more modifications may in particular be one or more substitutions. It is also possible that one or more amino acids are absent. For instance one or more amino acids of positions 1-15 or of positions 1-16 of SEQ ID NO: 2 may be absent (residue numbering starting from the NH₂-terminus of the polypeptide).

Compared to the valencene synthase of SEQ ID NO: 2, the valencene synthase with increased productivity, in particular a functional homologue of the valencene synthase of SEQ ID NO: 2, may comprise a modification at various positions of the valencene synthase. A modification may be present at a first shell position of the valencene synthase, or at a second shell position of the valencene synthase, or in a more remote part of the valencene synthase (relative from the substrate binding site). The terms 'first shell' and 'second shell' positions are generally known in the art.

In particular, it can be determined whether an amino acid forms part of the first shell, the second shell, or a more remote part of the valencene synthase, using a model structure of SEQ ID NO: 2. Such model structure was prepared in the homology modelling routine available in the molecular modelling package YASARA Structure version 11.2.18 (http://www.yasara.org/) (the 'YASARA-model'). The template used in the modelling was the structure of 5-epiaristolochene synthase from *Nicotiana tabacum* complexed with (2-cis, 6-trans)-2-fluorofarnesyl diphosphate (2F-FPP) as has been published by Noel J.P., et al., ACS Chem. Biol. (2010) 5: 377-392 (coordinates: PDB ID: 3M02). After loading of this template into the YASARA program, a homology modeling run was executed with the program's modeling speed set to 'slow', and maximum templates and maximum alignments per template set to 1. The other settings were kept at the default of the program.

The first shell positions are defined as the amino acid residues that have at least one non-hydrogen side chain or backbone atom within 7.5 Angstrom of an atom of the bound 2F-FPP in the model structure. A modification, compared to SEQ ID NO: 2, in the first shell is preferably present at a variable first shell position. These are the positions (aligning with) position 301, 303, 307, 310, 331-337, 341, 409, 413, 416, 420, 435, 437-441, 444, 476, 479, 481-484, 486, 487, 491, 552, 553, 556, 557, 560, 569, and 574 of SEQ ID NO: 2.

The second shell positions are defined as the amino acid residues that have at least one non-hydrogen side chain or backbone atom between 7.5 and 15 Angstrom of an atom of the bound 2F-FPP in the model structure, but no side chain or backbone atom within 7.5 Angstrom of an atom of the bound 2F-FPP in the model structure. A modification, compared to SEQ ID NO: 2, in the second shell is preferably present at a variable position. The variable second shell positions are the positions (aligning with) positions 25, 27-32, 34, 225-228, 230, 297-300, 302, 304-306, 308, 309, 311-315, 325-328, 330, 340, 343-346, 350, 353, 354, 357, 375, 378, 379, 382, 383, 402-408, 410-412, 414, 415, 417, 418, 421, 422, 424, 427-434, 436, 442, 443, 445-448, 464, 472-475, 477, 478, 485, 488-490, 492-503, 516, 517, 519-521, 523, 524, 527, 548-551, 554, 555, 558, 559, 561-564, 566-568, 570, 571, 573, 575, 576, 578, and 579 of SEQ ID NO: 2.

A modification in the second shell or in the first shell has in particular been found advantageous to obtain a valencene synthase with an increased productivity, more in particular for obtaining a valencene synthase with an increased specific productivity.

In an advantageous embodiment, the valencene synthase has at least one modification at a position corresponding to a cysteine position in SEQ ID NO: 2. These cysteine positions are the positions (aligning with positions) 16, 225, 244, 323, 327, 405, 503 and 527 of SEQ ID NO: 2.

Without being bound by theory, it is contemplated that an improved productivity in such valencene synthase may partially or fully be the result of an increased enzyme stability, more precisely an increased operational stability (lower tendency to loose catalytic activity over time). The cysteine position in SEQ ID NO: 2 may be a position at which cysteine occurs as a free cysteine or in a disulphide bridge in the YASARA-model of the valencene synthase in SEQ ID NO: 2. These cysteine positions are the positions (aligning with positions) 16, 225, 244, 323, 327, 405, 503, and 527 of SEQ ID NO: 2.

In a preferred embodiment, the valencene synthase according to the invention comprises an amino acid sequence as shown in SEQ ID NO: 3 or a functional homologue thereof. Herein, the positions marked with an 'X' can in principle contain any amino acid residue, with the proviso that preferably at least one amino acid residue is different from the corresponding amino acid residue in SEQ ID NO: 2. In a particularly preferred embodiment, the valencene synthase according to the invention comprises an amino acid sequence as shown in SEQ ID NO: 4 or a functional homologue thereof. Herein, the particularly preferred amino acid residues are given between parenthesis for positions that have been marked with an 'X' in SEQ ID NO: 3. Preferably at least one of these positions has an amino acid residue different from the corresponding amino acid residue in SEQ ID NO: 2.

In a specific embodiment, the valencene synthase with improved productivity, in particular a functional homologue of the valencene synthase of SEQ ID NO: 2, comprises a modification at one or more of the positions aligning with: 87, 93, 128, 171, 178, 187, 226, 302, 312, 319, 323, 398, 436, 448, 449, 450, 463, 488, 492, 502, 507, 530 or 559 of SEQ ID NO: 2.

In a preferred embodiment, the valencene synthase with increased productivity, in particular a functional homologue of the valencene synthase of SEQ ID NO: 2, has one or more modifications, in particular one or more substitutions, compared to the valence synthase represented by SEQ ID NO: 2, at an amino acid position corresponding to a position selected from the group of 16, 128, 171, 187, 225, 244, 300, 302, 307, 319, 323, 327, 331, 334, 398, 405, 409, 410, 412, 436, 438, 439, 444, 448, 449, 450, 463, 488, 490, 492, 502, 503, 507, 527, 556, 559, 560, 566, 568, 569, and 570 of SEQ ID NO: 2. More preferably, the valencene synthase with increased productivity comprises one or more modifications at a position corresponding to (aligning with) one or more positions selected from the group of 16, 225,244, 300, 302, 307, 323, 327, 331, 334, 405, 409, 410, 412, 436, 438, 439, 444, 448, 449, 450, 463, 488, 490, 492, 502, 503, 507, 527, 556, 559, 560, 566, 568, 569, and 570 of SEQ ID NO: 2.

In a particularly preferred embodiment, the valencene synthase has one or more modifications selected from the group of 16A, 16T, 16S, 128L, 171R, 187K, 225S, 244S. 244T, 300Y, 302D, 307T, 307A, 319Q, 323A, 327L, 331G, 334L, 398I, 398M, 398T, 405T, 405V, 409F, 410F, 410V, 410L, 412G, 436L, 436K, 436T, 436W, 438T, 439G, 439A, 444I, 444V, 448S, 449F, 449I, 449Y, 450L, 450M, 450V, 463E, 463S, 463G, 463W, 488Y, 488H, 488S, 490N, 490A, 490T, 490F, 492A, 492K, 502Q, 503S, 507E, 507Q, 527T, 527S, 527A, 556T, 559H, 559L, 559V, 560L, 566S, 566A, 566G, 568S, 569I, 569V,570T, 570G, 570A and 570P.

In particular, good results have been achieved with a valencene synthase having one or more modifications selected from the group of 16A, 16T, 16S, 244S, 244T, 300Y, 307T, 307A, 323A, 327L, 331G, 334L, 405T, 405V, 409F, 410F, 410V, 410L, 412G, 436L, 436K, 436T, 438T, 439G, 439A, 444I, 448S, 449F, 450M, 450L, 450V, 463E, 463S, 463G, 463W, 488Y, 488S, 488H, 490N, 490A, 490T, 490F, 492A, 492K, 502Q, 503S, 507E, 507Q, 527T, 527S, 527A, 556T, 559H, 559L, 559V, 560L, 566S, 566A, 566G, 568S, 569I, 569V, 570T, 570G and 570P.

A particularly high productivity has been observed for a valencene synthase having at least one modification selected from the group of 16A, 244S, 300Y, 307T, 307A, 323A, 327L, 331G, 334L, 405T, 409F, 410F, 410V, 410L, 412G, 436L, 436K, 436T, 438T, 439G, 439A, 449F, 450L, 450V, 488Y, 488H, 488S, 490N, 490A, 490T, 492A, 492K, 502Q, 503S, 507E, 507Q, 527T, 556T, 559H, 559L, 560L, 566S, 566A, 568S, 569I, 569V, 570T and 570G.

Preferred examples of valencene synthases comprising at least two modifications compared to SEQ ID NO: 2 are those wherein at least two modifications are selected from 128L, 187K, 302D, 398I, 398M, 398T, 436L, 436K, 436W, 449F, 449I, 449Y, 450L, 450F, 450V, 463E, 463S, 463G, 463W, 488S, 488Y and 488H. In particular good results have been achieved with a valencene synthase comprising at least two modifications compared to SEQ ID NO: 2, wherein at least two modifications are selected from modifications at at least two positions corresponding to 463, 488, 436, 450, preferably a mutation at a position corresponding to 463 and 488, or 436 and 450. The modifications may in particular be selected from substitutions that are indicated herein above as preferred.

Although good results have been obtained with valencene synthases having only one or two mutations (substitutions) compared to SEQ ID NO: 2, the valence synthase according to the invention may comprise more modifications, in particular three or more, four or more, five or more, six or more or seven or more modifications. In principle there is no limit to the number of modifications, provided that the enzyme retains sufficient catalytic properties as a valencene synthase. However, at least for functional homologues of the valencene synthase, it is generally preferred that the positions aligning with strictly conserved residues in the first and second shell of SEQ ID NO: 2 comprise the same amino acid residue as SEQ ID NO: 2. These are in particular positions 324, 329, 338, 339, 342, 359, 360, 369, 419, 426, 480, 532, 555 and 565 of SEQ ID NO: 2, in order to achieve advantageous productivity properties of the valencene synthase.

Valencene synthase having increased productivity compared to the valencene synthase of SEQ ID NO: 2, preferably is a functional homologue of the valencene synthase of SEQ ID NO: 2. The valencene synthase having increased productivity compared to the valencene synthase of SEQ ID NO: 2, preferably comprises an amino acid sequence having at least 55 %, at least 65 %, at least 75 %, at least 85 %, at least 90 %, at least 95 %, at least 97 %, at least 98 % or at least 99 % sequence identity with SEQ ID NO: 2 with the proviso that the valencene synthase contains at least one modification compared to SEQ ID NO: 2. The valencene synthase may consist of a polypeptide having such sequence identity with SEQ ID NO: 2. However, it is also possible that the valencene synthase comprises at least one segment having such sequence identity and at least one further peptide segment, such as a tag peptide.

Thus, in a specific embodiment, the invention relates to a valencene synthase, wherein the valencene synthase comprises a first polypeptide segment and a second polypeptide segment, the first segment comprising a tag-peptide and the second segment comprising a polypeptide according to the invention having valencene synthase activity.

The tag-peptide is preferably selected from the group of nitrogen utilization proteins (NusA), thioredoxins (Trx) and maltose-binding proteins (MBP). Moreover small peptides with large net negative charge, as have been described by Zhang, Y-B, et al., Protein Expression and Purification (2004) 36: 207-216, can be used as tag-peptide. Particularly suitable is maltose binding protein from *Escherichia coli.* The tag may in particular improve productivity of the enzyme, by increasing the expression of valencene synthase in active form. Preferably, a valencence synthase according to the invention having a tag-peptide segment has an increased specific productivity, increased stability or an increased product specificity (relative to the conversion of farnesyl diphosphate into Germacrene A), compared to a valencene synthase represented by SEQ ID NO: 2, in particular if the tag-peptide is selected from the group of nitrogen utilization proteins (NusA), thioredoxins (Trx) and maltose-binding proteins (MBP).

For improved solubility of the tagged valencene synthase (compared to the valencene synthase without the tag), the first segment of the enzyme is preferably bound at its C-terminus to the N-terminus of the second segment. Alternatively, the first segment of the tagged enzyme is bound at its N-terminus to the C-terminus of the second segment.

The invention further relates to a host cell comprising a vector according to the invention. By "host cell" is meant a cell which contains a vector and supports the replication and/or expression of the vector.

The nucleic acid encoding the valencene synthase in a host cell is generally heterologous to the host cell. The host cell may be a prokaryotic cell, a eukaryotic cell or a cell from a member of the *Archaea.* The host cell may be from any organism, in particular any non-human organism. In particular the host cell may be selected from bacterial cells, fungal cells, archaea, protists, plant cells (including algae), cells originating from an animal (in particular isolated from said animal). The host cell may form part of a multicellular organism, other than human or the organism from which the enzyme naturally originates. In a specific embodiment, host cells of the invention are in a culture of cells originating from a multicellular organism, yet isolated there from.

In general, the non human host cell is an organism comprising genes for expressing the enzymes for catalysing the reaction steps of the mevalonate pathway or another metabolic pathway (such as the deoxyxylulose-5-phosphate (DXP) pathway) enabling the production of the C5 prenyl diphosphates isopentenyl diphosphate (IPP) and dimethylallyl diphosphate (DMAPP), which are the universal isoprenoid building blocks. As far as known, unless specific genes have been knocked-out, all known organisms comprise such a pathway. Eukaryotes generally are naturally capable of preparing IPP via the mevalonate pathway. This IPP is then isomerized into DMAPP by the action of the enzyme isopentenyl diphosphate isomerase (Idi). The DXP pathway, which is furnishing IPP and DMAPP in a 5:1 ratio, is common to prokaryotes, although several prokaryotes are naturally capable of preparing IPP via the mevalonate pathway. These pathways are known in the art, and have been described, e.g., by Withers & Keasling in Appl. Microbiol. Biotechnol. (2007) 73: 980-990, of which the contents with respect to the description of these pathways, and in particular Figure 1 and the enzymes mentioned in said publication that play a role in one or both of said pathways, are enclosed by reference. The genes of these pathways may each independently be homologous or heterologous to the cell.

The host cells further will, either endogenically or from heterologous sources, comprise one or more genes for expressing enzymes with prenyl transferase activity catalysing the head-to-tail condensation of the C5 prenyl diphosphates producing longer prenyl diphosphates. The universal sesquiterpene precursor farnesyl diphosphate (FPP), for instance, is formed by the action of these enzymes through the successive head-to-tail addition of 2 molecules of IPP to 1 molecule of DMAPP.

In an embodiment, the host cell is a bacterium. The bacterium may be gram-positive or gram-negative. Gram-positive bacteria may be selected from the genera of *Bacillus* and *Lactobacillus,* in particular from the species of *Bacillus subtilis* and *Lactobacillus casei.*

In a preferred embodiment, the bacterium is selected from the group of gram-negative bacteria, in particular from the group of *Rhodobacter, Paracoccus* and *Escherichia,* more in particular from the group of *Rhodobacter capsulatus, Rhodobacter sphaeroides, Paracoccus carotinifaciens, Paracoccus zeaxanthinifaciens* and *Escherichia coli. Rhodobacter sphaeroides* is an example of an organism naturally containing all genes needed for expressing enzymes catalysing the various reaction steps in the DXP pathway, enabling the intracellular production of IPP and DMAPP.

In an embodiment, the host cell is a fungal cell, in particular a fungal cell selected from the group of *Aspergillus, Blakeslea, Penicillium, Phaffia* (*Xanthophyllomyces*), *Pichia, Saccharomyces* and *Yarrowia,* more in particular from the group of *Aspergillus nidulans, Aspergillus niger, Aspergillus oryzae, Blakeslea trispora,Penicillium chrysogenum, Phaffia rhodozyma (Xanthophyllomyces dendrorhous), Pichia pastoris, Saccharomyces cerevisiae* and *Yarrowia lipolytica.*

It is also possible to express the nucleic acids of the invention in cells derived from higher eukaryotic organisms, such as plant cells and animal cells, such as insect cell, or cells from mouse, rat or human. Said cells can be maintained in a cell or tissue culture and be used for *in vitro* production of valencene synthase.

A multicellular organism comprising host cells according to the invention may in particular be selected from the group of multicellular plants and mushrooms (*Basidiomycetes*).

Thus, in a specific embodiment, the invention relates to a transgenic plant or plant cell or tissue culture comprising transgenic plant cells, said plant or culture comprising plant host cells according to the invention. The transgenic plant or culture of transgenic plant cells may in particular be selected from *Nicotiana spp., Solanum spp., Cichorum intybus, Lactuca sativa, Mentha spp., Artemisia annua,* tuber forming plants, such as *Helianthus tuberosus,* cassava and *Beta vulgaris,* oil crops, such as *Brassica spp., Elaeis spp.* (oil palm tree), *Helianthus annuus, Glycine max* and *Arachis hypogaea,* liquid culture plants, such as duckweed *Lemna* spp., tobacco BY2 cells and *Physcomitrella patens,* trees, such as pine tree and poplar, respectively a cell culture or a tissue culture of any of said plants. In a specific embodiment, the tissue culture is a hairy root culture.

In a further specific embodiment the invention relates to a transgenic mushroom or culture comprising transgenic mushroom cells. The transgenic mushroom or culture comprising transgenic host cells, may in particular be selected from the group of *Schizophyllum, Agaricus* and *Pleurotus,* more in particular from *Schizophyllum commune,* the common mushroom (*Agaricus bisporus*),the oyster mushroom (*Pleurotus ostreotus* and *Pleurotus sapidus*), respectively a culture comprising cells of any of said mushrooms. One additional advantage for using mushrooms to express the valencene synthase is that at least some mushrooms are able to convert valencene into nootkatone (Fraatz, M.A. et al., J. Mol. Catal. B: Enzym. (2009) 61: 202-207).

Next to the production of valencene *per se,* expression of valencene synthase according to the invention and production of valencene in plants or mushrooms also provides resistance in these organisms. First of all, valencene is known to act as an insect repellent and is active against insects such as mosquitoes, cockroaches, ticks, fleas, termites and *Drosophila.* Further, valencene has been shown to make plants resistant to pathogens, such as the fungus *Phytophthora,* especially *P. ramorum* (Sudden oak death agent) (Manter, D.K. et al., Forest Pathology (2006) 36: 297-308).

A host cell according to the invention may be produced based on standard genetic and molecular biology techniques that are generally known in the art, *e.g.* as described in Sambrook, J., and Russell, D.W. "Molecular Cloning: A Laboratory Manual" 3d ed., Cold Spring Harbor Laboratory Press, Cold Spring Harbor, NY, (2001); and F.M. Ausubel et al, eds., "Current protocols in molecular biology", John Wiley and Sons, Inc., New York (1987), and later supplements thereto.

Methods to transform *Basidiomycetes* are known from, for example, Alves et al. (Appl. Environ. Microbiol. (2004) 70: 6379-6384), Godio et al. (Curr. Genet. (2004) 46: 287-294), Schuurs et al. (Genetics (1997) 147: 589-596), and WO 06/096050. To achieve expression of a suitable valencene synthase gene in basidiomycetes, its complete open reading frame is typically cloned into an expression vector suitable for transformation of basidiomycetes. The expression vector preferably also comprises nucleic acid sequences that regulate transcription initiation and termination. It is also preferred to incorporate at least one selectable marker gene to allow for selection of transformants. Expression of a valencene synthase can be achieved using a basidiomycete promoter, *e.g*. a constitutive promoter or an inducible promoter. An example of a strong constitutive promoter is the glyceraldehyde-3-phosphate dehydrogenase (gpdA) promoter. This promoter is preferred for constitutive expression when recombinant DNA material is expressed in a basidiomycete host. Other examples are the phosphoglycerate kinase (pgk) promoter, the pyruvate kinase (pki) promoter, TPI, the triose phosphate isomerase (tpi) promoter, the APC synthetase subunit g (oliC) promoter, the sc3 promoter and the acetamidase (amdS) promoter of a basidiomycete (WO 96/41882).

If needed, the primary nucleotide sequence of the valencene synthase gene can be adapted to the codon usage of the basidiomycete host.

Further, expression can be directed especially to the (monokaryotic) mycelium or to the (dikaryotic) fruiting bodies. In the latter case, the Fbh1 promoter of *Pleurotis* is especially useful (Penas, M.M. et al., Mycologia (2004) 96: 75-82).

Methodologies for the construction of plant transformation constructs are described in the art. Overexpression can be achieved by insertion of one or more than one extra copy of the selected gene. It is not unknown for plants or their progeny, originally transformed with one or more than one extra copy of a nucleotide sequence to exhibit overexpression.

Obtaining sufficient levels of transgenic expression in the appropriate plant tissues is an important aspect in the production of genetically engineered crops. Expression of heterologous DNA sequences in a plant host is dependent upon the presence of an operably linked promoter that is functional within the plant host. Choice of the promoter sequence will determine when and where within the organism the heterologous DNA sequence is expressed. Although many promoters from dicotyledons have been shown to be operational in monocotyledons and *vice versa,* ideally dicotyledonous promoters are selected for expression in dicotyledons, and monocotyledonous promoters for expression in monocotyledons. However, there is no restriction to the provenance of selected promoters; it is sufficient that they are operational in driving the expression of the nucleotide sequences in the desired cell or tissue. In some cases, expression in multiple tissues is desirable, and constitutive promoters such as the 35S promoter series may be used in this respect. However, in some of the embodiments of the present invention it is preferred that the expression in transgenic plants is leaf-specific, more preferably, the expression of the gene occurs in the leaf plastids. The promoter of the isoprene synthase gene from *Populus alba* (PaIspS) (Sasaki et al., FEBS Letters (2005) 579: 2514-2518) appears to drive plastid-specific expression. Hence, this promoter is a very suitable promoter for use in an expression vector of the present invention.

Other suitable leaf-specific promoters are the rbcS (Rubisco) promoter *(e.g.* from coffee, see WO 02/092822); from *Brassica,* see US 7,115,733; from soybean, see Dhanker, O., et al., Nature Biotechnol. (2002) 20: 1140-1145), the cy-FBPase promoter (see US 6,229,067), the promoter sequence of the light-harvesting chlorophyll a/b binding protein from oil-palm (see US 2006/0288409), the STP3 promoter from *Arabidopsis thaliana* (see, Büttner, M. et al., Plant cell & Environ. (2001) 23: 175-184), the promoter of the bean PAL2 gene (see Sablowski, R.W. et al., Proc. Natl. Acad. Sci. USA (1995) 92: 6901-6905), enhancer sequences of the potato ST-LS1 promoter (see Stockhaus, J. et al., Proc. Natl. Acad. Sci. USA (1985) 84: 7943-7947), the wheat CAB1 promoter (see Gotor, C. et al., Plant J. (1993) 3: 509-518), the stomata-specific promoter from the potato ADP-glucose-phosphorylase gene (see US 5,538,879), the LPSE1 element from the P(D540) gene of rice (see CN 2007/10051443), and the stomata specific promoter, *pGC1*(At1g22690) from *Arabidopsis thaliana* (see Yang, Y. et al., Plant Methods (2008) 4: 6).

Plant species may, for instance, be transformed by the DNA-mediated transformation of plant cell protoplasts and subsequent regeneration of the plant from the transformed protoplasts in accordance with procedures well known in the art.

Further examples of methods of transforming plant cells include microinjection (Crossway et al., Mol. Gen. Genet. (1986) 202: 179-185), electroporation (Riggs, C.D. and Bates, G.W., Proc. Natl. Acad. Sci. USA (1986), 83: 5602-5606), *Agrobacterium- mediated* transformation (Hinchee et al., Bio/Technol. (1988) 6: 915-922), direct gene transfer (Paszkowski, J. et al., EMBO J. (1984) 3: 2717-2722), and ballistic particle acceleration using devices available from Agracetus, Inc., Madison, Wisconsin and BioRad, Hercules, California (see, for example, Sanford et al., U. S. Pat. No. 4,945,050 and European Patent Application EP 0 332 581).

It is also possible to employ the protoplast transformation method for maize (European Patent Application EP 0 292 435, U. S. Pat. No. 5,350,689).

It is particularly preferred to use the binary type vectors of Ti and Ri plasmids of *Agrobacterium* spp. Ti-derived vectors transform a wide variety of higher plants, including monocotyledonous and dicotyledonous plants, such as soybean, cotton, rape, tobacco, and rice (Pacciotti et al., Bio/technol. (1985) 3: 241; Byrne M.C. et al., Plant Cell Tissue and Organ Culture (1987) 8: 3-15; Sukhapinda, K. et al., Plant Mol. Biol. (1987) 8: 209-217; Hiei, Y. et al., The Plant J. (1994) 6: 271-282). The use of T-DNA to transform plant cells has received extensive study and is amply described (*e.g*. EP-A 120 516). For introduction into plants, the chimeric genes of the invention can be inserted into binary vectors as described in the examples.

Other transformation methods are available to those skilled in the art, such as direct uptake of foreign DNA constructs (see EP-A 295 959), techniques of electroporation (Fromm, M.E. et al., Nature (1986), 319: 791-793) or high velocity ballistic bombardment with metal particles coated with the nucleic acid constructs (*e.g.* US 4,945,050). Once transformed, the cells can be regenerated by those skilled in the art. Of particular relevance are the methods to transform foreign genes into commercially important crops, such as rapeseed (De Block, M. et al., Plant Physiol. (1989) 91: 694-701), sunflower (Everett, N.P. et al., Bio/Technology (1987) 5: 1201-1204), soybean (EP-A 301 749), rice (Hiei, Y. et al., The Plant J. (1994) 6: 271-282), and corn (Fromm et al., 1990, Bio/Technology 8: 833-839).

Those skilled in the art will appreciate that the choice of method might depend on the type of plant, *i.e*., monocotyledonous or dicotyledonous.

In another embodiment, the vector as described herein may be directly transformed into the plastid genome. Plastid transformation technology is extensively described in, *e.g.,* US 5,451,513, US 5,545,817, US 5, 545,818 and WO 95/16783. The basic technique for chloroplast transformation involves introducing regions of cloned plastid DNA flanking a selectable marker together with the gene of interest into a suitable target tissue, e. g., using biolistics or protoplast transformation (*e.g*. calcium chloride or PEG mediated transformation).

*Agrobacterium tumefaciens* cells containing a vector according to the present invention, wherein the vector comprises a Ti plasmid, are useful in methods of making transformed plants. Plant cells are infected with an *Agrobacterium tumefaciens* as described above to produce a transformed plant cell, and then a plant is regenerated from the transformed plant cell. Numerous *Agrobacterium* vector systems useful in carrying out the present invention are known. These typically carry at least one T-DNA border sequence and include vectors such as pBIN19 (Bevan, Nucl. Acids Res. (1984) 12: 8711-8720).

Methods using either a form of direct gene transfer or *Agrobacterium-*mediated transfer usually, but not necessarily, are undertaken with a selectable marker which may provide resistance to an antibiotic (*e.g.* kanamycin, hygromycin or methotrexate) or a herbicide (*e.g.* phosphinothricin). The choice of selectable marker for plant transformation is not, however, critical to the invention.

General methods of culturing plant tissues are provided for example by Maki, K.Y. et al., Plant Physiol. (1993) 15: 473-497; and by Phillips, R.I. et al. In: Sprague GF, Dudley JW, eds. Corn and corn improvement. 3rd edn. Madison (1988) 345-387.

After transformation the transgenic plant cells are placed in an appropriate selective medium for selection of transgenic cells which are then grown to callus. Shoots are grown from callus and plantlets generated from the shoot by growing in rooting medium. The particular marker used will allow for selection of transformed cells as compared to cells lacking the DNA which has been introduced.

To confirm the presence of the transgenes in transgenic cells and plants, a variety of assays may be performed. Such assays include, for example, "molecular biological" assays well known to those of skill in the art, such as Southern and Northern blotting, in situ hybridization and nucleic acid-based amplification methods such as PCR or RT-PCR and "biochemical" assays, such as detecting the presence of a protein product, e.g., by immunological means (ELISAs and Western blots) or by enzymatic function. The presence of enzymatically active valencene synthase may be established by chemical analysis of the volatile products (valencene) of the plant.

A valencene synthase according to the invention may be used for the industrial production of valencene, which valencene may be used *per se* as a flavour or aroma, *e.g.* in a food product, or as a fragrance, *e.g.* in a household product, or as an intermediate for the production of another isoprenoid, *e.g*. nootkatone.

A method for producing valencene according to the invention comprises preparing valencene in the presence of valencene synthase. In principle such a method can be based on any technique for employing an enzyme in the preparation of a compound of interest.

The method can be a method wherein FPP or any of its precursors (such as farnesol, IPP, isopentenyl phosphate, 3-methylbut-3-en-1-ol and even mevalonate) is fed as a substrate to cells comprising the valencene synthase. Alternatively the method can also be a method wherein use is made of a living organism that comprises an enzyme system capable of forming FPP from a suitable carbon source, thus establishing a full fermentative route to valencene. It should be noted that the term "fermentative" is used herein in a broad sense for processes wherein use is made of a culture of an organism to synthesise a compound from a suitable feedstock *(e.g.* a carbohydrate, an amino acid source, a fatty acid source). Thus, fermentative processes as meant herein are not limited to anaerobic conditions, and extended to processes under aerobic conditions. Suitable feedstocks are generally known for specific species of (micro-)organisms.

Also, use may be made of the valencene synthase isolated from the cell wherein it has been produced, e.g. in a reaction system wherein the substrate (FPP) and the valencene synthase are contacted under suitable conditions (pH, solvent, temperature), which conditions may be based on the prior art referred to herein and the present disclosure, optionally in combination with some routine testing. The valencene synthase may *e.g.* be solubilised in an aqueous medium wherein also the FPP is present or the valencene synthase may be immobilised on a support material in a manner known in the art and then contacted with a liquid comprising the FPP. Since the enzyme has a high activity and/or selectivity towards the catalysis from FPP to valencene, the present invention is also advantageous for such an *in vitro* method, not only under acidic conditions, but also in case the pH is about neutral or alkaline. Suitable conditions may be based on known methodology for known valencene synthases, *e.g.* referred to in the literature referred to herein, the information disclosed herein, common general knowledge and optionally some routine experimentation.

In a particularly advantageous method of the invention, valencene is fermentatively prepared, *i.e*. by cultivating cells expressing valencene synthase in a culture medium. The actual reaction catalysed by the valencene synthase may take place intracellularly or - if the valencene synthase is excreted into the culture medium - extracellularly in the culture medium.

The cells used in a method for preparing valencene according to the invention may in particular be host cells according to the invention. If desired, these host cells may be engineered to supply the FPP to the valencene synthase in increased amounts. This can for instance be done by enhancing the flux of carbon towards FPP, which in itself can be realized in different ways. In host cells with an endogenous DXP pathway (like *E. coli* and *R. sphaeroides*) deregulation of the expression of these pathway's enzymes can have a clear positive effect on isoprenoids formation. Overexpression of *dxs* encoding 1-deoxy-D-xylulose-5-phosphate synthase (DXP-synthases), the first enzyme of the DXP pathway and thus one of the main targets for metabolic engineering, has resulted in increased biosynthesis of several isoprenoids (*e.g*., Matthews and Wurtzel, Appl. Microbiol. Biotechnol. (2000) 53: 396-400; Huang et al., Bioorg. Med. Chem. (2001) 9: 2237-2242; Harker and Bramley, FEBS Lett (1999) 448: 115-119; Jones et al. Metab. Eng. (2000) 2: 328-338; and Yuan et al. Metab. Eng. (2006) 8: 79-90). Also overexpression of *dxr* coding for DXP isomeroreductase (also known as 1-deoxy-D-xylulose-5-phosphate reductoisomerase), the enzyme catalyzing the second and committed step in the DXP pathway, can lead to increased isoprenoid production (Albrecht et al., Biotechnol. Lett. (1999) 21: 791-795), which effect can be further increased by co-overexpressing *dxs* at the same time (Kim & Keasling, Biotechnol Bioeng (2001) 72: 408-415). A positive effect on isoprenoid biosynthesis was further obtained by overexpression of isopentenyl diphosphate isomerase (IPP isomerase, Idi), the enzyme that catalyzes the interconversion of IPP to dimethylallyl diphosphate, DMAPP (*e.g.,* Kajiwara et al. Biochem. J. (1997) 324: 421-426); Misawa and Shimada, J. Biotech. (1998) 59: 169-181; and Yuan et al. Metab. Eng. (2006) 8: 79-90) and the enzymes MEP cytidylyltransferase (also known as 4-diphosphocytidyl-2-C-methyl-D-erythritol synthase, IspD) and 2C-methyl-D-erythritol 2,4-cyclodiphosphate synthase (IspF), that are transcribed as one operon *ispDF* in *E. coli* (Yuan et al. Metab. Eng. (2006) 8: 79-90).

An alternative and more efficient approach to engineer strains with an endogenous DXP pathway for high-level production of isoprenoids is the introduction of a heterologous mevalonate pathway. Coexpression in *E. coli* of the *Saccharomyces cerevisiae* mevalonate pathway with a synthetic amorpha-4,11-diene synthase gene resulted in the formation of the sesquiterpene amorphadiene in titres of more than 110 mg/L when the recombinant *E. coli* strain was cultivated in an LB+ glycerol medium (Martin et al. Nat. Biotechnol. (2003) 21: 796-802). This *E. coli* strain was subsequently improved by the introduction of extra copies of the gene *tHMG1* encoding the C-terminal catalytic domain of the yeast enzyme 3-hydroxy-3-methylglutaryl-coenzyme A (HMG-CoA) reductase. By increasing the formation and thus the activity of this enzyme, the intracellular level of the toxic mevalonate pathway intermediate HMG-CoA was reduced thereby overcoming growth inhibition and leading to an increased production of mevalonate (Pitera et al. Metab. Eng. (2007) 9: 193-207). Further improvement of the flux through the heterologous mevalonate pathway was obtained by codon optimization of the first three genes of this pathway in combination with replacement of the wild-type *lac* promoter with the two-fold stronger *lacUV5* promoter (Anthony et al. Met. Eng. (2009) 11: 13-19). The production of amorphadiene could be even more increased by replacing the yeast genes for HMG-CoA synthase and HMG-CoA reductase with the equivalent genes from the gram positive bacterium *Staphylococcus aureus.* In combination with an optimized fermentation protocol, cultivation of this novel engineered *E. coli* strain yielded an amorphadiene titre of 27.4 g/L (Tsuruta et al. PloS ONE (2009) 4(2): e4489. doi:10.1371/journal.pone.0004489). Similarly, an *E. coli* strain engineered with the mevalonate pathway from *Streptococcus pneumoniae* in combination with the *Agrobacterium tumefaciens* decaprenyl diphosphate synthase (*ddsA*) gene produced coenzyme Q₁₀ (CoQ₁₀) in more than 2400 µg/g cell dry weight (Zahiri et al. Met. Eng. (2006) 8: 406-416. Increased production of CoQ₁₀ was also obtained by engineering a *Rhodobacter sphaeroides* strain with the mevalonate pathway from *Paracoccus zeaxanthinifaciens* in its native (WO 2005/005650) and a mutated from (WO 2006/018211).

Also host cells with an endogenous MEV pathway (like *S. cerevisiae*) have been the subject of multiple engineering studies to obtain isoprenoid hyper producing strains. Introduction into *S. cerevisiae* of the heterologous *E. coli* derived DXP pathway in combination with the gene encoding the Citrus valencene synthase resulted in a strain accumulating approximately 10-fold more valencene compared to the strain expressing only the valencene synthase (WO 2007/093962). Most improvements in the industrially-important yeasts *Candida utilis* and *S. cerevisiae,* however, have centred on the engineering of the homologous MEV pathway. Especially overexpression of the enzyme HMG-CoA reductase, which is believed to be the main regulatory enzyme in the DXP pathway, in its full-length or truncated version, has appeared to be an efficient method to increase production of isoprenoids. This stimulating effect of overexpression of the N-terminal truncated HMG-CoA reductase has, for instance, been observed in case of lycopene production in C. *utilis* (Shimada et al. Appl. Env. Microbiol. (1998) 64: 2676-2680) and epi-cedrol production in *S. cerevisiae* (Jackson et al. Org. Lett. (2003) 5: 1629-1632). In the last case the production of this sesquiterpene could be further enhanced by introduction of *upc2-1,* an allele that elicitates an increase in the metabolic flux to sterol biosynthesis. Another method to increase the flux through the MEV pathway is the employment of a mevalonate kinase variant that is less sensitive for feedback inhibition by FPP and other isoprenoid precursors. WO 2006/063752, for instance, shows that *Paracoccus zeaxanthinifaciens* R114, a bacterium with an endogenous MEV pathway, after introduction of the *S. cerevisiae* mevalonate kinase mutant N66K/I152M and the *ddsA* gene from *P. zeaxanthinifaciens* ATCC 21588 produces significantly more coenzyme Q₁₀ than the corresponding *P. zeaxanthinifaciens* strain expressing the wild type *S. cerevisiae* mevalonate kinase. Similar positive results on CoQ₁₀ production with *P. zeaxanthinifaciens* R114 have also been obtained with the feedback resistant variant K93E of the *P. zeaxanthinifaciens* mevalonate kinase (WO 2004/111214).

A second approach to increased amounts of FPP is based on reducing or elimination of enzymatic side activities on FPP. In yeast the gene *ERG9* encodes the enzyme farnesyl diphosphate farnesyl transferase (squalene synthase), which catalyzes the condensation of two farnesyl diphosphate moieties to form squalene. Because this is the first step after FPP in the sterol biosynthesis and thus regulates the flux of isoprene units into the sterol pathway, *ERG9* is a frequent target in yeast metabolic engineering for increased sesquiterpene and carotenoids production. Disruption of *ERG9* in combination with overexpression of the tHMG-CoA reductase in the yeast C. *utilis* led to increased production of lycopene (Shimada et al. Appl. Env. Microbiol. (1998) 64: 2676-2680). A similar combination of overexpression of tHMG-CoA reductase and downregulation of *ERG9* using a methionine repressible promoter increased the production of the sesquiterpene amorphadiene in yeast with approx. 10-fold as compared to the yeast strain only expressing the amorphadiene synthase gene (Ro et al. Nature (2006) 440: 940-943; Lenihan et al. Biotechnol. Prog. (2008) 24: 1026-1032). Since ergosterol is vital for yeast growth and yeast cells cannot assimilate externally fed ergosterol during aerobic growth, downregulation/knockout of *ERG9* is frequently combined with mutations that equip the yeast strain with efficient aerobic uptake of ergosterol from the culture medium. Examples are the *sue* allele (Takahishi et al. Biotechnol. Bioeng. (2007) 97: 170-181) and the *upc2-1* allele (Jackson et al. Org. Lett. (2003) 5: 1629-1632). Takahashi et al (Biotechnol. Bioeng. (2007) 97: 170-181) also investigated the effect of limiting the endogenous phosphatase activity by knocking out the phosphatase gene *dpp1* in yeast. Although this knockout clearly limited the dephosphorylation of FPP reflected by much less farnesol accumulation, it did not improve sesquiterpene production beyond that of the combined *erg9*/*sue* mutations under the growth conditions applied.

Reaction conditions for fermentatively preparing valencene may be chosen depending upon known conditions for the species of host cell used (e.g. *Rhodobacter capsulatus, Rhodobacter sphaeroides, Paracoccus zeaxanthinifaciens, Escherichia coli, Aspergillus nidulans, Aspergillus niger, Aspergillus oryzae, Saccharomyces cerevisiae, Yarrowia lipolytica, Penicillium chrysogenum, Phaffia rhodozyma* and *Pichia pastoris*), the information disclosed herein, common general knowledge and optionally some routine experimentation.

In principle, the pH of the reaction medium (culture medium) used in a method according to the invention may be chosen within wide limits, as long as the valencene synthase (in the host cell) is active and displays a wanted specificity under the pH conditions. In case the method includes the use of cells, for expressing the valencene synthase, the pH is selected such that the cells are capable of performing its intended function or functions. The pH may in particular be chosen within the range of four pH units below neutral pH and two pH units above neutral pH, i.e. between pH 3 and pH 9 in case of an essentially aqueous system at 25°C. Good results have e.g. been achieved in an aqueous reaction medium having a pH in the range of 6.8 to 7.5.

A system is considered aqueous if water is the only solvent or the predominant solvent (> 50 wt. %, in particular > 90 wt. %, based on total liquids), wherein e.g. a minor amount of alcohol or another solvent (< 50 wt. %, in particular < 10 wt. %, based on total liquids) may be dissolved (e.g. as a carbon source, in case of a full fermentative approach) in such a concentration that micro-organisms which are present remain active.

In particular in case a yeast and/or a fungus is used, acidic conditions may be preferred, in particular the pH may be in the range of pH 3 to pH 8, based on an essentially aqueous system at 25°C. If desired, the pH may be adjusted using an acid and/or a base or buffered with a suitable combination of an acid and a base.

Anaerobic conditions are herein defined as conditions without any oxygen or in which substantially no oxygen is consumed by the cultured cells, in particular a micro-organism, and usually corresponds to an oxygen consumption of less than 5 mmol/l.h, preferably to an oxygen consumption of less than 2.5 mmol/l.h, or more preferably less than 1 mmol/l.h. Aerobic conditions are conditions in which a sufficient level of oxygen for unrestricted growth is dissolved in the medium, able to support a rate of oxygen consumption of at least 10 mmol/l.h, more preferably more than 20 mmol/l.h, even more preferably more than 50 mmol/l.h, and most preferably more than 100 mmol/l.h.

Oxygen-limited conditions are defined as conditions in which the oxygen consumption is limited by the oxygen transfer from the gas to the liquid. The lower limit for oxygen-limited conditions is determined by the upper limit for anaerobic conditions, i.e. usually at least 1 mmol/l.h, and in particular at least 2.5 mmol/l.h, or at least 5 mmol/l.h. The upper limit for oxygen-limited conditions is determined by the lower limit for aerobic conditions, i.e. less than 100 mmol/l.h, less than 50 mmol/l.h, less than 20 mmol/l.h, or less than to 10 mmol/l.h.

Whether conditions are aerobic, anaerobic or oxygen-limited is dependent on the conditions under which the method is carried out, in particular by the amount and composition of ingoing gas flow, the actual mixing/mass transfer properties of the equipment used, the type of micro-organism used and the micro-organism density.

In principle, the temperature used is not critical, as long as the valencene synthase (in the cells), shows substantial activity. Generally, the temperature may be at least 0°C, in particular at least 15°C, more in particular at least 20°C. A desired maximum temperature depends upon the valencene synthase and the cells, in case of a method wherein use is made of cells for expressing the valencene synthase. The temperature is 70°C or less, preferably 50°C or less, more preferably 40°C or less, in particular 35°C or less.

In case of a fermentative process, the incubation conditions can be chosen within wide limits as long as the cells show sufficient activity and/ or growth. This includes aerobic, oxygen-limited and anaerobic conditions.

In particular if the catalytic reaction whereby valencene is formed, is carried out outside a host cell, a reaction medium comprising an organic solvent may be used in a high concentration (*e.g*. more than 50 %, or more than 90 wt. %, based on total liquids), in case the valencene synthase that is used retains sufficient activity and specificity in such a medium.

If desired, valencene produced in a method according to the invention, or a further compound into which valencene has been converted after its preparation (such as nootkatone), is recovered from the reaction medium, wherein it has been made. A suitable method is liquid-liquid extraction with an extracting liquid that is non-miscible with the reaction medium.

In particular suitable (for extraction from an aqueous reaction medium) is extraction with a liquid organic solvent, such as a liquid hydrocarbon. From initial results it is apparent that this method is also suitable to extract the valencene (or further product) from a reaction medium comprising cells according to the invention used for its production, without needing to lyse the cells for recovery of the valencene (or further product).In particular, the organic solvent may be selected from liquid alkanes, liquid long-chain alcohols (alcohols having at least 12 carbon atoms), and liquid esters of long-chain fatty acids (acids having at least 12 carbon atoms). Suitable liquid alkanes in particular include C6-C16 alkanes, such as hexane, octane, decane, dodecane, isododecane and hexadecane. Suitable long-chain aliphatic alcohol in particular include C12-C18 aliphatic alcohols, like oleyl alcohol and palmitoleyl alcohol. Suitable esters of long-chain fatty acids in particular include esters of C1-C4 alcohols of C12-C18 fatty acids, like isopropyl myristate, and ethyl oleate.

In an advantageous embodiment, valencene (or a further product) is produced in a reactor comprising a first liquid phase (the reaction phase), said first liquid phase containing cells according to the invention in which cells the valencene (or a further product) is produced, and a second liquid phase (organic phase that remains essentially phase-separated with the first phase when contacted), said second liquid phase being the extracting phase, for which the formed product has a higher affinity. This method is advantageous in that it allows *in situ* product recovery. Also, it contributes to preventing or at least reducing potential toxic effects of valencene (or a further product) to the cells, because due to the presence of the second phase, the valencene (or a further product) concentration in the reaction phase may be kept relatively low throughout the process. Finally, there are strong indications that the extracting phase contributes to extracting the valencene (or further product) out of the reaction phase.

In a preferred method of the invention the extracting phase forms a layer on top of the reaction phase or is mixed with the reaction phase to form a dispersion of the reaction phase in the extracting phase or a dispersion of the extracting phase in the reaction phase. Thus, the extracting phase not only extracts product from the reaction phase, but also helps to reduce or completely avoid losses of the formed product from the reactor through the off-gas, that may occur if valencene is produced in the (aqueous) reaction phase or excreted into the (aqueous) reaction phase. Valencene is poorly soluble in water and therefore easily volatilizes from water. It is contemplated that valencene solvated in the organic phase (as a layer or dispersion) is at least substantially prevented from volatilization.

Suitable liquids for use as extracting phase combine a lower density than the reaction phase with a good biocompatibility (no interference with the viability of living cells), low volatility, and near absolute immiscibility with the aqueous reaction phase. Examples of suitable liquids for this application are liquid alkanes like decane, dodecane, isododecane, tetradecane, and hexadecane or long-chain aliphatic alcohols like oleyl alcohol, and palmitoleyl alcohol, or esters of long-chain fatty acids like isopropyl myristate, and ethyl oleate (see *e.g.* Asadollahi et al. (Biotechnol. Bioeng. (2008) 99: 666-677), Newman et al. (Biotechnol. Bioeng. (2006) 95: 684-691) and WO 2009/042070).

The valencene produced in accordance with the invention may be used as such, *e.g.* for use as a flavour or fragrance, or as an insect repellent, or may be used as a starting material for another compound, in particular another flavour or fragrance. In particular, valencene may be converted into nootkatone. The conversion of valencene into nootkatone may be carried out intracellularly, or extracellularly. If this preparation is carried out inside a cell, the nootkatone is usually isolated from the host cell after its production.

Suitable manners of converting valencene to nootkatone are known in the art, *e.g.* as described in Fraatz et al. Appl.Microbiol.Biotechnol (2009) 83: 35-41, of which the contents are incorporated by reference, or the references cited therein.

In general, suitable methods to prepare nootkatone from valence may be divided in: i. purely chemical methods, ii. biocatalytic methods (*e.g*. those using laccases in combination with a mediator), iii. bioconversion (*i.e.* methods applying whole living cells), and iv. full fermentation. In methods i-iii externally fed valencene is converted, whereas in method iv the valencene is produced *in situ.*

In a specific embodiment, the conversion comprises a regiospecific hydroxylation of valencene at the 2-position to alpha- and/or beta-nootkatol, followed by oxidation thereof forming nootkatone.

In a further embodiment, valencene is converted into the hydroperoxide of valencene, which is thereafter converted in nootkatone. U. S. patent no. 5,847,226 describes the chemical conversion of (+)-valencene into nootkatone in an oxygen-containing atmosphere in the presence of a hydroperoxyde of an unsaturated fatty acid. This fatty acid hydroperoxide is generated in situ by, *e.g*., autooxidation, photooxidation or enzymatic oxidation using a lipoxyygenase, after which this hydroperoxide catalyzes the autooxidation of valencene.

(+)-Valencene can be converted in high yields into nootkatone by different species of the green alga *Chlorella* or the fungus *Botryosphaeria* (Furusawa et al. Chem. Pharm. Bull. (2005) 53: 1513-1514, and JP 2003-070492).

EP-A 1 083 233 describes the preparation of nootkatone applying cell-free (biocatalytic) systems based on laccase catalyzed conversion of valencene into valencene hydroperoxide, which is subsequently degraded to form nootkatone. Optionally, a mediator and/or a solvent at a concentration that maintains laccase activity may be included.

WO 2006/079020 describes amongst other things a novel plant derived cytochrome P450 enzyme, the Premnaspirodiene oxygenase (HPO) from *Hyoscyamus muticus* which catalyzes the mono-hydroxylation of (+)-valencene to mainly beta-nootkatol. Nootkatone formation was only observed at very high concentrations of nootkatol (>30 µM) but only at a very low reaction rate (Takahashi et al. J.Biol.Chem. (2007) 282: 31744-31754). In the same paper, Takahashi *et al.* report on an HPO mutant with a 5-fold improvement in its catalytic efficiency for nootkatol biosynthesis without significantly changing the overall reaction product profiles. This nootkatol might be further oxidized to nootkatone by co-expression of an alcohol dehydrogenase enzyme in the same host cell.

Another suitable plant derived cytochrome P450 enzyme has recently been identified in Chicory (*Cichorium intybus* L.). Co-expression of this novel P450 enzyme with a valencene synthase in yeast, led to formation of *trans*-nootkatol, *cis-*nootkatol and (+)-nootkatone (Cankar, K., et al., FEBS Letters (2011) 585: 178-182).

Besides plant derived cytochrome P450 enzymes, also the bacterial cytochrome 450 monooxygenases P450cam and P450BM-3 and mutants thereof have been reported to oxidize (+)-valencene (Sowden et al. Org. Biomol. Chem. (2005) 3: 57-64). Whereas wild type P450cam did not catalyze this oxidation reaction, mutants showed relatively high regioselectivity for the desired C2 position in (+)-valencene, (+)-trans-nootkatol and (+)-nootkatone constituting >85% of the products formed. The activity of these mutants was still rather low. The P450BM-3 mutants, on the other hand, displayed a higher activity but were unselective because of the multiple binding orientations of (+)-valencene in the active site. Recently, much more selective BM-3 mutants have been reported, the best of which has a C2-regioselectivity of 95% (Seifert et al. ChemBioChem (2009) 10: 853-861).

It is contemplated that one or more genes encoding an enzyme or plurality of enzymes for catalysing the conversion of valencene into nootkatone may be incorporated in a host cell according to the invention. Such enzymes may in for instance be selected from the enzymes of *Chlorella* or *Botryosphaeria,* or Premnaspirodiene oxidase from *Hyoscyamus muticus,* or the P450cam or P450BM-3 mutants referred to herein above.

As indicated above, the invention relates to an antibody having specific binding affinity to a valencene synthase according to the invention. The term "antibody" includes reference to antigen binding forms of antibodies (*e.g*., Fab, F (ab) 2). The term "antibody" frequently refers to a polypeptide substantially encoded by an immunoglobulin gene or immunoglobulin genes, or fragments thereof which specifically bind and recognize an analyte (antigen). However, while various antibody fragments can be defined in terms of the digestion of an intact antibody, one of skill will appreciate that such fragments may be synthesized de novo either chemically or by utilizing recombinant DNA methodology. Thus, the term antibody, as used herein, also includes antibody fragments such as single chain Fv, chimeric antibodies (*i.e.*, comprising constant and variable regions from different species), humanized antibodies (*i**.**e.*, comprising a complementarity determining region (CDR) from a non-human source) and heteroconjugate antibodies (*e.g.*, bispecific antibodies).

The antibodies or fragments thereof can be produced by any method known in the art for the synthesis of antibodies, in particular, by chemical synthesis or preferably, by recombinant expression techniques.

Polyclonal antibodies to valencene synthase can be produced by various procedures well known in the art. For example, a heterologous valencene synthase can be administered to various host animals including, but not limited to, rabbits, mice, rats, etc. to induce the production of sera containing polyclonal antibodies specific for valencene synthase. Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and *Corynebacterium parvum.* Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art. Briefly, mice can be immunized with valencene synthase and once an immune response is detected, *e.g*., antibodies specific for the valencene synthase are detected in the mouse serum, the mouse spleen is harvested and splenocytes isolated. The splenocytes are then fused by well known techniques to any suitable myeloma cells, for example cells from cell line SP20 available from the ATCC. Hybridomas are selected and cloned by limited dilution. The hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

In certain embodiments, a method of generating monoclonal antibodies comprises culturing a hybridoma cell secreting an antibody of the invention wherein, preferably, the hybridoma is generated by fusing splenocytes isolated from a mouse immunized with valencene synthase with myeloma cells and then screening the hybridomas resulting from the fusion for hybridoma clones that secrete an antibody able to bind valencene synthase. An antibody according to the invention may for instance be used in a method for isolating a valencene synthase produced in accordance with the invention, *e.g*. by using the antibody immobilised on a chromatographic support material.

Further, the present disclosure is directed to a method for preparing valencene or nootkatone, the method comprising converting a polyprenyl diphosphate substrate into the valencene or nootkatone in the presence of a valencene synthase according to the invention. Such method can be based on methodology known in the art, *e.g.* as described in the cited prior art, with the proviso that a valencene synthase according to the invention is used. The valencene or nootkatone can be made intracellularly or using an isolated enzyme. The valencene or nootkatone can be isolated from the medium wherein it is formed. This can be accomplished in a manner known *per se.*

It is also possible to prepare the nootkatone in a host cell expressing one or more enzymes catalysing a reaction step for the conversion of valencene to nootkatone; the host cell preferably also comprising the valencene synthase.

The invention will now be illustrated by the following examples.

### EXAMPLES:

### Example 1: Preparation of Valencene Synthase mutants for activity testing

Synthetic DNA fragments with the genes encoding the Valencene Synthase mutants according to the current invention (ValC_mutant) and with the gene encoding the Valencene Synthase wild type (ValC_wt, SEQ ID NO: 2) were obtained from DNA2.0 (Menlo Park, CA, USA) in expression vector pACYCDuet-1 (Novagen, Merck, KGaA, Darmstadt, Germany). The Valencene Synthase genes had been codon optimized for improved expression in *Rhodobacter* by DNA2.0. The plasmids were designated pACYCDuet:ValC_mutant or pACYCDuet:ValC_wt. The nucleotide sequence of pACYCDuet:ValC_wt is shown in SEQ ID NO: 5. The nucleotide sequence of the pACYCDuet:ValC_mutant plasmids is identical to that of pACYCDuet:ValC_wt except for the nucleotide substitutions needed for the formation of the Valencene Synthase mutants.

The expression of these pACYCDuet-1 based recombinant vectors in *E. coli* resulted in the formation of the valencene synthase (including its N-terminal methionine residue) with the following, pACYC-Duet-1 derived, N-terminal extension: NH₂-MGSSHHHHHHSQDPH-COOH.

The freeze-dried pACYC-Duet-1 based vectors obtained from DNA2.0 were redissolved in 50 µL of sterile water each and incubated at 37°C for 1.5 h. Subsequently, commercially available chemically competent *E. coli* BL21(DE3) cells in 96-well format were transformed with 1 µL redissolved plasmid solution per well applying the supplier's protocol (Novagen; protocol TB 313 Rev.B0304). Each transformation mixture (50 µL plus 50 µL SOC medium to facilitate uniform distribution of the liquid over the plate) were plated onto selective plates (LB-medium with 1% (w/v) glucose and 30 µg/mL chloramphenicol) and incubated at 37°C for 16 h. All selective plates contained colonies, but the number of transformants obtained greatly varied from 1 colony per plate to over 50. Plates were stored at 4°C until further use.

Single colonies of each individual transformant were inoculated in 5 mL of LB medium with 1% (w/v) glucose and 50 µg/mL chloramphenicol, and grown overnight at 37°C. These overnight cultures were used to prepare glycerol stocks, which were stored at -70°C. In addition, 200 µL of each overnight culture were transferred to a 100 mL Erlenmeyer flask with 20 mL 2xYT medium with 50 µg/mL chloramphenicol. Flasks were closed with Rotilabo® culture plugs (Carl Roth GmbH + Co. KG, Karlsruhe, Germany), and incubated at 250 rpm and 37°C until the OD600nm was 0.6-0.8. Then, 20 µL of a 1 M IPTG solution was added and incubation was continued overnight at 250 rpm and 18°C. The next day, cultures were harvested by centrifugation in a 50 mL tube at 3,400 rpm for 15 min, after which the culture medium was removed. Subsequently, cell pellets were stored frozen at -20°C. As a reference, *E. coli* BL21(DE3) transformed with pACYC-Duet-1 containing the gene encoding the wild type valencene synthase (SEQ ID NO: 2) was treated accordingly.

For preparation of cleared lysates, the cell pellets were thawed on ice and resuspended in 1 mL of 50 mM Tris-HCl (pH 7.5) buffer. Then about 0.2 g of Zirconia/Silica beads 0.1 mm (BioSpec Products Inc.; http://www.biospec.com/) were added and lysis was effected by shaking for 10 seconds in a Bio101/Savant FastPrep FP 120 machine (MP Biomedicals LLC., Illkirch, France) at speed 6.5, followed by transfer of the tubes to ice for 2 min., and another round of shaking for 10 seconds at speed 6.5. Subsequently, the lysates were centrifuged for 10 min. at 13,000 x g and 4°C, and supernatants (= cleared lysate) were immediately used for enzyme assays.

Because the number of valencene synthase variants to be tested was larger than the number of cleared lysates that could be produced per day, each day also a cleared lysate was prepared from the cells expressing the wild type valencene synthase as reference.

### Example 2: Determination of (specific) productivity and product specificity

### of valencene synthase mutants

For determination of the valencene productivity of the mutant valencene synthases, the following assay was used.

In a glass tube were mixed:
- 65 µL of 50 mM Tris-HCl, pH 7.5;
- 800 µL of Assay buffer (15 mM MOPSO (3-[N-morpholino]-2-hydroxypropane sulphonic acid) pH = 7.0; 12.5 % (v/v) glycerol; 1 mM ascorbic acid; 0.1% Tween 20; 1 mM MgCl₂; 2 mM dithiothreitol [added just before use]; pH to 7.0 with NaOH);
- 20 µL of 250 mM Na-orthovanadate;
- and 5 µL of 10 mM farnesyl diphosphate (FPP, dry-evaporated and dissolved in 0.2 M ammonium carbamate and 50% ethanol, Sigma);

The reaction was started by the addition of 35 µL of cleared lysate. After mixing, the glass tube was incubated at 30°C with mild agitation for 2 hours. The reaction mixture was then extracted with 2 mL ethylacetate and vortexed for 10 sec. After centrifugation for 10 min. at 1,200 x g, the ethylacetate layer was collected, dried over a sodium sulphate column and used for GC-MS analysis. To this end, analytes from 1 µL of the ethylacetate layer were separated using gas chromatography on a 7890A GC system (Agilent Technologies) equipped with a 30 m x 0.25 mm, 0.25 mm film thickness column (ZB-5, Phenomenex) using helium as carrier gas at a flow rate of 1 mL/min. The injector (7683B Series, Agilent Technologies) was used in splitless mode with the inlet temperature set to 250°C. The initial oven temperature of 55°C was increased after 1 min. to 300°C at a rate of 15°C/min and held for 5 min. at 300°C. The GC was coupled to a mass-selective detector (model 5975C, Agilent Technologies). Compounds were identified and quantified by their mass spectra, retention times and surface area of MS chromatograms in comparison with those of an authentic standard of valencene. Germacrene A was identified by the incidence of its Cope-reaction product β-elemene, and quantified by comparison of its total-ion-count surface area to that of valencene.

The amount of cleared lysate (35 µL) and the reaction time (2 hours) in this assay were chosen in the range where the amount of valencene formed is linearly dependent on the amount of lysate. This approach secured that valencene synthase mutants with improved productivity are identifiable by this assay.

To compensate the valencene productivities of the different valencene synthase mutants for differences in their expression level, the relative amount of the valencene synthase protein in each cleared lysate was quantified. From each cleared lysate, 60 µL was added to 20 µL of 4x sample buffer (composition below), incubated for 2 min. at 100°C, and stored frozen. For the analysis, this stock solution was subsequently diluted 1:50 in 1x sample buffer, incubated for 2 min. at 100°C, and briefly centrifuged. 10 µL was then loaded on a 12-wells RunBlue SDS-PAGE gel (4-20%) (Expedeon, Harston, Cambridgeshire, UK). On each gel, also a negative control (cleared lysate of *E. coli* BL21(DE3) containing an empty pACYCDuet-1 vector) and a positive control (cleared lysate of *E. coli* BL21(DE3) containing pACYCDuet-1 with the gene encoding the wild type valencene synthase) were loaded. Gels were run in running buffer (composition below) under cooled conditions for 30 min. at 25 mA/gel, followed by 60 min. at 50 mA/gel. Gels were stained using SYPRO Ruby protein gel stain (Invitrogen, Breda, The Netherlands). The staining procedure included fixation for 30 min. in 40% (v/v) ethanol + 10% (v/v) acetic acid solution, a single wash step in demi water, followed by 4 hours incubation in non-diluted SYPRO Ruby protein gel stain in the dark. Subsequently gels were washed for 1 hour in 10% (v/v) ethanol + 7% (v/v) acetic acid solution, and then briefly washed in demi water. This staining procedure did yield a linear response curve.

Protein bands were quantified by scanning the gels on an Ettan DIGE Imager (GE Healthcare, Diegem, Belgium) using Poststain mode, 100 µm pixels, Matrix type: Gel, and the Sypro Ruby 1 channel (Excitation Filter 480/30 nm, Emission Filter 595/25 nm, exp 0.2). Bands were detected by ImageQuant TL software package (GE Healthcare, Diegem, Belgium) using manual lane creation, rolling-ball background subtraction and manual peak detection.

Lanes with the positive control always showed an extra band that ran slightly faster than the bovine serum albumin (BSA, 66 kDa) band in the lane with the molecular weight marker. Because this extra band was consistently absent from the negative control, and present in the lanes loaded with the cleared lysate from the *E. coli* clones expressing the mutant valencene synthases according to the invention, it was concluded that this band represents the valencene synthase, although the calculated molecular weight of valencene synthase (including the N-terminal His₆-tag) is 70,967 Da. Quantification of the valencene synthase band was then done relative towards the intensity of the corresponding band in the lane with the positive control, with subtraction of the background intensity in the lane with the negative control.

The composition of the sample buffer (1x) was:
- 10% (v/v) Glycerol
- 1% (w/v) Sodium Dodecyl Sulfate (SDS)
- 0.2 M Triethanolamine-HCl, pH 7.6
- 1% (w/v) Ficoll-400
- 0.006% (w/v) Phenol Red
- 0.006% (w/v) Coomassie Brilliant Blue G250
- 0.5 mM EDTA

The composition of the running buffer was:
- 0.04 M Tricine
- 0.06 M Tris
- 0.1% (w/v) Sodium Dodecyl Sulfate (SDS)
- 2.5 mM Sodium Bisulfite
- pH = 8.2

The results of these *in vitro* tests for the valencene synthase mutants with a single amino acid modification are presented in Table 1.

**Table 1: Results obtained by in-vitro testing valencene synthase mutants with a singly point mutation compared to SEQ ID NO: 2, and overview of location of the mutated amino acid positions in the 3D structural model of the valencene synthase.**

| **Position^{a}** | **WT AA** | **Mutant AA** | **Prod. (% rel. to wt)^{b}** | **Spec. Prod. (% rel. to wt)^{c}** | **Rel. Prot. Band Intensity^{d}** | **Ratio Val/Ger-A^{e}** | **Distance to nearest FPP atom** | **Location in model structure^{f}** |
|---|---|---|---|---|---|---|---|---|
| WT^{g} | - | - | 100.0 | 100.0 | | 8.6 | | |
| 16 | C | A | 166.5 | 82.5 | 1.31 | 9.7 | 40 | Cys |
| | | T | 133.1 | 86.8 | 1.31 | 7.8 | | |
| | | S | 148.6 | 95.2 | 1.43 | 7.9 | | |
| 128 | I | L | only tested in double mutant | | | | 28.6 | far |
| 171 | K | R | 107.0 | 98.6 | 1.75 | 7.7 | 25.3 | far |
| 187 | R | K | only tested in double mutant | | | | 22.5 | far |
| 225 | C | S | 117.2 | 92.9 | 1.66 | 5.3 | Cys | Cys |
| 244 | C | C | 188.9 | 77.7 | 1.77 | 10.8 | Cys | Cys |
| | | T | 127.1 | 105.2 | 1.59 | 8.0 | | |
| 300 | F | Y | 237.5 | 150.8 | 1.34 | 4.7 | 8.4 | 2nd |
| 302 | H | D | 103.9 | 42.4 | 2.25 | 3.4 | 11.4 | 2nd |
| 307 | S | T | 198.1 | 59.0 | 2.44 | 11.6 | 6.4 | 1 st |
| | | A | 243.6 | 143.4 | 1.56 | 2.7 | | |
| 319 | E | Q | 103.6 | 62.2 | 1.53 | 9.3 | 22.6 | far |
| 323 | C | A | 180.2 | 119.5 | 1.98 | 8.2 | Cys | Cys |
| 327 | C | L | 284.4 | 243.4 | 1.88 | 6.9 | Cys | Cys |
| 331 | S | G | 155.2 | 89.2 | 1.13 | 4.9 | 3.5 | 1 st |
| 334 | M | L | 174.2 | 59.4 | 2.50 | 2.6 | 3.9 | 1 st |
| 398 | V | M | only tested in double mutant | | | | 20.6 | far |
| | | I | 114.2 | 83.3 | 2.21 | 6.6 | | |
| | | T | only tested in double mutant | | | | | |
| 405 | C | T | 154.7 | 103.7 | 1.37 | 9.2 | Cys | Cys |
| | | V | 123.0 | 75.6 | 1.49 | 9.5 | | |
| 409 | Y | F | 187.6 | 224.1 | 1.40 | 6.0 | 7.3 | 1 st |
| 410 | I | F | 397.8 | 261.2 | 0.99 | 6.6 | 11.2 | 2nd |
| | | V | 314.6 | 162.7 | 2.54 | 7.6 | | |
| | | L | 155.4 | 291.0 | 0.89 | 7.6 | | |
| 412 | A | G | 184.3 | 175.5 | 1.69 | 8.9 | 11.4 | 2nd |
| 436 | V | L | 223.9 | 175.7 | 0.83 | 8.6 | 8.1 | 2nd |
| | | K | 192.4 | 80.8 | 1.55 | 7.8 | | |
| | | T | 168.3 | 151.2 | 1.86 | 9.1 | | |
| | | W | only tested in double mutant | | | | | |
| 438 | S | T | 210.0 | 90.2 | 1.98 | 3.9 | 4.4 | 1 st |
| 439 | S | G | 249.2 | 85.0 | 2.13 | 11.7 | 3.7 | 1 st |
| | | A | 159.9 | 120.3 | 1.22 | 8.7 | | |
| 444 | L | I | 126.6 | 52.2 | 1.58 | 23.7 | 5.5 | 1 st |
| | | V | 107.9 | 120.0 | 1.50 | 16.7 | | |
| 448 | A | S | 119.2 | 104.1 | 1.05 | 7.8 | 14.1 | 2nd |
| 449 | L | F | 158.5 | 175.4 | 1.46 | 9.9 | 17.7 | far |
| | | Y | only tested in double mutant | | | | | |
| | | I | only tested in double mutant | | | | | |
| 450 | I | L | 168.1 | 93.9 | 1.64 | 8.1 | 17.8 | far |
| | | M | 120.2 | 103.9 | 0.98 | 8.5 | | |
| | | V | only tested in double mutant | | | | | |
| | | F | only tested in double mutant | | | | | |
| 463 | Q | S | only tested in double mutant | | | | 22 | far |
| | | E | 123.0 | 60.4 | 1.48 | 8.9 | | |
| | | G | only tested in double mutant | | | | | |
| | | W | only tested in double mutant | | | | | |
| 488 | F | Y | 152.2 | 84.3 | 1.30 | 6.4 | 10 | 2nd |
| | | S | 242.4 | 216.9 | 1.87 | 3.3 | | |
| | | H | 277.5 | 132.2 | 3.50 | 6.2 | | |
| 490 | D | N | 302.7 | 258.3 | 1.96 | 4.1 | 9.6 | 2nd |
| | | A | 231.1 | 188.3 | 2.05 | 2.4 | | |
| | | T | 207.2 | 134.4 | 2.03 | 2.0 | | |
| | | F | 133.5 | 66.1 | 1.47 | 4.8 | | |
| 492 | Q | A | 161.2 | 175.8 | 1.48 | 7.3 | 12 | 2nd |
| | | K | 264.4 | 184.7 | 0.93 | 8.5 | | |
| 502 | E | Q | 319.1 | 148.1 | 1.40 | 9.7 | 14.4 | 2nd |
| 503 | C | S | 198.8 | 117.8 | 1.22 | 7.0 | Cys | Cys |
| 507 | D | E | 264.8 | 195.8 | 1.24 | 8.1 | 20.5 | far |
| | | Q | 165.3 | 116.9 | 1.86 | 8.5 | | |
| 527 | C | T | 173.5 | 66.5 | 1.69 | 9.7 | Cys | Cys |
| | | S | 134.8 | 99.2 | 1.25 | 7.5 | | |
| | | A | 127.3 | 40.1 | 2.29 | 7.4 | | |
| 556 | V | T | 272.2 | 507.2 | 0.86 | 4.3 | 6.8 | 1 st |
| 559 | F | H | 152.5 | 94.1 | 1.38 | 3.1 | 10.2 | 2nd |
| | | L | 183.7 | 103.7 | 1.62 | 3.3 | | |
| | | V | 126.2 | 58.3 | 1.84 | 5.3 | | |
| 560 | M | L | 199.9 | 73.9 | 1.97 | 5.0 | 3.8 | 1 st |
| 566 | L | S | 317.3 | 150.2 | 1.52 | 5.7 | 8.7 | 2nd |
| | | A | 195.1 | 119.4 | 1.06 | 5.7 | | |
| | | G | 141.3 | 141.8 | 1.66 | 4.8 | | |
| 568 | T | S | 321.0 | 130.3 | 1.60 | 3.6 | 10.1 | 2nd |
| 569 | H | I | 382.7 | 147.1 | 1.89 | 8.1 | 5.7 | 1 st |
| | | V | 203.4 | 142.8 | 1.21 | 5.1 | | |
| 570 | S | T | 258.5 | 200.5 | 2.15 | 2.3 | 10.1 | 2nd |
| | | G | 288.3 | 271.1 | 1.78 | 3.4 | | |
| | | A | 116.2 | 44.1 | 1.92 | 7.6 | | |
| | | P | 140.3 | 43.6 | 2.32 | 1.8 | | |

| | | | | | | | | |
|---|---|---|---|---|---|---|---|---|
| ^{a} Position of the Valencene Synthase amino acid residue mutated; residue numbering starting from the N-terminal methionine residue in SEQ ID NO: 2 (= Met-1) ^{b} Valencene productivity relative to that obtained with wild type valence synthase (= control) {( Val[Sample] / Val[Control]) x 100%} ^{c} Specific valencene productivity relative to that of the wild type valence synthase (= control) {(Val[Sample] / Prot[Sample) / (Val[Control] / Prot[Control])} x 100% ^{d} Intensity of the valencene synthase protein band on an SDS-PAGE gel relative to the intensity of that band in the positive control ^{e} Ratio valencene to Germacrene A formed from FPP in the standard productivity assay. Nb. Under the GC conditions applied, Germacrene A is actually detected as its thermal rearrangement product β-elemene ^{f} 1st: first shell amino acid residue; 2nd: second shell amino acid residue; far: amino acid residues that are not residing in the first and second shell; Cys: cysteine residue ^{g} Valencene Synthase wild-type (SEQ ID NO: 2) | | | | | | | | |

The results in Table 1 show that the valencene synthase mutants with a single point mutation according to the invention demonstrate clearly improved *in-vitro* valencene productivities. The valencene productivity of these mutants relative to that obtained in this test system with the wild type valencene synthase ranges from 103.6% (ValC:E319Q) to 397.8% (ValC:I410F). For many of the valencene synthase mutants tested this improved valencene productivity goes together with an improved specific valencene productivity pointing to the fact that the improved valencene productivity is not primarily the result of an increased expression of these mutants in *E. coli.* Other valencene synthase mutants according to the invention are clear expression mutants as the intensity of the valencene synthase band on SDS-PAGE gel compared to a positive control is much higher than that of the band belonging to the wild type valencene synthase. Finally, valencene synthase mutants have been obtained that produce much less germacrene A compared to valencene as the wild type valencene synthase.

The data in Table 2 prove that also the valencene synthase mutants containing at least two point mutations according to the invention show clearly improved *in-vitro* valencene productivities, ranging from 100.6% (ValC:V436W,L449Y) to 266.7% (ValC:Q463S,F488S) compared to the wild type valencene synthase.

**Table 2: Results obtained by in-vitro testing valencene synthase mutants with two point mutations compared to SEQ ID NO: 2^{a}.**

| **Position 1** | **WT amino acid** | **Position 2** | **WT amino acid** | **Mutation position 1** | **Mutation position 2** | **Productivit y(% rel. to weight)^{b}** | **Specific Producitvity (% rel to wt)** | **Rel. Prot. Band Intensity** | **Ratio Val/Ger-A** |
|---|---|---|---|---|---|---|---|---|---|
| 128 | I | 302 | H | L | D | 114.1 | 69.0 | 1.07 | 4.5 |
| 398 | V | 449 | L | I | Y | 116.8 | 118.0 | 0.71 | 6.7 |
| | | | | T | I | 116.4 | 51.7 | 1.46 | 9.0 |
| 463 | Q | 488 | F | S | S | 266.7 | 178.0 | 1.97 | 3.4 |
| | | | | E | Y | 226.9 | 122.8 | 2.43 | 6.5 |
| | | | | G | Y | 144.0 | 99.4 | 1.91 | 6.4 |
| | | | | W | H | 137.8 | 99.9 | 0.99 | 5.1 |
| 436 | V | 449 | L | L | F | 133.6 | 123.0 | 1.43 | 6.9 |
| | | | | K | I | 114.5 | 89.2 | 1.69 | 5.6 |
| | | | | W | Y | 100.6 | 140.0 | 1.16 | 3.3 |
| 436 | V | 450 | I | L | V | 190.4 | 109.6 | 1.59 | 8.4 |
| | | | | K | L | 177.2 | 212.2 | 1.35 | 8.3 |
| | | | | W | F | 133.0 | 92.1 | 1.04 | 7.3 |
| 187 | R | 398 | V | K | M | 47.8 | 170.5 | 0.45 | 3024.9 |

| | | | | | | | | | |
|---|---|---|---|---|---|---|---|---|---|
| ^{a} For the meaning of the different column headers, see table 1. | | | | | | | | | |

### Example 3: Construction of Rhodobacter sphaeroides strains expressing improved valencene synthase mutants

### Description of novel synthetic mevalonate operon in plasmid pJ241-59440-mev A2415 T4088 mod1

A synthetic DNA fragment containing a modified version of the mevalonate operon from *P. zeaxanthanafacaens* (described in WO 06/018211 and Humbelin, M., et al., Gene (2002) 297: 129-139 [Accession AJ431696]) was purchased from DNA2.0 inserted in pJ241 (proprietary plasmid from DNA2.0). The plasmid was designated pJ241-59440-mev_A2415_T4088_mod1 and its nucleotide sequence is shown in SEQ ID NO: 6. The modifications compared to the wild type nucleotide sequence, which were meant to facilitate further cloning steps and were effected by silent mutations, involved the removal of the recognition sites for restriction enzymes *Bam*HI*, Bgl*II*, Eco*RI and *Nde*I and introduction of unique recognition sites for restriction enzymes *Kpn*I and *Xho*I within the mevalonate operon insert (position 1240 to position 7875 in SEQ ID NO: 6).

### Subcloning of the novel synthetic mevalonate operon into pBBR-based plasmid resulting in plasmid p-mevAT-PcrtE-trx

Plasmid pBBR-K-mev-op-4-89-P*crtE-trx-ualFpoR-rev* (described in the patent applications International patent application number PCT/NL2010/050848 and European patent application number EP09174999.0) was digested *with Ase*I and the larger fragment was isolated and self-ligated resulting in plasmid p-m-489-P*crtE-trx.* The new plasmid was cut with *Zra*I and the 6,636 bp fragment was isolated. Plasmid pJ241-59440-mev_A2415_T4088_mod1 was also cut with *Zra*I and the 5,569 bp fragment was isolated and ligated with the 6,636 bp fragment originating from p-m*-*489-P*crtE-trx.* The new construct was checked for insertion in the correct orientation and designated p-mevAT-P*crtE-trx*.

### Construction of plasmids p-mevAT-PcrtE-trx-ValC mutant and p-mevAT-PcrtE-trx-ValC wt by insertion of the genes encoding the Valencene Synthase mutants according to the invention and the wild type Valencene Synthase into plasmid p-mevAT-PcrtE-trx

Synthetic DNA fragments with the genes encoding the Valencene Synthase mutants according to the current invention (ValC_mutant) and with the gene encoding the Valencene Synthase wild type (ValC_wt, SEQ ID NO: 2) were obtained from DNA2.0 (Menlo Park, CA, USA) in general cloning vector pJ201 (proprietary vector DNA2.0). The Valencene Synthase genes had been codon optimized by DNA2.0 for improved expression in *Rhodobacter.* The plasmids were designated pJ201:ValC_mutant or pJ201:ValC_wt. The nucleotide sequence of pJ201:ValC_wt is shown in SEQ ID NO: 7. The nucleotide sequence of the pJ201:ValC_mutant plasmids is identical to that of pJ201:ValC_wt except for the nucleotide substitutions needed for the formation of the Valencene Synthase mutants according to the current invention.

Plasmid p-mevAT-P*crtE-trx* was cut *with Ase*I and *Bam*HI and the 12,136 bp fragment was isolated. Plasmids pJ201:ValC_mutant (see Table 3, column 2) and plasmid pJ201:ValC_wt were cut with *Nde*I and *Bam*HI and the 1,777 bp fragments were isolated and ligated with the 12,136 bp fragment from p-mevAT-P*crtE-trx*, resulting in the 18 p-mevAT-P*crtE-trx*-ValC_mutant plasmids listed in Table 3, column 3, and in p-mevAT-P*crtE-trx*-ValC_wt.

### Construction of plasmids p-m-4-89-PcrtE-trx-ValC mutant by replacing the synthetic mevalonate operon mev A2415 T4088 mod1 in plasmids p-mevAT-PcrtE-trx-ValC mutant with mevalonate operon mev-op-4-89

Plasmid p-m-4-89-P*crtE*-*trx*-*valC*-*opt* is essentially the same plasmid as pBBR-K-mev-op-4-89-P*crtE-trx-valFpoR*-rev except for the fact that the *valF* coding region from pBBR-K-mev-op-4-89-P*crtE-trx-valFpoR*-rev was replaced by the *valC-opt* coding region (SEQ ID NO: 18 in PCT/NL2010/050848 and EP09174999, which sequence is incorporated herein by reference).

Plasmid p-m-4-89-P*crtE-trx-valC-opt* was cut with *Zra*I*, Blp*I and *Sac*I and the 6,739 bp *Zra*I *- Blp*I fragment was isolated. The p-mevAT-P*crtE-trx*-ValC_mutant plasmids (Table 3, column 3) were cut with *Zra*I*, Blp*I and *Asi*SI and the 7,234 bp *Zra*I *- Blp*I fragments were isolated. These fragments were subsequently ligated with the 6,739 bp fragment from p-m-4-89-P*crtE-trx-valC-opt* leading to the plasmids p-m-4-89-P*crtE-trx*-ValC_mutant (Table 4, column 3).

### Construction of Rhodobacter sphaeroides Rs265-9c strains expressing the mevalonate pathway and mutant valencene synthases according to the invention

*E. coli* S17-1 was transformed with the plasmids shown in Table 4, column 3, and the resulting strains were used to transfer the plasmids into *R. sphaeroides Rs265-9c* by conjugation as described in PCT/NL2010/050848 and EP09174999.

**Table 3. Nomenclature of plasmids constructed from the 12,136 bp p-mevAT-PcrtE-trx fragment and the 1,777 bp fragments originating from the pJ201:mutant plasmids**

| **Recipient plasmid (12,136 bp *Ase*I - *Bam*HI fragment)** | **Origin of insert (1,777 bp *Nde*I *- Bam*HI fragment comprising *valC*-mut)** | **New plasmid constructed** |
|---|---|---|
| | pJ201:V556T | p-mevAT-P*crtE-trx*-ValC_V556T |
| | pJ201:I410L | p-mevAT-P*crtE-trx-*ValC_I410L |
| | pJ201:S570G | p-mevAT-P*crtE-trx*-ValC_S570G |
| | pJ201:I410F | p-mevAT-P*crtE-trx*-ValC_I410F |
| | pJ201:D490N | p-mevAT-P*crtE-trx*-ValC_D490N |
| | pJ201:C327L | p-mevAT-P*crtE-trx*-ValC_C327L |
| | pJ201:I410V | p-mevAT-P*crtE-trx*-ValC_I410V |
| | pJ201:L566S | p-mevAT-P*crtE-trx*-ValC_L566S |
| | pJ201:E502Q | p-mevAT-P*crtE-trx*-ValC_E502Q |
| p-mevAT-P*crtE-trx* | pJ201:H569I | p-mevAT-P*crtE-trx*-ValC_H569I |
| | pJ201:F488H | p-mevAT-P*crtE-trx*-ValC_F488H |
| | pJ201:T568S | p-mevAT-P*crtE-trx*-ValC_T568S |
| | pJ201:Q492K | p-mevAT-P*crtE-trx*-ValC_Q492K |
| | pJ201:Q463S-F488S | p-mevAT-P*crtE-trx*-ValC_Q463S-F488S |
| | pJ201:F300Y | p-mevAT-P*crtE-trx*-ValC_F300Y |
| | pJ201:Q463E-F488Y | p-mevAT-P*crtE-trx*-ValC_Q463E-F488Y |
| | pJ201:S439G | p-mevAT-P*crtE-trx*-ValC_S439G |
| | pJ201:C503S | p-mevAT-P*crtE-trx-*ValC_C503S |

**Table 4. Nomenclature of plasmids constructed from the 6,739 bp p-m-4-89-PcrtE-trx-valC-opt fragment and the 7,234 bp fragments originating from the p-mevAT-PcrtE-trx-ValC_Mutant plasmids**

| **Recipient plasmid (6,739 bp *Zra*I *- Blp*I fragment)** | **Origin of insert (7,234 bp *Zra*I *- Blp*I fragment)** | **New plasmid constructed** |
|---|---|---|
| | p-mevAT-P*crtE-trx*-ValC_V556T | p-m-4-89-P*crtE-trx*-ValC_V556T |
| | p-mevAT-P*crtE-trx*-ValC_I410L | p-m-4-89-P*crtE-trx*-ValC_I410L |
| | p-mevAT-P*crtE-trx*-ValC_S570G | p-m-4-89-P*crtE-trx*-ValC_S570G |
| | p-mevAT-P*crtE-trx*-ValC_I410F | p-m-4-89-P*crtE-trx*-ValC_I410F |
| | p-mevAT-P*crtE-trx*-ValC_D490N | p-m-4-89-P*crtE-trx*-ValC_D490N |
| | p-mevAT-P*crtE-trx*-ValC_C327L | p-m-4-89-P*crtE-trx*-ValC_C327L |
| | p-mevAT-P*crtE-trx*-ValC_I410V | p-m-4-89-P*crtE-trx*-ValC_I410V |
| | p-mevAT-P*crtE-trx*-ValC_L566S | p-m-4-89-P*crtE-trx*-ValC_L566S |
| p-m-4-89-P*crtE-trx-valC-opt* | p-mevAT-P*crtE-trx*-ValC_E502Q | p-m-4-89-P*crtE-trx*-ValC_E502Q |
| | p-mevAT-*PcrtE-trx*-ValC_H569I | p-m-4-89-P*crtE-trx*-ValC_H569I |
| | p-mevAT-P*crtE-trx*-ValC_F488H | p-m-4-89-P*crtE-trx*-ValC_F488H |
| | p-mevAT-P*crtE-trx*-ValC_T568S | p-m-4-89-P*crtE-trx*-ValC T568S |
| | p-mevAT-P*crtE-trx*-ValC_Q492K | p-m-4-89-P*crtE-trx*-ValC_Q492K |
| | p-mevAT-P*crtE-trx*-ValC_Q463S-F488S | p-m-4-89-P*crtE-trx*-ValC_Q463S-F488S |
| | p-mevAT-P*crtE-trx*-ValC_F300Y | p-m-4-89-P*crtE-trx*-ValC_F300Y |
| | p-mevAT-P*crtE-trx*-ValC_Q463E-F488Y | p-m-4-89-Pc*rtE-trx*-ValC_Q463E-F488Y |
| | p-mevAT-P*crtE-trx*-ValC_S439G | p-m-4-89-P*crtE-trx*-ValC_S439G |
| | p-mevAT-P*crtE-trx*-ValC_C503S | p-m-4-89-P*crtE-trx*-ValC_C503S |

### Example 4: Cultivation of Rhodobacter sphaeroides strains under standard shake-flask conditions and evaluation of valencene production.

### Preparation of frozen cell stocks

Frozen cell stocks of *R. sphaeroides* strains were prepared by introducing a loop-full of frozen cells into 2 mL RS102 medium containing 50 mg/L kanamycin (if applicable for plasmid maintenance). The preculture was grown at 30°C with agitation at 220 rpm for 24 h. A 250 µL aliquot of preculture was transferred to 25 mL of RS102 medium containing 50 mg/L kanamycin to initiate (t = 0) growth. The 25 mL main culture was grown in a 250-mL baffled Erlenmeyer flasks at 30°C with agitation at 220 rpm for about 24 h. Bacterial cell cultures were mixed with sterile anhydrous glycerol and sterile water so as to reach a final glycerol content of 25% and a final optical density at 660 nanometers (OD₆₆₀) of 12. The resulting cell suspension was aseptically distributed in 1.2 mL-aliquots into 2 mL-cryovials then frozen at -80°C until used.

### Shake-flask procedure

Inoculants of *R. sphaeroides* strains were started by introducing 250 µL of a thawed and homogenized frozen cell stock into 25 mL of RS102 medium containing 50 mg/L of kanamycin (if applicable for plasmid maintenance). Precultures were grown in 250-mL baffled Erlenmeyer flasks for 24-28 h at 30°C with agitation at 220 rpm. A suitable aliquot of preculture was transferred to 22.5 mL of RS102 medium containing 50 mg/L of kanamycin (if applicable for plasmid maintenance) to initiate (t = 0) shake-flask experiments with an initial optical density at 660 nm (OD₆₆₀) of 0.16. Main cultures were grown in 250-mL baffled Erlenmeyer flasks at 30°C with agitation at 220 rpm. After 8 h cultivation, 2.5 mL of n-dodecane were added to the bacterial culture. Shake-flask cultivation continued at 30°C with agitation at 220 rpm for 72 h from inoculation. Each seed culture served to inoculate two duplicate shake-flasks with a final volume of 25 mL whole broth, composed of culture medium and n-dodecane for in situ product recovery. Samples (0.5 mL) of biphasic culture broth were removed at 24 h intervals and analyzed for growth (OD₆₆₀), pH, and glucose in supernatant. At the end of the experiments (t = 72 h), the biphasic culture broth was analyzed for presence of valencene (see analytical methods below). At the end of the experiments, 10 µL of culture broth were aseptically plated on general cultivation count agar plates (Becton Dickinson GmbH, Heidelberg, Germany) and incubated at 37°C for 24 h to test for contamination.

### Analytical methods

### Sample preparation for analysis of isoprenoid content in whole broth

In a typical procedure, 400 µL whole broth samples are transferred to a disposable sterile 15 mL polypropylene conical tube, treated with 4 mL acetone, vigorously shaken on an IKA Vibrax orbital shaker at 1,500 rpm for 20 min, then incubated in a bench top ultrasonic bath for 30 min at ambient temperature. Finally samples are centrifuged at maximum speed and the supernatant transferred to amber chromatography vials for analysis by gas chromatography (see below). Product yields are determined based on calibration curves established using a standard solution of authentic valencene prepared as follows: 0.5 mL of authentic valencene are added into a 10 mL volumetric flask and dissolved with analytical grade n-dodecane. Aliquots of valencene standard solution (20, 40 and 80 µl) are transferred to disposable sterile 15 mL polypropylene conical tubes, treated with deionized sterile water (380, 360, and 320 µL, respectively) and 4 mL acetone. Each mixture is homogenized vigorously on a vortex shaker then transferred to amber chromatography vials for analysis by gas chromatography, wherefrom a calibration curve is derived.

### Gas Chromatography

Gas chromatography is performed on a Hewlett-Packard GC 6890 instrument equipped with a Restek Rtx-5Sil MS capillary column (30.0 m x 0.28 mm x 0.5 µm). The injector and FID detector temperatures are set to 300°C and 250°C, respectively. Gas flow through the column is set at 1.5 mL/min. The oven initial temperature is held at 70°C for 0.5 min, increased to 150°C at a rate of 20°C/min, then increased to 205°C at a rate of 5°C/min, further increased to 300°C at a rate of 40°C/min, then cooled down to 60°C and held at that temperature for 3 min until the next injection. Injected sample volume is 1 µL with a 4:1 split-ratio. Product yields are determined based on calibration curves established for authentic samples. Germacrene A is detected as β-Elemene and quantified with the response factor determined for valencene. The content of Valencene and β-element in the organic phase wis extrapolated from whole broth analyses. The measured concentration of Valencene and β-Elemene in whole broth is set in relation to the one found for n-dodecane.

### Example 5: In vivo expression of C. noothatensis valencene synthase in yeast

The full length open reading frame encoding the *C. nootkatensis* valencene synthase (ValC, SEQ ID: NO:2) was amplified from plasmid pAC-65-3 with the primers 65-3ATGDuetFw
5'-tatatggatccATGGCTGAAATGTTTAATGGAAATTCCAGC-3'
(*Bam*HI recognition site underlined), and DuetAS1 5'-GATTATGCGGCCGTGTACAA-3' in a manner as described in Example 9 of International patent application number PCT/NL2010/050848, which Example is incorporated herein by reference. Variants of this valenene synthase, such as valencence synthases comprising a sequence as shown in SEQ ID NO: 3 or 4 can be expressed in yeast in a similar manner, using common general knowledge and the information disclosed herein.

### Example 6: Expression of ValC in plants

The valencene synthase comprising a sequence according to SEQ ID NO: 2 was expressed in a plant in a manner as described in Example 10 of International patent application number PCT/NL2010/050848, which Example is incorporated herein by reference. Variants of this valenene synthase, such as valencence synthases comprising a sequence as shown in SEQ ID NO: 3 or 4 can be expressed in a plant in a similar manner, using common general knowledge and the information disclosed herein.

### SEQUENCES

SEQ ID NO: 1
   *valC*
   *Chamaecyparis nootkatensis*
   Nucleotide sequence 
ATGGCTGAAATGTTTAATGGAAATTCCAGCAATGATGGAAGTTCTTGCATGCCCGTGAAGGACGCCCTTCGTC
GGACTGGAAATCATCATCCTAACTTGTGGACTGATGATTTCATACAGTCCCTCAATTCTCCATATTCGGATTC
TTCATACCATAAACATAGGGAAATACTAATTGATGAGATTCGTGATATGTTTTCTAATGGAGAAGGCGATGAG
TTCGGTGTACTTGAAAATATTTGGTTTGTTGATGTTGTACAACGTTTGGGAATAGATCGACATTTTCAAGAGG
AAATCAAAACTGCACTTGATTATATCTACAAGTTCTGGAATCATGATAGTATTTTTGGCGATCTCAACATGGT
GGCTCTAGGATTTCGGATACTACGACTGAATAGATATGTCGCTTCTTCAGATGTTTTTAAAAAGTTCAAAGGT
GAAGAAGGACAATTCTCTGGTTTTGAATCTAGCGATCAAGATGCAAAATTAGAAATGATGTTAAATTTATATA
AAGCTTCAGAATTAGATTTTCCTGATGAAGATATCTTAAAAGAAGCAAGAGCGTTTGCTTCTATGTACCTGAA
ACATGTTATCAAAGAATATGGTGACATACAAGAATCAAAAAATCCACTTCTAATGGAGATAGAGTACACTTTT
AAATATCCTTGGAGATGTAGGCTTCCAAGGTTGGAGGCTTGGAACTTTATTCATATAATGAGACAACAAGATT
GCAATATATCACTTGCCAATAACCTTTATAAAATTCCAAAAATATATATGAAAAAGATATTGGAACTAGCAAT
ACTGGACTTCAATATTTTGCAGTCACAACATCAACATGAAATGAAATTAATATCCACATGGTGGAAAAATTCA
AGTGCAATTCAATTGGATTTCTTTCGGCATCGTCACATAGAAAGTTATTTTTGGTGGGCTAGTCCATTATTTG
AACCTGAGTTCAGTACATGTAGAATTAATTGTACCAAATTATCTACAAAAATGTTCCTCCTTGACGATATTTA
TGACACATATGGGACTGTTGAGGAATTGAAACCATTCACAACAACATTAACAAGATGGGATGTTTCCACAGTT
GATAATCATCCAGACTACATGAAAATTGCTTTCAATTTTTCATATGAGATATATAAGGAAATTGCAAGTGAAG
CCGAAAGAAAGCATGGTCCCTTTGTTTACAAATACCTTCAATCTTGCTGGAAGAGTTATATCGAGGCTTATAT
GCAAGAAGCAGAATGGATAGCTTCTAATCATATACCAGGTTTTGATGAATACTTGATGAATGGAGTAAAAAGT
AGCGGCATGCGAATTCTAATGATACATGCACTAATACTAATGGATACTCCTTTATCTGATGAAATTTTGGAGC
AACTTGATATCCCATCATCCAAGTCGCAAGCTCTTCTATCATTAATTACTCGACTAGTGGATGATGTCAAAGA
CTTTGAGGATGAACAAGCTCATGGGGAGATGGCATCAAGTATAGAGTGCTACATGAAAGACAACCATGGTTCT
ACAAGGGAAGATGCTTTGAATTATCTCAAAATTCGTATAGAGAGTTGTGTGCAAGAGTTAAATAAGGAGCTTC
TCGAGCCTTCAAATATGCATGGATCTTTTAGAAACCTATATCTCAATGTTGGCATGCGAGTAATATTTTTTAT
GCTCAATGATGGTGATCTCTTTACACACTCCAATAGAAAAGAGATACAAGATGCAATAACAAAATTTTTTGTG
GAACCAATCATTCCATAG
SEQ ID NO: 2
   ValC
   *Chamaecyparis nootkatensis*
   Amino acid sequence 
MAEMFNGNSSNDGSSCMPVKDALRRTGNHHPNLWTDDFIQSLNSPYSDSSYHKHREILIDEIRDMFSNGEGDE
FGVLENIWFVDVVQRLGIDRHFQEEIKTALDYIYKFWNHDSIFGDLNMVALGFRILRLNRYVASSDVFKKFKG
EEGQFSGFESSDQDAKLEMMLNLYKASELDFPDEDILKEARAFASMYLKHVIKEYGDIQESKNPLLMEIEYTF
KYPWRCRLPRLEAWNFIHIMRQQDCNISLANNLYKIPKIYMKKILELAILDFNILQSQHQHEMKLISTWWKNS
SAIQLDFFRHRHIESYFWWASPLFEPEFSTCRINCTKLSTKMFLLDDIYDTYGTVEELKPFTTTLTRWDVSTV
DNHPDYMKIAFNFSYEIYKEIASEAERKHGPFVYKYLQSCWKSYIEAYMQEAEWIASNHIPGFDEYLMNGVKS
SGMRILMIHALILMDTPLSDEILEQLDIPSSKSQALLSLITRLVDDVKDFEDEQAHGEMASSIECYMKDNHGS
TREDALNYLKIRIESCVQELNKELLEPSNMHGSFRNLYLNVGMRVIFFMLNDGDLFTHSNRKEIQDAITKFFV
EPIIP
SEQ ID NO: 3 
MAEMFNGNSSNDGSSXMPVKDALRRTGNHHPNLWTDDFIQSLNSPYSDSSYHKREILIDEIRDMFSNGEGDE
FGVLENIWFVDVVQRLGIDRHFQEEIKTALDYIYKFWNHDSIFGDLNMVALGFRXLRLNRYVASSDVFKKFKG
EEGQFSGFESSDQDAKLEMMLNLYXASELDFPDEDILKEAXAFASMYLKHVIKEYGDIQESKNPLLMEIEYTF
KYPWRXRLPRLEAWNFIHIMRQQDXNISLANNLYKIPKIYMKKILELAILDFNILQSQHQHEMKLISTWWKNS
SAIQLDFXRXRHIEXYFWWASPLFEPXFSTXRINXTKLXTKXFLLDDIYDTYGTVEELKPFTTTLTRWDVSTV
DNHPDYMKIAFNFSYEIYKEIASEAERKHGPFXYKYLQSXWKSXXEXYMQEAEWIASNHIPGFDEYLMNGXKX
XGMRIXMIHXXXLMDTPLSDEILEXLDIPSSKSQALLSLITRLVDDVKDXEXEXAHGEMASSIXXYMKXNHGS
TREDALNYLKIRIESXVQELNKELLEPSNMHGSFRNLYLNVGMRXIFXXLNDGDXFXXXNRKEIQDAITKFFV
EPIIP
SEQ ID NO: 4 
MAEMFNGNSSNDGSS(CATS)MPVKDALRRTGNHHPNLWTDDFIQSLNSPYSDSSYHKHREILIDEIRDMFSN
GEGDEFGVLENIWFVDVVQRLGIDRHFQEEIKTALDYIYKFWNHDSIFGDLNMVALGFR(IL)LRLNRYVASS
DVFKKFKGEEGQFSGFESSDQDAKLEMMLNLY(KR)ASELDFPDEDILKEA(RK)AFASMYLKHVIKEYGDIQ
ESKNPLLMEIEYTFKYPWR(CS)RLPRLEAWNFIHIMRQQD(CST)NISLANNLYKIPKIYMKKILELAILDF
NILQSQHQHEMKLISTWWKNSSAIQLDF(FY)R(HD)RHIE(STA)YFWWASPLFEP(EQ)FST(CA)RIN(C
L)TKL(SG)TK(ML)FLLDDIYDTYGTVEELKPFTTTLTRWDVSTVDNHPDYMKIAFNFSYEIYKEIASEAER
KHGPF(VIMT)YKYLQS(CTV)WKS(YF)(IFVL)E(AG)YMQEAEWIASNHIPGFDEYLMNG(VLKTW)K(S
T)(SGA)GMRI(LIV)MIH(AS)(LFIY)(ILMV)LMDTPLSDEILE(QESGW)LDIPSSKSQALLSLITRLV
DDVKD(FYHS)E(DNATF)E(QAK)AHGEMASSI(EQ)(CS)YMK(DEQ)NHGSTREDALNYLKIRIES(CTS
A)VQELNKELLEPSNMHGSFRNLYLNVGMR(VT)IF(FHLV)(ML)LNDGD(LSAG)F(TS)(HIV)(SGAPT
)NRKEIQDAITKFFVEPIIP
SEQ ID NO: 5
   *pACYCDuet : ValC_wt*
   *E. coli* expression vector pACYCDuet-1 with the codon optimized gene (*valC-opt*) encoding the wild type Valencene Synthase Nucleotide sequence 
GGGGAATTGTGAGCGGATAACAATTCCCCTGTAGAAATAATTTTGTTTAACTTTAATAAGGAGATATACCATG
GGCAGCAGCCATCACCATCATCACCACAGCCAGGATCCGCATATGGCCGAAATGTTCAATGGCAATTCCAGCA
ATGATGGCAGCTCCTGCATGCCGGTCAAGGACGCGCTGCGCCGCACCGGAACCACCATCCGAACCTCTGGAC
CGACGATTTCATCCAGTCGCTGAACTCCCCCTATTCGGATTCCTCGTATCATAAACATCGCGAGATCCTGATC
GATGAGATCCGGGACATGTTCTCCAACGGCGAGGGGGATGAGTTCGGGGTCCTCGAGAACATCTGGTTCGTCG
ACGTGGTCCAGCGGCTGGGCATCGATCGGCACTTCCAGGAAGAGATCAAGACGGCCCTGGATTATATCTATAA
GTTCTGGAACCATGATAGCATCTTCGGCGACCTCAACATGGTGGCGCTGGGGTTCCGCATCCTGCGGCTCAAT
CGCTACGTGGCGTCGTCGGACGTGTTCAAGAAGTTCAAGGGCGAGGAGGGCCAGTTCTCGGGGTTCGAGAGCA
GCGATCAGGACGCCAAGCTGGAGATGATGCTGAACCTCTACAAGGCCTCGGAACTCGACTTCCCGGATGAGGA
CATCCTCAAGGAAGCGCGGGCCTTCGCGTCGATGTATCTCAAGCATGTCATCAAGGAGTATGGGGACATCCAG
GAATCGAAGAACCCCCTGCTCATGGAGATCGAGTACACCTTCAAGTACCCCTGGCGCTGCCGCCTCCCGCGGC
TGGAGGCGTGGAACTTCATCCACATCATGCGGCAGCAGGACTGCAATATCTCGCTCGCCAACAACCTCTATAA
GATCCCGAAGATCTATATGAAGAAGATCCTGGAGCTGGCGATCCTCGACTTCAACATCCTCCAGAGCCAGCAT
CAGCATGAGATGAAACTGATCAGCACGTGGTGGAAGAACTCGTCCGCGATCCAGCTCGACTTCTTCCGCCACC
GCCATATCGAGAGCTACTTCTGGTGGGCCAGCCCGCTGTTCGAGCCCGAGTTCTCCACCTGCCGCATCAACTG
CACCAAGCTGTCCACCAAGATGTTCCTCCTGGACGACATCTATGACACGTACGGGACCGTCGAGGAACTCAAG
CCGTTCACGACCACCCTCACGCGCTGGGATGTCAGCACGGTGGACAATCACCCGGACTACATGAAGATCGCGT
TCAATTTCTCCTACGAGATCTACAAGGAGATCGCGTCCGAGGCCGAGCGCAAGCACGGCCCGTTCGTGTATAA
GTATCTCCAGTCGTGCTGGAAGTCGTATATCGAGGCGTATATGCAGGAGGCCGAGTGGATCGCCTCCAACCAC
ATCCCCGGCTTCGACGAGTACCTGATGAATGGCGTGAAGAGCTCGGGGATGCGCATCCTCATGATCCATGCGC
TGATCCTGATGGATACGCCCCTGTCCGACGAGATCCTCGAGCAGCTCGACATCCCGAGCAGCAAGAGCCAGGC
CCTGCTGTCGCTCATCACGCGGCTCGTCGATGATGTGAAGGATTTCGAGGACGAGCAGGCGCATGGGGAGATG
GCCTCGTCGATCGAATGCTATATGAAGGATAATCACGGCTCCACGCGCGAGGACGCCCTGAACTACCTGAAAA
TCCGCATCGAGAGCTGCGTGCAGGAGCTCAACAAGGAACTCCTCGAACCGAGCAACATGCATGGCAGCTTCCG
CAACCTGTACCTCAACGTGGGCATGCGGGTGATCTTCTTCATGCTGAACGACGGGGACCTCTTCACCCATTCG
AATCGGAAGGAGATCCAGGATGCGATCACGAAGTTCTTCGTGGAACCGATCATCCCGTGATAAGGATCCCTGC
AGGTCGACAAGCTTGCGGCCGCATAATGCTTAAGTCGAACAGAAAGTAATCGTATTGTACACGGCCGCATAAT
CGAAATTAATACGACTCACTATAGGGGAATTGTGAGCGGATAACAATTCCCCATCTTAGTATATTAGTTAAGT
ATAAGAAGGAGATATACATATGGCAGATCTCAATTGGATATCGGCCGGCCACGCGATCGCTGACGTCGGTACC
CTCGAGTCTGGTAAAGAAACCGCTGCTGCGAAATTTGAACGCCAGCACATGGACTCGTCTACTAGCGCAGCTT
AATTAACCTAGGCTGCTGCCACCGCTGAGCAATAACTAGCATAACCCCTTGGGGCCTCTAAACGGGTCTTGAG
GGGTTTTTTGCTGAAACCTCAGGCATTTGAGAAGCACACGGTCACACTGCTTCCGGTAGTCAATAAACCGGTA
AACCAGCAATAGACATAAGCGGCTATTTAACGACCCTGCCCTGAACCGACGACCGGGTCGAATTTGCTTTCGA
ATTTCTGCCATTCATCCGCTTATTATCACTTATTCAGGCGTAGCACCAGGCGTTTAAGGGCACCAATAACTGC
CTTAAAAAAATTACGCCCCGCCCTGCCACTCATCGCAGTACTGTTGTAATTCATTAAGCATTCTGCCGACATG
GAAGCCATCACAGACGGCATGATGAACCTGAATCGCCAGCGGCATCAGCACCTTGTCGCCTTGCGTATAATAT
TTGCCCATAGTGAAAACGGGGGCGAAGAAGTTGTCCATATTGGCCACGTTTAAATCAAAACTGGTGAAACTCA
CCCAGGGATTGGCTGAGACGAAAAACATATTCTCAATAAACCCTTTAGGGAAATAGGCCAGGTTTTCACCGTA
ACACGCCACATCTTGCGAATATATGTGTAGAAACTGCCGGAAATCGTCGTGGTATTCACTCCAGAGCGATGAA
AACGTTTCAGTTTGCTCATGGAAAACGGTGTAACAAGGGTGAACACTATCCCATATCACCAGCTCACCGTCTT
TCATTGCCATACGGAACTCCGGATGAGCATTCATCAGGCGGGCAAGAATGTGAATAAAGGCCGGATAAAACTT
GTGCTTATTTTTCTTTACGGTCTTTAAAAAGGCCGTAATATCCAGCTGAACGGTCTGGTTATAGGTACATTGA
GCAACTGACTGAAATGCCTCAAAATGTTCTTTACGATGCCATTGGGATATATCAACGGTGGTATATCCAGTGA
TTTTTTTCTCCATTTTAGCTTCCTTAGCTCCTGAAAATCTCGATAACTCAAAAAATACGCCCGGTAGTGATCT
TATTTCATTATGGTGAAAGTTGGAACCTCTTACGTGCCGATCAACGTCTCATTTTCGCCAAAAGTTGGCCCAG
GGCTTCCCGGTATCAACAGGGACACCAGGATTTATTTATTCTGCGAAGTGATCTTCCGTCACAGGTATTTATT
CGGCGCAAAGTGCGTCGGGTGATGCTGCCAACTTACTGATTTAGTGTATGATGGTGTTTTTGAGGTGCTCCAG
TGGCTTCTGTTTCTATCAGCTGTCCCTCCTGTTCAGCTACTGACGGGGTGGTGCGTAACGGCAAAAGCACCGC
CGGACATCAGCGCTAGCGGAGTGTATACTGGCTTACTATGTTGGCACTGATGAGGGTGTCAGTGAAGTGCTTC
ATGTGGCAGGAGAAAAAAGGCTGCACCGGTGCGTCAGCAGAATATGTGATACAGGATATATTCCGCTTCCTCG
CTCACTGACTCGCTACGCTCGGTCGTTCGACTGCGGCGAGCGGAAATGGCTTACGAACGGGGCGGAGATTTCC
TGGAAGATGCCAGGAAGATACTTAACAGGGAAGTGAGAGGGCCGCGGCAAAGCCGTTTTTCCATAGGCTCCGC
 
CCCCCTGACAAGCATCACGAAATCTGACGCTCAAATCAGTGGTGGCGAAACCCGACAGGACTATAAAGATACC
AGGCGTTTCCCCTGGCGGCTCCCTCGTGCGCTCTCCTGTTCCTGCCTTTCGGTTTACCGGTGTCATTCCGCTG
TTATGGCCGCGTTTGTCTCATTCCACGCCTGACACTCAGTTCCGGGTAGGCAGTTCGCTCCAAGCTGGACTGT
ATGCACGAACCCCCCGTTCAGTCCGACCGCTGCGCCTTATCCGGTAACTATCGTCTTGAGTCCAACCCGGAAA
GACATGCAAAAGCACCACTGGCAGCAGCCACTGGTAATTGATTTAGAGGAGTTAGTCTTGAAGTCATGCGCCG
GTTAAGGCTAAACTGAAAGGACAAGTTTTGGTGACTGCGCTCCTCCAAGCCAGTTACCTCGGTTCAAAGAGTT
GGTAGCTCAGAGAACCTTCGAAAAACCGCCCTGCAAGGCGGTTTTTTCGTTTTCAGAGCAAGAGATTACGCGC
AGACCAAAACGATCTCAAGAAGATCATCTTATTAATCAGATAAAATATTTCTAGATTTCAGTGCAATTTATCT
CTTCAAATGTAGCACCTGAAGTCAGCCCCATACGATATAAGTTGTAATTCTCATGTTAGTCATGCCCCGCGCC
CACCGGAAGGAGCTGACTGGGTTGAAGGCTCTCAAGGGCATCGGTCGAGATCCCGGTGCCTAATGAGTGAGCT
AACTTACATTAATTGCGTTGCGCTCACTGCCCGCTTTCCAGTCGGGAAACCTGTCGTGCCAGCTGCATTAATG
AATCGGCCAACGCGCGGGGAGAGGCGGTTTGCGTATTGGGCGCCAGGGTGGTTTTTCTTTTCACCAGTGAGAC
GGGCAACAGCTGATTGCCCTTCACCGCCTGGCCCTGAGAGAGTTGCAGCAAGCGGTCCACGCTGGTTTGCCCC
AGCAGGCGAAAATCCTGTTTGATGGTGGTTAACGGCGGGATATAACATGAGCTGTCTTCGGTATCGTCGTATC
CCACTACCGAGATGTCCGCACCAACGCGCAGCCCGGACTCGGTAATGGCGCGCATTGCGCCCAGCGCCATCTG
ATCGTTGGCAACCAGCATCGCAGTGGGAACGATGCCCTCATTCAGCATTTGCATGGTTTGTTGAAAACCGGAC
ATGGCACTCCAGTCGCCTTCCCGTTCCGCTATCGGCTGAATTTGATTGCGAGTGAGATATTTATGCCAGCCAG
CCAGACGCAGACGCGCCGAGACAGAACTTAATGGGCCCGCTAACAGCGCGATTTGCTGGTGACCCAATGCGAC
CAGATGCTCCACGCCCAGTCGCGTACCGTCTTCATGGGAGAAAATAATACTGTTGATGGGTGTCTGGTCAGAG
ACATCAAGAAATAACGCCGGAACATTAGTGCAGGCAGCTTCCACAGCAATGGCATCCTGGTCATCCAGCGGAT
AGTTAATGATCAGCCCACTGACGCGTTGCGCGAGAAGATTGTGCACCGCCGCTTTACAGGCTTCGACGCCGCT
TCGTTCTACCATCGACACCACCACGCTGGCACCCAGTTGATCGGCGCGAGATTTAATCGCCGCGACAATTTGC
GACGGCGCGTGCAGGGCCAGACTGGAGGTGGCAACGCCAATCAGCAACGACTGTTTGCCCGCCAGTTGTTGTG
CCACGCGGTTGGGAATGTAATTCAGCTCCGCCATCGCCGCTTCCACTTTTTCCCGCGTTTTCGCAGAAACGTG
GCTGGCCTGGTTCACCACGCGGGAAACGGTCTGATAAGAGACACCGGCATACTCTGCGACATCGTATAACGTT
ACTGGTTTCACATTCACCACCCTGAATTGACTCTCTTCCGGGCGCTATCATGCCATACCGCGAAAGGTTTTGC
GCCATTCGATGGTGTCCGGGATCTCGACGCTCTCCCTTATGCGACTCCTGCATTAGGAAATTAATACGACTCA
CTATA
SEQ ID NO: 6
   *pJ241-59440-mev_A2415_T4088_mod1*
   Synthetic DNA fragment with a modified version of the P. *zeaxanthinifaciens* mevalonate operon cloned into vector pJ241 Nucleotide sequence 
TAGAAAAACTCATCGAGCATCAAATGAAACTGCAATTTATTCATATCAGGATTATCAATACCATATTTTTGAA
AAAGCCGTTTCTGTAATGAAGGAGAAAACTCACCGAGGCAGTTCCATAGGATGGCAAGATCCTGGTATCGGTC
TGCGATTCCGACTCGTCCAACATCAATACAACCTATTAATTTCCCCTCGTCAAAAATAAGGTTATCAAGTGAG
AAATCACCATGAGTGACGACTGAATCCGGTGAGAATGGCAAAAGTTTATGCATTTCTTTCCAGACTTGTTCAA
CAGGCCAGCCATTACGCTCGTCATCAAAATCACTCGCATCAACCAAACCGTTATTCATTCGTGATTGCGCCTG
AGCGAGGCGAAATACGCGATCGCTGTTAAAAGGACAATTACAAACAGGAATCGAGTGCAACCGGCGCAGGAAC
ACTGCCAGCGCATCAACAATATTTTCACCTGAATCAGGATATTCTTCTAATACCTGGAACGCTGTTTTTCCGG
GGATCGCAGTGGTGAGTAACCATGCATCATCAGGAGTACGGATAAAATGCTTGATGGTCGGAAGTGGCATAAA
TTCCGTCAGCCAGTTTAGTCTGACCATCTCATCTGTAACATCATTGGCAACGCTACCTTTGCCATGTTTCAGA
AACAACTCTGGCGCATCGGGCTTCCCATACAAGCGATAGATTGTCGCACCTGATTGCCCGACATTATCGCGAG
CCCATTTATACCCATATAAATCAGCATCCATGTTGGAATTTAATCGCGGCCTCGACGTTTCCCGTTGAATATG
GCTCATATTCTTCCTTTTTCAATATTATTGAAGCATTTATCAGGGTTATTGTCTCATGAGCGGATACATATTT
GAATGTATTTAGAAAAATAAACAAATAGGGGTCAGTGTTACAACCAATTAACCAATTCTGAACATTATCGCGA
GCCCATTTATACCTGAATATGGCTCATAACACCCCTTGTTTGCCTGGCGGCAGTAGCGCGGTGGTCCCACCTG
ACCCCATGCCGAACTCAGAAGTGAAACGCCGTAGCGCCGATGGTAGTGTGGGGACTCCCCATGCGAGAGTAGG
GAACTGCCAGGCATCAAATAAAACGAAAGGCTCAGTCGAAAGACTGGGCCTTTCGCCCGGGCTAATTAGGGGG
TGTCGCCCTTATTCGACTCTATAGTGAAGTTCCTATTCTCTAGAAAGTATAGGAACTTCTGAAGTGGGGACGT
CTCGATTCTGCCCGCGAAGAATGCGATGCATCCAGATGATGCAGAACGAAGAAGCGGAAGCGCCCGTGAAAGA
CCAGATGATTTCCCATACCCCGGTGCCCACGCAATGGGTCGGCCCGATCCTGTTCCGCGGCCCCGTCGTCGAG
GGCCCGATCAGCGCGCCGCTGGCCACCTACGAGACGCCGCTCTGGCCCTCGACCGCGCGGGGGGCAGGGGTTT
CCCGGCATTCGGGCGGCATCCAGGTCTCGCTGGTCGACGAACGCATGAGCCGCTCGATCGCGCTGCGGGCGCA
TGACGGGGCGGCGGCGACCGCCGCCTGGCAGTCGATCAAGGCCCGCCAGGAAGAGGTCGCGGCCGTGGTCGCC
 
ACCACCAGCCGCTTCGCCCGCCTTGTCGAGCTGAATCGCCAGATCGTGGCAACCTGCTTTACATCCGCATCG
AATGCGTGACGGGCGACGCCTCGGGTCACAACATGGTCACCAAGGCCGCCGAGGCCGTGCAGGGCTGGATTCT
GTCGGAATACCCGATGCTGGCCTATTCCACGATCTCGGGGAACCTGTGCACCGACAAGAAGGCGTCGGCGGTC
AACGGCATCCTGGGCCGCGGCAAATACGCCGTCGCCGAGGTCGAGATCCCGCGCAAGATCCTGACCCGCGTGC
TGCGCACCAGCGCCGAGAAGATGGTCCGCCTGAACTACGAGAAGAACTATGTCGGGGGTACGCTGGCGGGGTC
GCTGCGCAGTGCGAACGCGCATTTCGCCAACATGCTGCTGGGCTTCTACCTGGCGACGGGGCAGGACGCGGCC
AACATCATCGAGGCCAGCCAGGGCTTCGTCCATTGCGAGGCCCGCGGCGAGGATCTGTATTTCTCGTGCACGC
TGCCCAACCTCATCATGGGCTCGGTCGGTGCCGGCAAGGGCATCCCCTCGATCGAGGAGAACCTGTCGCGGAT
GGGCTGCCGCCAGCCGGGCGAACCCGGCGACAACGCGCGCCGTCTTGCGGCGATCTGCGCGGGCGTCGTGCTG
TGTGGTGAATTGTCGCTGCTTGCGGCCCAGACCAACCCCGGAGAGTTGGTCCGCACCCACATGGAGATGGAGC
GATGACCGACAGCAAGGATCACCATGTCGCGGGGCGCAAGCTGGACCATCTGCGTGCATTGGACGACGATGCG
GATATCGACCGGGGCGACAGCGGCTTCGACCGCATCGCGCTGACCCATCGCGCCCTGCCCGAGGTGGATTTCG
ACGCCATCGACACGGCGACCAGCTTCCTGGGCCGTGAACTGTCCTTCCCGCTGCTGATCTCGTCCATGACCGG
CGGCACCGGCGAGGAGATCGAGCGCATCAACCGCAACCTGGCCGCTGGTGCCGAGGAGGCCCGCGTCGCCATG
GCGGTGGGCTCGCAGCGCGTGATGTTCACCGACCCCTCGGCGCGGGCCAGCTTCGACCTGCGCGCCCATGCGC
CCACCGTGCCGCTGCTGGCCAATATCGGCGCGGTGCAGCTGAACATGGGGCTGGGGCTGAAGGAATGCCTGGC
CGCGATCGAGGTGCTGCAGGCGGACGGCCTGTATCTGCACCTGAACCCCCTGCAAGAGGCCGTCCAGCCCGAG
GGGGATCGCGACTTTGCCGATCTGGGCAGCAAGATCGCGGCCATCGCCCGCGACGTTCCCGTGCCCGTCCTGC
TGAAGGAGGTGGGCTGCGGCCTGTCGGCGGCCGATATCGCCATCGGGCTGCGCGCCGGCATCCGGCATTTCGA
CGTGGCCGGTCGCGGCGGCACATCCTGGAGCCGGATCGAGTATCGCCGCCGCCAGCGGGCCGATGACGACCTG
GGCCTGGTCTTCCAGGACTGGGGCCTGCAGACCGTGGACGCCCTGCGCGAGGCGCGGCCCGCGCTTGCGGCCC
ATGATGGAACCAGCGTGCTGATCGCCAGCGGCGGCATCCGCAACGGTGTCGACATGGCGAAATGCGTCATCCT
GGGGGCCGACATGTGCGGGGTCGCCGCGCCCCTGCTGAAAGCGGCCCAAAACTCGCGCGAGGCGGTTGTATCC
GCCATCCGGAAACTGCATCTGGAGTTCCGGACAGCCATGTTCCTCCTGGGTTGCGGCACGCTTGCCGACCTGA
AGGACAATTCCTCGCTTATCCGTCAATGAAAGTGCCTAAGATGACCGTGACAGGAATCGAAGCGATCAGCTTC
TACACCCCCCAGAACTACGTGGGACTGGATATCCTTGCCGCGCATCACGGGATCGACCCCGAGAAGTTCTCGA
AGGGGATCGGGCAGGAGAAAATCGCACTGCCCGGCCATGACGAGGATATCGTGACCATGGCCGCCGAGGCCGC
GCTGCCGATCATCGAACGCGCGGGTACCCAGGGCATCGACACGGTTCTGTTCGCCACCGAGAGCGGGATCGAC
AAGTCGAAGGCCGCCGCCATCTATCTGCGCCGCCTGCTGGACCTGTCGCCCAACTGCCGTTGCGTCGAGCTGA
AGCAGGCCTGCTATTCCGCGACGGCGGCGCTGCAGATGGCCTGCGCGCATGTCGCCCGCAAGCCCGACCGCAA
GGTGCTGGTGATCGCGTCCGATGTCGCGCGCTATGACCGCGAAAGCTCGGGCGAGGCGACGCAGGGTGCGGGC
GCCGTCGCCATCCTTGTCAGCGCCGATCCCAAGGTGGCCGAGATCGGCACCGTCTCGGGGCTGTTCACCGAGG
ATATCATGGATTTCTGGCGGCCGAACCACCGCCGCACGCCCCTGTTCGACGGCAAGGCATCGACGCTGCGCTA
TCTGAACGCGCTGGTCGAGGCGTGGAACGACTATCGCGCGAATGGCGGCCACGAGTTCGCCGATTTCGCGCAT
TTCTGCTATCACGTGCCGTTCTCGCGGATGGGCGAGAAGGCGAACAGCCACCTGGCCAAGGCGAACAAGACGC
CGGTGGACATGGGGCAGGTGCAGACGGGCCTGATCTACAACCGGCAGGTCGGGAACTGCTATACCGGGTCGAT
CTACCTGGCATTCGCCTCGCTGCTGGAGAACGCTCAGGAGGACCTGACCGGCGCGCTGGTCGGTCTGTTCAGC
TATGGCTCGGGTGCGACGGGCGAGTTCTTCGATGCGCGGATCGCGCCCGGTTACCGCGACCACCTGTTCGCGG
AACGCCATCGCGAATTGCTGCAGGATCGCACGCCCGTCACGTATGACGAATACGTTGCCCTGTGGGACGAGAT
CGACCTGACGCAGGGCGCGCCCGACAAGGCGCGCGGTCGTTTCAGGCTGGCAGGTATCGAGGACGAGAAGCGC
ATCTATGTCGACCGGCAGGCCTGAAGCAGGCGCCCATGCCCCGGGCAAGCTGATCCTGTCCGGGGAACATTCC
GTGCTCTATGGTGCGCCCGCGCTTGCCATGGCCATCGCCCGCTATACCGAGGTGTGGTTCACGCCGCTTGGCA
TTGGCGAGGGGATACGCACGACATTCGCCAATCTCTCGGGCGGGGCGACCTATTCGCTGAAGCTGCTGTCGGG
GTTCAAGTCGCGGCTGGACCGCCGGTTCGAGCAGTTCCTGAACGGCGACCTAAAGGTGCACAAGGTCCTGACC
CATCCCGACGATCTGGCGGTCTATGCGCTGGCGTCGCTTCTGCACGACAAGCCGCCGGGGACCGCCGCGATGC
CGGGCATCGGCGCGATGCACCACCTGCCGCGACCGGGTGAGCTGGGCAGCCGGACGGAGCTGCCCATCGGCGC
GGGCATGGGGTCGTCTGCGGCCATCGTCGCGGCCACCACGGTCCTGTTCGAGACGCTGCTGGACCGGCCCAAG
ACGCCCGAACAGCGCTTCGACCGCGTCCGCTTCTGCGAGCGGTTGAAGCACGGCAAGGCCGGTCCCATCGACG
CGGCCAGCGTCGTGCGCGGCGGGCTTGTCCGCGTGGGCGGGAACGGGCCGGGTTCGATCAGCAGCTTCGATTT
GCCCGAGGATCACGACCTTGTCGCGGGACGCGGCTGGTACTGGGTACTGCACGGGCGCCCCGTCAGCGGGACC
GGCGAATGCGTCAGCGCGGTCGCGGCGGCGCATGGTCGCGATGCGGCGCTGTGGGACGCCTTCGCAGTCTGCA
CCCGCGCGTTGGAGGCCGCGCTGCTGTCTGGGGGCAGCCCCGACGCCGCCATCACCGAGAACCAGCGCCTGCT
CGAGCGCATCGGCGTCGTGCCGGCAGCGACGCAGGCCCTCGTGGCCCAGATCGAGGAGGCGGGTGGCGCGGCC
AAAATCTGCGGCGCAGGTTCCGTGCGGGGCGATCACGGCGGGGCGGTCCTCGTGCGGATTGACGACGCGCAGG
CGATGGCTTCGGTCATGGCGCGCCATCCCGACCTCGACTGGGCGCCCCTGCGCATGTCGCGCACGGGGGCGGC
ACCCGGCCCCGCGCCGCGTGCGCAACCGCTGCCGGGGCAGGGCTGATGGATCAGGTCATCCGCGCCAGCGCGC
CGGGTTCGGTCATGATCACGGGCGAACATGCCGTGGTCTATGGACACCGCGCCATCGTCGCCGGGATCGAGCA
 
GCGCGCCCATGTGACGATCGTCCCGCGTGCCGACCGCATGTTTCGCATCACCTCGCAGATCGGGGCGCCGCAG
CAGGGGTCGCTGGACGATCTGCCTGCGGGCGGGACCTATCGCTTCGTGCTGGCCGCCATCGCGCGACACGCGC
CGGACCTGCCTTGCGGGTTCGACATGGACATCACCTCGGGGATCGATCCGAGGCTCGGGCTTGGGTCCTCGGC
GGCGGTGACGGTCGCCTGCCTCGGCGCGCTGTCGCGGCTGGCGGGGCGGGGGACCGAGGGGCTGCATGACGAC
GCGCTGCGCATCGTCCGCGCCATCCAGGGCAGGGGCAGCGGGGCCGATCTGGCGGCCAGCCTGCATGGCGGCT
TCGTCGCCTATCGCGCGCCCGATGGCGGTGCCGCGCAGATCGAGGCGCTTCCGGTGCCGCCGGGGCCGTTCGG
CCTGCGCTATGCGGGCTACAAGACCCCGACAGCCGAGGTGCTGCGCCTTGTGGCCGATCGGATGGCGGGCAAC
GAGGCCGCTTTCGACGCGCTCTACTCCCGGATGGGCGCAAGCGCAGATGCCGCGATCCGCGCGGCGCAAGGGC
TGGACTGGGCTGCATTCCACGACGCGCTGAACGAATACCAGCGCCTGATGGAGCAGCTGGGCGTGTCCGACGA
CACGCTGGACGCGATCATCCGCGAGGCGCGCGACGCGGGCGCCGCAGTCGCCAAAATCTCCGGCTCGGGGCTG
GGGGATTGCGTGCTGGCACTGGGCGACCAGCCCAAGGGTTTCGTGCCCGCAAGCATTGCCGAGAAGGGACTTG
TTTTCGATGACTGATGCCGTCCGCGACATGATCGCCCGTGCCATGGCGGGCGCGACCGACATCCGAGCAGCCG
AGGCTTATGCGCCCAGCAACATCGCGCTGTCGAAATACTGGGGCAAGCGCGACGCCGCGCGGAACCTTCCGCT
GAACAGCTCCGTCTCGATCTCGTTGGCGAACTGGGGCTCTCATACGCGGGTCGAGGGGTCCGGCACGGGCCAC
GACGAGGTGCATCACAACGGCACGCTGCTCGATCCGGGCGACGCCTTCGCGCGCCGCGCGTTGGCATTCGCTG
ACCTGTTCCGGGGGGGGAGGCACCTGCCGCTGCGGATCACGACGCAGAACTCGATCCCGACGGCGGCGGGGCT
TGCCTCGTCGGCCTCGGGGTTCGCGGCGCTGACCCGTGCGCTGGCGGGGGCGTTCGGGCTGGATCTGGACGAC
ACGGATCTGAGCCGCATCGCCCGGATCGGCAGTGGCAGCGCCGCCCGCTCGATCTGGCACGGCTTCGTCCGCT
GGAACCGGGGCGAGGCCGAGGATGGGCATGACAGCCACGGCGTCCCGCTGGACCTGCGCTGGCCCGGCTTCCG
CATCGCGATCGTGGCCGTGGACAAGGGGCCCAAGCCTTTCAGTTCGCGCGACGGCATGAACCACACGGTCGAG
ACCAGCCCGCTGTTCCCGCCCTGGCCTGCGCAGGCGGAAGCGGATTGCCGCGTCATCGAGGATGCGATCGCCG
CCCGCGACATGGCCGCCCTGGGTCCGCGGGTCGAGGCGAACGCCCTTGCGATGCACGCCACGATGATGGCCGC
GCGCCCGCCGCTCTGCTACCTGACGGGCGGCAGCTGGCAGGTGCTGGAACGCCTGTGGCAGGCCCGCGCGGAC
GGGCTTGCGGCCTTTGCGACGATGGATGCCGGCCCGAACGTCAAGCTGATCTTCGAGGAAAGCAGCGCCGCCG
ACGTGCTGTACCTGTTCCCCGACGCCAGCCTGATCGCGCCGTTCGAGGGGCGTTGAACGCGTAAGACGACCAC
TGGGTAAGGTTCTGCCGCGCGTGGTCTCGACTGCCTGCAAAGAGGTGCTTGAGTTGCCGCGTGACTGCGGCGG
CCGACTTCGTGGGACTTGCCCGCCACGCTGACGAAGGGCGTAATCCAGCACACTGGCGGCCGTTACTAGTTTC
AGAGCGGCCGCCACCGCGGTGGAGGGCGGCACCTCGCTAACGGATTCACCGTTTTTATCAGGCTCTGGGAGGC
AGAATAAATGATCATATCGTCAATTATTACCTCCACGGGGAGAGCCTGAGCAAACTGGCCTCAGAATTCAAAA
TGAAGTGAAGTTCCTATACTTTCTAGAGAATAGGAACTTCTATAGTGAGTCGAATAAGGGCGACACAAAATTT
ATTCTAAATGCATAATAAATACTGATAACATCTTATAGTTTGTATTATATTTTGTATTATCGTTGACATGTAT
AATTTTGATATCAAAAACTGATTTTCCCTTTATTATTTTCGAGATTTATTTTCTTAATTCTCTTTAACAAACT
AGAAATATTGTATATACAAAAAATCATAAATAATAGATGAATAGTTTAATTATAGGTGTTCATCAATCGAAAA
AGCAACGTATCTTATTTAAAGTGCGTTGCTTTTTTCTCATTTATAAGGTTAAATAATTCTCATATATCAAGCA
AAGTGACAGGCGCCCTTAAATATTCTGACAAATGCTCTTTCCCTAAACTCCCCCCATAAAAAAACCCGCCGAA
GCGGGTTTTTACGTTATTTGCGGATTAACGATTACTCGTTATCAGAACCGCCCAGGGGGCCCGAGCTTAAGAC
TGGCCGTCGTTTTACAACACAGAAAGAGTTTGTAGAAACGCAAAAAGGCCATCCGTCAGGGGCCTTCTGCTTA
GTTTGATGCCTGGCAGTTCCCTACTCTCGCCTTCCGCTTCCTCGCTCACTGACTCGCTGCGCTCGGTCGTTCG
GCTGCGGCGAGCGGTATCAGCTCACTCAAAGGCGGTAATACGGTTATCCACAGAATCAGGGGATAACGCAGGA
AAGAACATGTGAGCAAAAGGCCAGCAAAAGGCCAGGAACCGTAAAAAGGCCGCGTTGCTGGCGTTTTTCCATA
GGCTCCGCCCCCCTGACGAGCATCACAAAAATCGACGCTCAAGTCAGAGGTGGCGAAACCCGACAGGACTATA
AAGATACCAGGCGTTTCCCCCTGGAAGCTCCCTCGTGCGCTCTCCTGTTCCGACCCTGCCGCTTACCGGATAC
CTGTCCGCCTTTCTCCCTTCGGGAAGCGTGGCGCTTTCTCATAGCTCACGCTGTAGGTATCTCAGTTCGGTGT
AGGTCGTTCGCTCCAAGCTGGGCTGTGTGCACGAACCCCCCGTTCAGCCCGACCGCTGCGCCTTATCCGGTAA
CTATCGTCTTGAGTCCAACCCGGTAAGACACGACTTATCGCCACTGGCAGCAGCCACTGGTAACAGGATTAGC
AGAGCGAGGTATGTAGGCGGTGCTACAGAGTTCTTGAAGTGGTGGGCTAACTACGGCTACACTAGAAGAACAG
TATTTGGTATCTGCGCTCTGCTGAAGCCAGTTACCTTCGGAAAAAGAGTTGGTAGCTCTTGATCCGGCAAACA
AACCACCGCTGGTAGCGGTGGTTTTTTTGTTTGCAAGCAGCAGATTACGCGCAGAAAAAAAGGATCTCAAGAA
GATCCTTTGATCTTTTCTACGGGGTCTGACGCTCAGTGGAACGACGCGCGCGTAACTCACGTTAAGGGATTTT
GGTCATGAGCTTGCGCCGTCCCGTCAAGTCAGCGTAATGCTCTGCTTT
SEQ ID NO: 7
   *pJ201 : ValC_wt*
   *E. coli* cloning vector pJ201 with the codon optimized gene (*valC-opt*) encoding the wild type Valencene Synthase
   Nucleotide sequence

TAGAAAAACTCATCGAGCATCAAATGAAACTGCAATTTATTCATATCAGGATTATCAATACCATATTTTTGAA
AAAGCCGTTTCTGTAATGAAGGAGAAAACTCACCGAGGCAGTTCCATAGGATGGCAAGATCCTGGTATCGGTC
TGCGATTCCGACTCGTCCAACATCAATACAACCTATTAATTTCCCCTCGTCAAAAATAAGGTTATCAAGTGAG
AAATCACCATGAGTGACGACTGAATCCGGTGAGAATGGCAAAAGTTTATGCATTTCTTTCCAGACTTGTTCAA
CAGGCCAGCCATTACGCTCGTCATCAAAATCACTCGCATCAACCAAACCGTTATTCATTCGTGATTGCGCCTG
AGCGAGGCGAAATACGCGATCGCTGTTAAAAGGACAATTACAAACAGGAATCGAGTGCAACCGGCGCAGGAAC
ACTGCCAGCGCATCAACAATATTTTCACCTGAATCAGGATATTCTTCTAATACCTGGAACGCTGTTTTTCCGG
GGATCGCAGTGGTGAGTAACCATGCATCATCAGGAGTACGGATAAAATGCTTGATGGTCGGAAGTGGCATAAA
TTCCGTCAGCCAGTTTAGTCTGACCATCTCATCTGTAACATCATTGGCAACGCTACCTTTGCCATGTTTCAGA
AACAACTCTGGCGCATCGGGCTTCCCATACAAGCGATAGATTGTCGCACCTGATTGCCCGACATTATCGCGAG
CCCATTTATACCCATATAAATCAGCATCCATGTTGGAATTTAATCGCGGCCTCGACGTTTCCCGTTGAATATG
GCTCATATTCTTCCTTTTTCAATATTATTGAAGCATTTATCAGGGTTATTGTCTCATGAGCGGATACATATTT
GAATGTATTTAGAAAAATAAACAAATAGGGGTCAGTGTTACAACCAATTAACCAATTCTGAACATTATCGCGA
GCCCATTTATACCTGAATATGGCTCATAACACCCCTTGTTTGCCTGGCGGCAGTAGCGCGGTGGTCCCACCTG
ACCCCATGCCGAACTCAGAAGTGAAACGCCGTAGCGCCGATGGTAGTGTGGGGACTCCCCATGCGAGAGTAGG
GAACTGCCAGGCATCAAATAAAACGAAAGGCTCAGTCGAAAGACTGGGCCTTTCGCCCGGGCTAATTAGGGGG
TGTCGCCCTTAGGTACGAACTCGATTGACGGGTCTCAAGCTTGTCGACCTGCAGGGATCCTTATCACGGGATG
ATCGGTTCCACGAAGAACTTCGTGATCGCATCCTGGATCTCCTTCCGATTCGAATGGGTGAAGAGGTCCCCGT
CGTTCAGCATGAAGAAGATCACCCGCATGCCCACGTTGAGGTACAGGTTGCGGAAGCTGCCATGCATGTTGCT
CGGTTCGAGGAGTTCCTTGTTGAGCTCCTGCACGCAGCTCTCGATGCGGATTTTCAGGTAGTTCAGGGCGTCC
TCGCGCGTGGAGCCGTGATTATCCTTCATATAGCATTCGATCGACGAGGCCATCTCCCCATGCGCCTGCTCGT
CCTCGAAATCCTTCACATCATCGACGAGCCGCGTGATGAGCGACAGCAGGGCCTGGCTCTTGCTGCTCGGGAT
GTCGAGCTGCTCGAGGATCTCGTCGGACAGGGGCGTATCCATCAGGATCAGCGCATGGATCATGAGGATGCGC
ATCCCCGAGCTCTTCACGCCATTCATCAGGTACTCGTCGAAGCCGGGGATGTGGTTGGAGGCGATCCACTCGG
CCTCCTGCATATACGCCTCGATATACGACTTCCAGCACGACTGGAGATACTTATACACGAACGGGCCGTGCTT
GCGCTCGGCCTCGGACGCGATCTCCTTGTAGATCTCGTAGGAGAAATTGAACGCGATCTTCATGTAGTCCGGG
TGATTGTCCACCGTGCTGACATCCCAGCGCGTGAGGGTGGTCGTGAACGGCTTGAGTTCCTCGACGGTCCCGT
ACGTGTCATAGATGTCGTCCAGGAGGAACATCTTGGTGGACAGCTTGGTGCAGTTGATGCGGCAGGTGGAGAA
CTCGGGCTCGAACAGCGGGCTGGCCCACCAGAAGTAGCTCTCGATATGGCGGTGGCGGAAGAAGTCGAGCTGG
ATCGCGGACGAGTTCTTCCACCACGTGCTGATCAGTTTCATCTCATGCTGATGCTGGCTCTGGAGGATGTTGA
AGTCGAGGATCGCCAGCTCCAGGATCTTCTTCATATAGATCTTCGGGATCTTATAGAGGTTGTTGGCGAGCGA
GATATTGCAGTCCTGCTGCCGCATGATGTGGATGAAGTTCCACGCCTCCAGCCGCGGGAGGCGGCAGCGCCAG
GGGTACTTGAAGGTGTACTCGATCTCCATGAGCAGGGGGTTCTTCGATTCCTGGATGTCCCCATACTCCTTGA
TGACATGCTTGAGATACATCGACGCGAAGGCCCGCGCTTCCTTGAGGATGTCCTCATCCGGGAAGTCGAGTTC
CGAGGCCTTGTAGAGGTTCAGCATCATCTCCAGCTTGGCGTCCTGATCGCTGCTCTCGAACCCCGAGAACTGG
CCCTCCTCGCCCTTGAACTTCTTGAACACGTCCGACGACGCCACGTAGCGATTGAGCCGCAGGATGCGGAACC
CCAGCGCCACCATGTTGAGGTCGCCGAAGATGCTATCATGGTTCCAGAACTTATAGATATAATCCAGGGCCGT
CTTGATCTCTTCCTGGAAGTGCCGATCGATGCCCAGCCGCTGGACCACGTCGACGAACCAGATGTTCTCGAGG
ACCCCGAACTCATCCCCCTCGCCGTTGGAGAACATGTCCCGGATCTCATCGATCAGGATCTCGCGATGTTTAT
GATACGAGGAATCCGAATAGGGGGAGTTCAGCGACTGGATGAAATCGTCGGTCCAGAGGTTCGGATGGTGGTT
CCCGGTGCGGCGCAGCGCGTCCTTGACCGGCATGCAGGAGCTGCCATCATTGCTGGAATTGCCATTGAACATT
TCGGCCATATGCGGATCTGAGACCTTCAGCGACTAAGTACGTGTAAAGGGCGACACAAAATTTATTCTAAATG
CATAATAAATACTGATAACATCTTATAGTTTGTATTATATTTTGTATTATCGTTGACATGTATAATTTTGATA
TCAAAAACTGATTTTCCCTTTATTATTTTCGAGATTTATTTTCTTAATTCTCTTTAACAAACTAGAAATATTG
TATATACAAAAAATCATAAATAATAGATGAATAGTTTAATTATAGGTGTTCATCAATCGAAAAAGCAACGTAT
CTTATTTAAAGTGCGTTGCTTTTTTCTCATTTATAAGGTTAAATAATTCTCATATATCAAGCAAAGTGACAGG
CGCCCTTAAATATTCTGACAAATGCTCTTTCCCTAAACTCCCCCCATAAAAAAACCCGCCGAAGCGGGTTTTT
ACGTTATTTGCGGATTAACGATTACTCGTTATCAGAACCGCCCAGGGGGCCCGAGCTTAAGACTGGCCGTCGT
TTTACAACACAGAAAGAGTTTGTAGAAACGCAAAAAGGCCATCCGTCAGGGGCCTTCTGCTTAGTTTGATGCC
TGGCAGTTCCCTACTCTCGCCTTCCGCTTCCTCGCTCACTGACTCGCTGCGCTCGGTCGTTCGGCTGCGGCGA
GCGGTATCAGCTCACTCAAAGGCGGTAATACGGTTATCCACAGAATCAGGGGATAACGCAGGAAAGAACATGT
GAGCAAAAGGCCAGCAAAAGGCCAGGAACCGTAAAAAGGCCGCGTTGCTGGCGTTTTTCCATAGGCTCCGCCC
CCCTGACGAGCATCACAAAAATCGACGCTCAAGTCAGAGGTGGCGAAACCCGACAGGACTATAAAGATACCAG
GCGTTTCCCCCTGGAAGCTCCCTCGTGCGCTCTCCTGTTCCGACCCTGCCGCTTACCGGATACCTGTCCGCCT
TTCTCCCTTCGGGAAGCGTGGCGCTTTCTCATAGCTCACGCTGTAGGTATCTCAGTTCGGTGTAGGTCGTTCG
CTCCAAGCTGGGCTGTGTGCACGAACCCCCCGTTCAGCCCGACCGCTGCGCCTTATCCGGTAACTATCGTCTT
GAGTCCAACCCGGTAAGACACGACTTATCGCCACTGGCAGCAGCCACTGGTAACAGGATTAGCAGAGCGAGGT

ATGTAGGCGGTGCTACAGAGTTCTTGAAGTGGTGGCTAACTACGGCTACACTAGAAGAACAGTATTTGGTAT
CTGCGCTCTGCTGAAGCCAGTTACCTTCGGAAAAAGAGTTGGTAGCTCTTGATCCGGCAAACAAACCACCGCT
GGTAGCGGTGGTTTTTTTGTTTGCAAGCAGCAGATTACGCGCAGAAAAAAAGGATCTCAAGAAGATCCTTTGA
TCTTTTCTACGGGGTCTGACGCTCAGTGGAACGACGCGCGCGTAACTCACGTTAAGGGATTTTGGTCATGAGC
TTGCGCCGTCCCGTCAAGTCAGCGTAATGCTCTGCTTT

## Claims

1. Valencene synthase comprising an amino acid sequence having at least 85 % sequence identity with SEQ ID NO: 2, wherein
the valencene synthase has one or more modifications, in particular one or more substitutions, compared to the valencene synthase represented by SEQ ID NO: 2, at an amino acid position corresponding to a position selected from the group of 16A, 16T, 16S, 128L, 171R, 187K, 225S, 244S. 244T, 300Y, 302D, 307T, 307A, 319Q, 323A, 327L, 331G, 334L, 398I, 398M, 398T, 405T, 405V, 409F, 410F, 410V, 410L, 412G, 436L, 436K, 436T, 436W, 438T, 439G, 439A, 444I, 444V, 448S, 449F, 449I, 449Y, 450L, 450M, 450V, 463E, 4635, 463G, 463W, 488Y, 488H, 488S, 490N, 490A, 490T, 490F, 492A, 492K, 502Q, 503S, 507E, 507Q, 527T, 527S, 527A, 556T, 559H, 559L, 559V, 560L, 566S, 566A, 566G, 568S, 569I, 569V,570T, 570G, 570A and 570P,and wherein
the valencene synthase has an increased productivity towards the conversion of farnesyl diphosphate into valencene (expressed as molar amount of valencene formed per hour) compared to a valencene synthase represented by SEQ ID NO: 2.

2. Valencene synthase according to claim 1, wherein the valencene synthase has an increased specific productivity, increased stability, increased product specificity (relative to the conversion of farnesyl diphosphate into Germacrene A) or an increased expression in a host cell, compared to a valencene synthase represented by SEQ ID NO: 2.

3. Valencene synthase according to claim 2, wherein the product specificity, expressed as the molar ratio valencene formed from farnesyl diphospate to Germacrene A formed from farnesyl diphosphate (under test conditions), is 10 or more, preferably 13-30, in particular 15-25.

4. Valencene synthase according to any of the preceding claims, wherein the specific productivity of the valencene synthase, expressed as the molar amount of valencene formed per hour per amount of valencene synthase is at least 1.5 times, preferably 2.0 to 10 times, in particular 2.5 to 5 times the specific productivity of the valencene synthase represented by SEQ ID NO: 2.

5. Valencene synthase according to any of the preceding claims, wherein at a position corresponding to a position having a cysteine in SEQ ID NO: 2 a different amino acid is present.

6. Valencene synthase according to any of the preceding claims, wherein the valencene synthase comprises a first polypeptide segment and a second polypeptide segment, the first segment comprising a tag-peptide and the second segment comprising a polypeptide having valencene synthase activity.

7. Nucleic acid, comprising a nucleic acid sequence encoding a valencene synthase according to any of the preceding claims, or a complementary sequence thereof.

8. Expression vector comprising a nucleic acid according to claim 7.

9. Antibody having specific binding activity to a valencene synthase according to any of the claims 1 to 6.

10. A host cell, which may be an organism *per se* or part of a multi-cellular organism, said host cell comprising an expression vector according to claim 8, which host cell is selected from the group of bacterial cells, fungal cells and plant cells, and more preferably is:
a bacterial cell selected from the group of gram negative bacteria, such as *Rhodobacter, Paracoccus* or *Escherichia;*
a fungal cell selected from the group of *Aspergillus, Blakeslea, Penicillium, Phaffia* (*Xanthophyllomyces*), *Pichia, Saccharomyces,* and *Yarrowia;*
a transgenic plant or culture comprising transgenic plant cells, wherein the host cell is of a transgenic plant selected from *Nicotiana spp, Solanum spp, Cichorum intybus, Lactuca sativa, Mentha spp, Artemisia annua,* tuber forming plants, oil crops and trees; or
a transgenic mushroom or culture comprising transgenic mushroom cells, wherein the host cell is selected from *Schizophyllum, Agaricus* and *Pleurotus.*

11. Method for preparing valencene, comprising converting farnesyl diphosphate to valencene in the presence of a valencene synthase according to any of the claims 1-6, wherein preferably the valencene is prepared using a host cell according to claim 10.

12. Method for preparing nootkatone, comprising converting a polyprenyl diphosphate substrate to nootkatone in the presence of a valencene synthase according to any of the claims 1-6.

## Patentansprüche

1. Valencen-Synthase, umfassend eine Aminosäuresequenz, die mindestens 85% Sequenzidentität zu SEQ ID NO: 2 aufweist, wobei
die Valencen-Synthase eine oder mehr Modifikationen, insbesondere eine oder mehr Substitutionen, im Vergleich zu der Valencen-Synthase aufweist, die durch SEQ ID NO: 2 dargestellt wird, an einer Aminosäureposition, die einer Position entspricht, ausgewählt aus der Gruppe der Folgenden 16A, 16T, 16S, 128L, 171R, 187K, 225S, 244S, 244T, 300Y, 302D, 307T, 307A, 319Q, 323A, 327L, 331G, 334L, 3981, 398M, 398T, 405T, 405V, 409F, 410F, 410V, 410L, 412G, 436L, 436K, 436T, 436W, 438T, 439G, 439A, 444I, 444V, 448S, 449F, 449I, 449Y, 450L, 450M, 450V, 463E, 463S, 463G, 463W, 488Y, 488H, 488S, 490N, 490A, 490T, 490F, 492A, 492K, 502Q, 503S, 507E, 507Q, 527T, 527S, 527A, 556T, 559H, 559L, 559V, 560L, 566S, 566A, 566G, 568S, 569I, 569V, 570T, 570G, 570A und 570P, und wobei
die Valencen-Synthase eine erhöhte spezifische Produktivität hinsichtlich der Umwandlung von Farnesyl-Diphosphat in Valencen hat (ausgedrückt als Molmenge an Valencen, die pro Stunde gebildet wird), im Vergleich zu einer durch SEQ ID NO: 2 dargestellten Valencen-Synthase.

2. Valencen-Synthase nach Anspruch 1, wobei die Valencen-Synthase eine erhöhte spezifische Produktivität, erhöhte Stabilität, erhöhte Produktspezifität (bezogen auf die Umwandlung von Farnesyl-Diphosphat in Germacren A) oder eine erhöhte Expression in einer Wirtszelle im Vergleich zu einer durch SEQ ID NO: 2 dargestellten Valencen-Synthase aufweist.

3. Valencen-Synthase nach Anspruch 2, wobei die Produktspezifität, ausgedrückt als das Molverhältnis von aus Farnesyldiphospat gebildetem Valencen zu aus Farnesyldiphosphat gebildetem Germacren A (unter Testbedingungen) 10 oder mehr, vorzugsweise 13 bis 30, insbesondere 15 bis 25, beträgt.

4. Valencen-Synthase nach einem der vorhergehenden Ansprüche, worin die spezifische Produktivität der Valencen-Synthase, ausgedrückt als die Molmenge an Valencen, die pro Stunde pro Menge an Valencen-Synthase gebildet wird, mindestens das 1,5-Fache, vorzugsweise das 2,0- bis 10-Fache, insbesondere 2,5- bis 5-Fache der spezifischen Produktivität der durch SEQ ID NO: 2 dargestellten Valencen-Synthase ist.

5. Valencen-Synthase nach einem der vorhergehenden Ansprüche, wobei an einer Position, die einer Position mit einem Cystein in SEQ ID NO: 2 entspricht, eine andere Aminosäure zugegen ist.

6. Valencen-Synthase nach einem der vorhergehenden Ansprüche, wobei die Valencen-Synthase ein erstes Polypeptidsegment und ein zweites Polypeptidsegment umfasst, wobei das erste Segment ein tag-Peptid umfasst und das zweite Segment ein Polypeptid mit Valencen-Synthase-Aktivität umfasst.

7. Nukleinsäure, umfassend eine Nukleinsäuresequenz, die eine Valencen-Synthase nach einem der vorhergehenden Ansprüche codiert, oder eine komplementäre Sequenz davon.

8. Expressionsvektor, der eine Nukleinsäure nach Anspruch 7 umfasst.

9. Antikörper mit spezifischer Bindungsaktivität an eine Valencen-Synthase nach einem der Ansprüche 1 bis 6.

10. Wirtszelle, die ein Organismus an sich oder ein Teil eines vielzelligen Organismus sein kann, wobei die Wirtszelle einen Expressionsvektor nach Anspruch 8 umfasst, wobei die Wirtszelle aus der Gruppe von Bakterienzellen, Pilzzellen und Pflanzenzellen ausgewählt ist und stärker bevorzugt ist:
eine Bakterienzelle, ausgewählt aus der Gruppe der gramnegativen Bakterien, wie *Rhodobacter, Paracoccus* oder *Escherichia*;
eine Pilzzelle, ausgewählt aus der Gruppe *Aspergillus, Blakeslea, Penicillium, Phaffia* (*Xanthophyllomyces*), *Pichia, Saccharomyces,* und *Yarrowia*;
eine transgene Pflanze oder Kultur, die transgene Pflanzenzellen umfasst, wobei die Wirtszelle aus einer transgenen Pflanze stammt, ausgewählt aus *Nicotiana spp, Solanum spp, Cichorum intybus, Lactuca sativa, Mentha spp, Artemisia annua,* Knollen-bildenden Pflanzen, Ölpflanzen und Bäumen; oder
ein transgener Pilz oder eine Kultur, die transgene Pilzzellen umfasst, wobei die Wirtszelle aus *Schizophyllum, Agaricus* und *Pleurotus* ausgewählt ist.

11. Verfahren zur Herstellung von Valencen, umfassend das Umwandeln von Farnesyldiphosphat in Valencen in Gegenwart einer Valencen-Synthase nach einem der Ansprüche 1 bis 6, wobei das Valencen vorzugsweise unter Verwendung einer Wirtszelle nach Anspruch 10 hergestellt wird.

12. Verfahren zur Herstellung von Nootkaton, umfassend das Umwandeln eines Polyprenyldiphosphat-Substrats in Nootkaton in Gegenwart einer Valencen-Synthase nach einem der Ansprüche 1 bis 6.

## Revendications

1. Valencène synthase comprenant une séquence d'acides aminés ayant une identité de séquence, d'au moins 85 %, avec la SEQ ID n° : 2, dans laquelle la valencène synthase a une ou plusieurs modification(s), en particulier une ou plusieurs substitution(s), en comparaison avec la valencène synthase représentée par la SEQ ID n° : 2, en une position d'acide aminé correspondant à une position choisie dans le groupe des 16A, 16T, 16S, 128L, 171R, 187K, 225S, 244S, 244T, 300Y, 302D, 307T, 307A, 319Q, 323A, 327L, 331G, 334L, 398I, 398M, 398T, 405T, 405V, 409F, 410F, 410V, 410L, 412G, 436L, 436K, 436T, 436W, 438T, 439G, 439A, 444I, 444V, 448S, 449F, 449I, 449Y, 450L, 450M, 450V, 463E, 463S, 463G, 463W, 488Y, 488H, 488S, 490N, 490A, 490T, 490F, 492A, 492K, 502Q, 503S, 507E, 507Q, 527T, 527S, 527A, 556T, 559H, 559L, 559V, 560L, 566S, 566A, 566G, 568S, 569I, 569V, 570T, 570G, 570A et 570P, et dans laquelle
la valencène synthase a une productivité accrue pour la conversion du diphosphate de farnésyle en valencène (exprimée sous forme de quantité molaire de valencène formée par heure), en comparaison avec une valencène synthase représentée par la SEQ ID n° : 2.

2. Valencène synthase selon la revendication 1, dans laquelle la valencène synthase a une productivité spécifique accrue, une stabilité accrue, une spécificité de produit accrue (par rapport à la conversion du diphosphate de farnésyle en Germacrène A) ou une expression accrue dans une cellule hôte, en comparaison avec une valencène synthase représentée par la SEQ ID n° : 2.

3. Valencène synthase selon la revendication 2, dans laquelle la spécificité de produit, exprimée sous forme du rapport molaire du valencène, formé à partir du diphosphate de farnésyle, au Germacrène A, formé à partir du diphosphate de farnésyle (dans des conditions de test), est de 10 ou plus, de préférence de 13 à 30, en particulier de 15 à 25.

4. Valencène synthase selon l'une quelconque des revendications précédentes, dans laquelle la productivité spécifique de la valencène synthase, exprimée sous forme de la quantité molaire de valencène formée par heure par quantité de valencène synthase, est d'au moins 1,5 fois, de préférence de 2,0 à 10 fois, en particulier de 2,5 à 5 fois la productivité spécifique de la valencène synthase représentée par la SEQ ID n° : 2.

5. Valencène synthase selon l'une quelconque des revendications précédentes, dans laquelle à une position correspondant à une position ayant une cystéine dans la SEQ ID n° : 2 est présent un acide aminé différent.

6. Valencène synthase selon l'une quelconque des revendications précédentes, dans laquelle la valencène synthase comprend un premier segment polypeptidique et un deuxième segment polypeptidique, le premier segment comprenant un peptide étiquette et le deuxième segment comprenant un polypeptide, ayant une activité de valencène synthase.

7. Acide nucléique, comprenant une séquence d'acide nucléique qui code pour une valencène synthase, selon l'une quelconque des revendications précédentes, ou une séquence complémentaire de celle-ci.

8. Vecteur d'expression comprenant un acide nucléique selon la revendication 7.

9. Anticorps ayant une activité de liaison spécifique à une valencène synthase selon l'une quelconque des revendications 1 à 6.

10. Cellule hôte, qui peut être un organisme en soi ou une partie d'un organisme multicellulaire, ladite cellule hôte comprenant un vecteur d'expression, selon la revendication 8, cellule hôte qui est choisie dans le groupe des cellules bactériennes, cellules fongiques et cellules végétales et, de manière davantage préférée, qui est :
une cellule bactérienne choisie dans le groupe des bactéries gram négatives, telles que *Rhodobacter, Paracoccus* ou *Escherichia* ;
une cellule fongique choisie dans le groupe d'*Aspergillus,* de *Blakeslea,* de *Penicillium,* de *Phaffia* (*Xanthophyllomyces*), de *Pichia,* de *Saccharomyces* et de *Yarrowia ;*
une plante ou culture transgénique comprenant des cellules végétales transgéniques, dans laquelle la cellule hôte est celle d'une plante transgénique choisie parmi *Nicotiana spp, Solanum spp, Cichorum intybus, Lactuca sativa, Mentha spp, Artemisia annua,* des plantes formant des tubercules, des cultures et arbres oléagineux ; ou
un champignon ou une culture transgénique comprenant des cellules de champignon transgéniques, dans laquelle la cellule hôte est choisie parmi *Schizophyllum, Agaricus* et *Pleurotus.*

11. Procédé de préparation de valencène, comprenant la conversion de diphosphate de farnésyle en valencène en présence d'une valencène synthase, selon l'une quelconque des revendications 1 à 6, dans laquelle le valencène est préparé, de préférence, en utilisant une cellule hôte selon la revendication 10.

12. Procédé de préparation de nootkatone, comprenant la conversion d'un substrat de diphosphate de polyprényle en nootkatone, en présence d'une valencène synthase selon l'une quelconque des revendications 1 à 6.
